# EUROPEAN PATENT SPECIFICATION

(11) **EP 3 843 757 B1**
(45) Date of publication and mention of the grant of the patent: **24.04.2024**
(21) Application number: 19769715.4
(22) Date of filing: 26.08.2019
(51) Int. Cl.: A61K 35/17, A61K 39/395, C07K 16/28, C07K 16/30, A01N 1/02, C12N 5/0783

(54) **CRYOPRESERVED NK CELLS PRELOADED WITH AN ANTIBODY CONSTRUCT**
KRYOPRESERVIERTE NK-ZELLEN, VORGELADEN MIT EINEM ANTIKÖRPERKONSTRUKT
CELLULES NK CRYOPRESERVES PRECHARGEES D'UN CONSTRUCTION ANTICORPS

(30) Priority: 27.08.2018 EP 18191031
(43) Date of publication of application: 07.07.2021
(60) Divisional application: 24163976.4
(73) Proprietor: Affimed GmbH, 68165 Mannheim (DE)
(72) Inventor: REUSCH, Uwe, 68165 Mannheim (DE); KOCH, Joachim, 68165 Mannheim (DE); TREDER, Martin, 68165 Mannheim (DE); DULAT, Holger J., 68165 Mannheim (DE)
(74) Representative: Schüssler, Andrea
(86) International application number: PCT/EP2019/072727
(87) International publication number: WO 2020/043670

(56) References cited:
- WO-A1-2011/004201
- UWE REUSCH ET AL: "A novel tetravalent bispecific TandAb (CD30/CD16A) efficiently recruits NK cells for the lysis of CD30 + tumor cells", MABS, vol. 6, no. 3, 26 March 2014 (2014-03-26), pages 727-738, XP055197046, ISSN: 1942-0870, DOI: 10.4161/mabs.28591
- Yamashita Makiko et al.: "A novel method for evaluating antibody-dependent cell-mediated cytotoxicity by flowcytometry using cryopreserved human peripheral blood mononuclear cells.", Science Reports, 27 January 2016 (2016-01-27), pages 1-10, XP002796192, Retrieved from the Internet: URL:https://www.ncbi.nlm.nih.gov/pmc/artic les/PMC4728441/

## Description

### Field of the invention:

The present invention provides isolated human NK cells in a cryopreserved state, which have been preloaded prior to the freezing with an antibody construct, the antibody construct comprising at least a first binding domain binding to a receptor antigen on the cell surface of an immunological effector cell, especially one that is expressed on NK cells, and a second binding domain binding to the cell surface an antigen on the cell surface of a target cell.

### Background of the invention:

Natural Killer (NK) cells are potent cytotoxic immune effector cells of the innate immune system. They are capable of recognizing and destroying tumor cells as well as cells that have been infected by viruses or bacteria. NK cells can induce an antigen-independent immune response against malignant cells. Moreover, in addition to the NK cells there are other immunological effector cells, e.g. monocytes, macrophages, neutrophils etc., which are capable of killing tumor cells or cells infected by viruses or bacteria. In general, this is understood as the innate immunity.

A growing number of scientific reports and clinical studies have shown promising and potent anti-tumor effects when using NK cell-based immunotherapy. Currently, various approaches are being investigated with the aim of enhancing the number and function of NK cells. One of said approaches utilizes either bi- or multi-specific antibody constructs with the aim of enhancing the specificity of endogenous NK cells by crosslinking them with the respective target cells. In order to make use of such synergies, the use of adoptive cellular therapy in combination with antibody-based therapy has been combined in various indications. Various sources of NK cells for adoptive transfer are under evaluation and include allogeneic haplo-identical NK cells that have undergone short- or long-term activation or expansion, umbilical cord NK cells, which are also expanded/activated under defined conditions, NK cell lines or stem cell derived/differentiated NK cells. All sources can theoretically also be used to generate CAR-NK cells, which are derived from a new type of genetic modification with lenti- or retroviruses to produce stable antigen specific NK cells. First examples include a CD19-CAR-NK cell approach that is currently tested in NHL patients.

However, when combining antibody constructs with any of the afore mentioned sources of NK cells for adoptive transfer, it is always necessary to prepare the cells at the point of use, i.e. the hospital in which the therapy is applied to the respective patient, and, subsequently, to co-administer to said patient the required dose of the respective antibody constructs. Apart from the fact that this might be a logistic issue for the majority of hospitals it is further of note that all work packages required for the preparation of cells for administration must be performed in accordance with strictly controlled protocols in order to rule out any avoidable issue which increases such risk.

REUSCH U ET AL.. (MABS 2014, 6(3): 727-738) describe an anti-CD30/anti-CD16A antibody for engaging NK cells to lyse CD30⁺ Hodgkin Reed-Sternberg (HRS) lymphoma cells.

YAMASHITA ET AL. (Scientific Reports 6: 19772) describe the use of frozen NK cells for an *in-vitro* cytotoxicity test with Trastuzumab, which is an IgG1 mAb against HER2

WO 2011/004201 describes activated frozen NK cells free of activation agent.

Accordingly, there is a need for means and methods to simplify such therapy in order to allow the applicability for a larger group of patients, especially in non-specialized hospitals or medical centers.

### The Figures show:

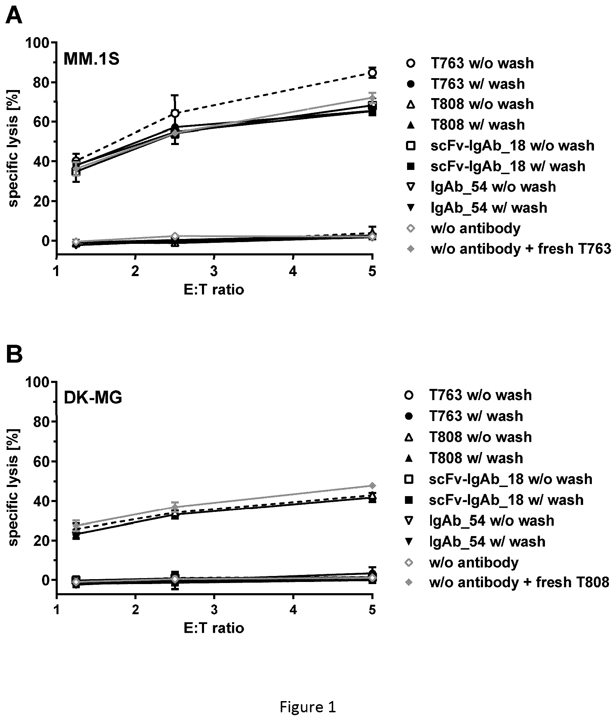

**Figure 1****:** 4 h calcein-release cytotoxicity assay on MM.1S (A) or DK-MG (B) target cells NK cells as effector cells at the indicated E:T ratio that were preloaded with 10 µg/mL of the indicated antibodies and washed (w/) or not washed (w/o) prior one freeze/thaw cycle. As a control, the same NK cells were not preloaded but washed and subjected to one freeze/thaw cycle and the indicated antibody was added to the cytotoxicity assay (fresh; solid grey diamonds).

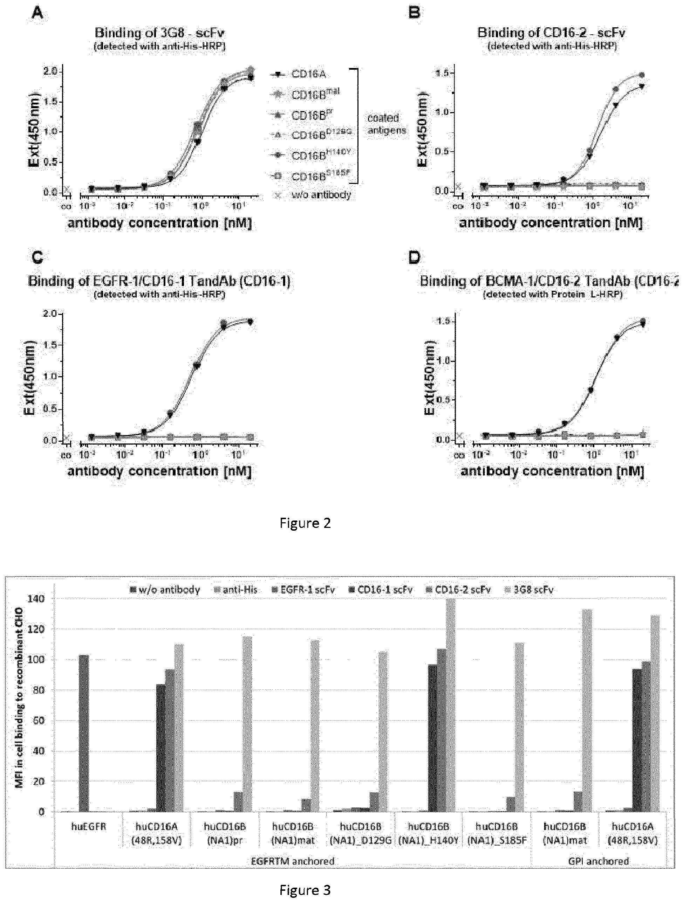

**Figure 2****:** Binding of different anti-CD16 antibodies to coated CD16 antigen variants analyzed in ELISA. 96-well ELISA plates were coated with recombinant antigen variants shown in the legend of panel A. Different antibodies shown in (A)-(D) were serially diluted and incubated on the plates at the indicated concentration range. Bound antibodies were detected with anti-His-HRP in (A)-(C), or Protein L-HRP. TMB substrate reactions were measured at 450nm (Ext (450nm)). Binding curves were fitted using four-parameter logistic (4PL) curve model log(agonist) vs. response - Variable slope in Graphpad Prism.
**Figure 3****:** Reactivity of different anti-CD16 scFv antibodies or control scFv or mAbs with recombinant CD16 antigen variants or a control EGFR antigen expressed on CHO cells with a EGFR transmembrane domain (TM) or GPI-anchor.

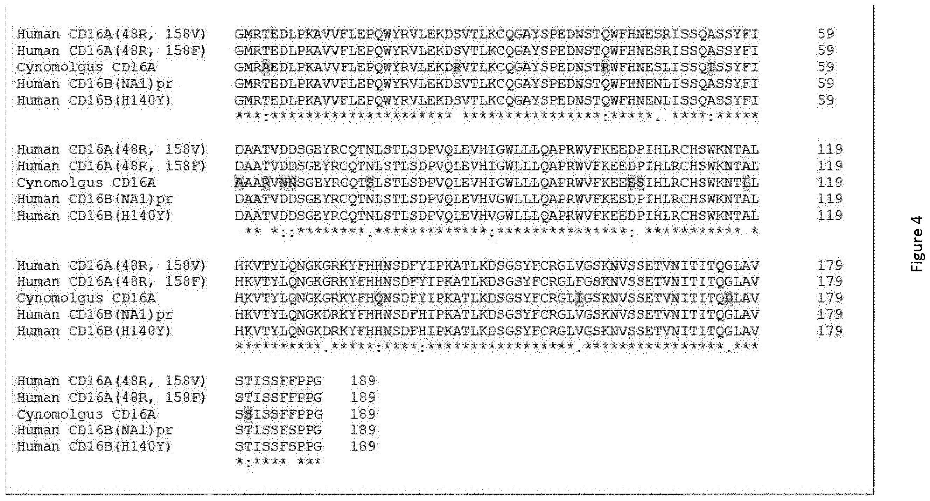

**Figure 4****:** Sequence alignment of extracellular domains of human and cynomolgus CD16A and human CD16B variants used as recombinant antigens in Experiments summarized in Table 3. Variations in the cynomolgus CD16A ECD sequence are highlighted in grey. CLUSTAL O (1.2.4) multiple sequence alignment tool was used for the alignment.

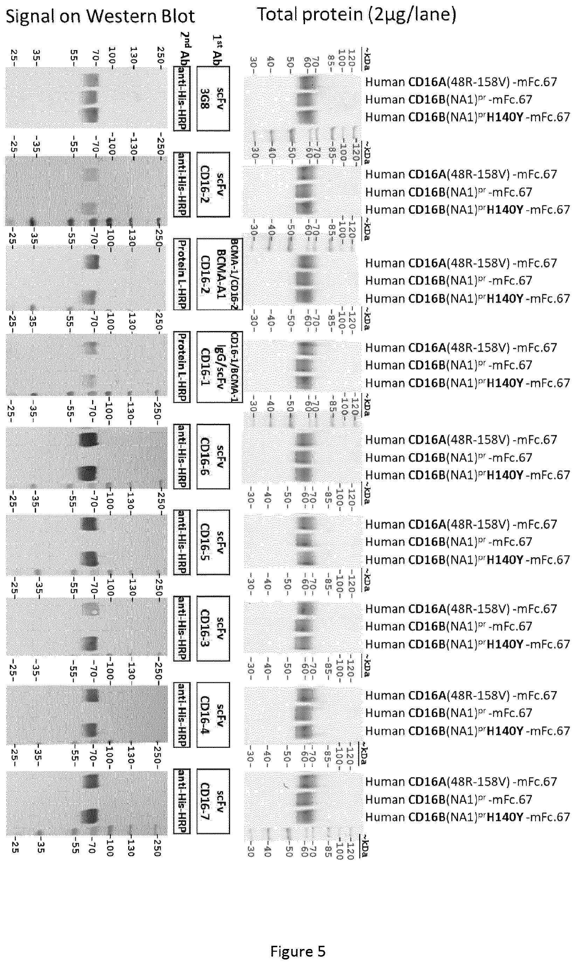

**Figure 5****:** Binding of different anti-CD16 antibodies to CD16 antigen variants after separation by SDS-PAGE and Western blotting. 2µg of recombinant antigen protein were loaded per lane, separated by SDS-PAGE, blotted on PVDF membranes, and incubated with the indicated primary and secondary antibodies. Signals from colorimetric development with DAB substrate shows distinct recognition of CD16A and CD16B antigen variants.

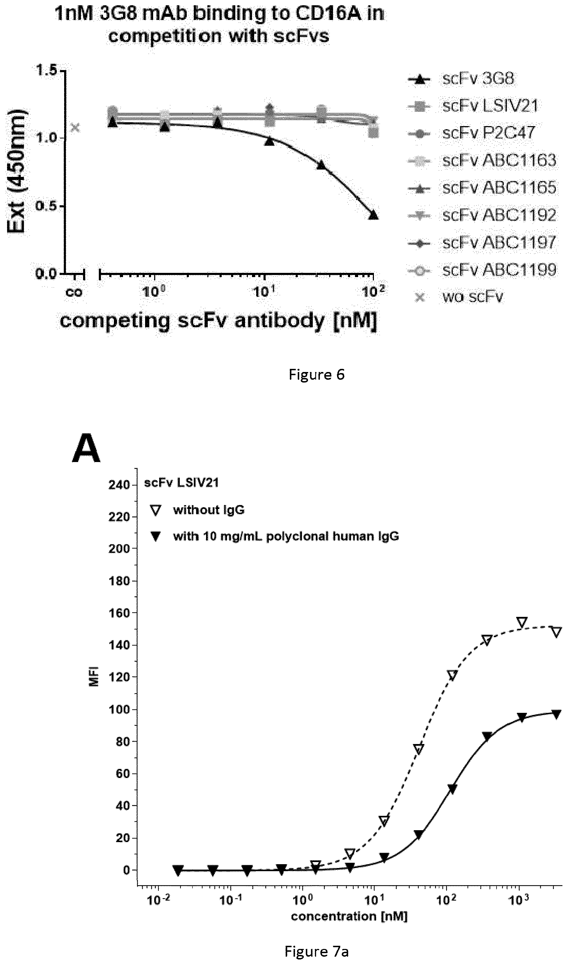

**Figure 6****:** Binding competition of different anti-CD16 scFv antibodies with mAb 3G8 to CD16A in ELISA. Plates coated with CD16A (48R, 158V)-mFc.67 antigen were incubated with a mixture of 1nM of mAb 3G8 and serial dilutions of different anti-CD16 scFvs in the indicated concentration range. CD16A-bound 3G8 was detected with goat anti-mouse IgG(H+L)-HRPO. TMB substrate reactions were measured at 450nm (Ext (450nm)). Binding curves were fitted using four-parameter logistic (4PL) curve model log(agonist) vs. response - Variable slope in Graphpad Prism.

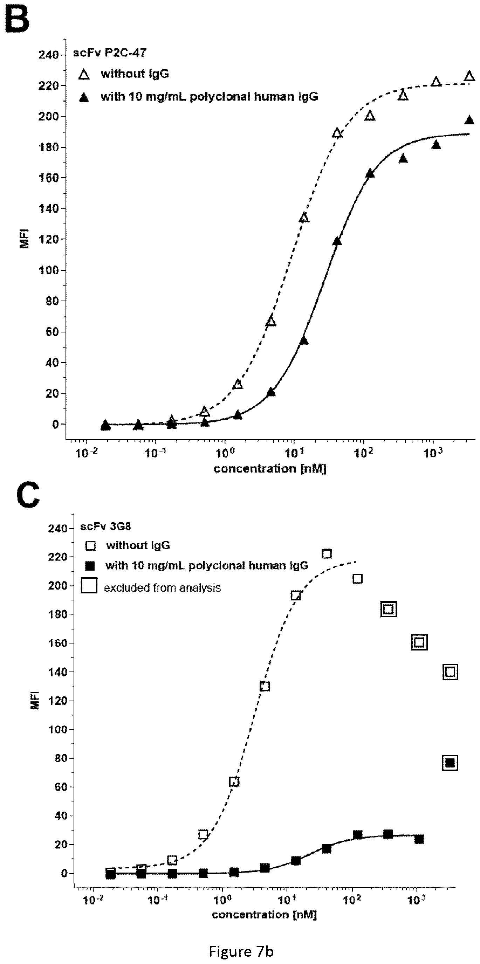

**Figure 7****:** Titration of scFv_CD16-1 containing the human Fv domains from clone CD16-1 (A), scFv_CD16-2 containing the human anti-CD16 Fv domains from clone CD16-2 (B), and scFv_3G8, containing the murine Fv domains from mAb 3G8 (C) on primary human NK cells at 37°C in the presence or absence of 10 mg/mL polyclonal human IgG.

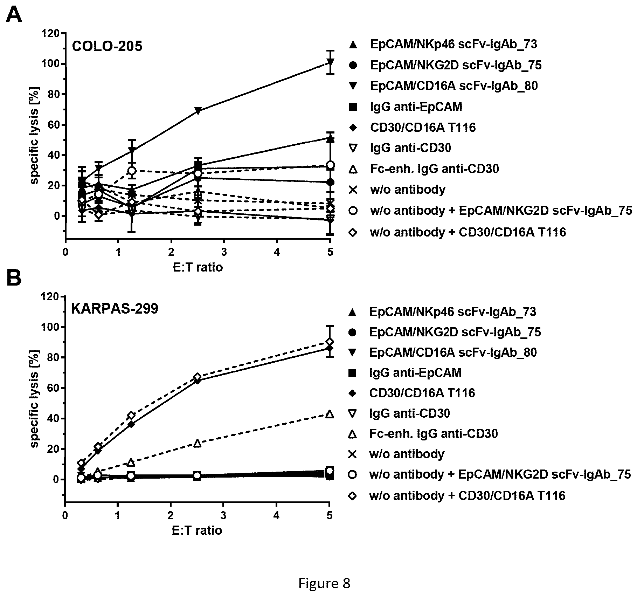

**Figure 8****:** 4 h calcein-release cytotoxicity assay on COLO 205 (A) or KARPAS-299 (B) target cells NK cells as effector cells at the indicated E:T ratio that were preloaded with 10 µg/mL of the indicated antibodies and frozen at -80°C. As a control, aliquots of the same NK cells were not preloaded (w/o antibody) but also subjected to one freeze/thaw cycle, and the indicated antibodies (EpCAM/NKG2D scFv-lgAb_75 or CD30/CD16A TandAb) were freshly added at a concentration of 10 µg/mL to the cytotoxicity assay. Mean and SD of duplicate lysis values are plotted. Exp. No.: RBL 1464.

### Definitions

The term "antibody construct" refers to a molecule in which the structure and/or function is/are based on the structure and/or function of an antibody, e.g., of a full-length or whole immunoglobulin molecule and/or is/are drawn from the variable heavy chain (VH) and/or variable light chain (VL) domains of an antibody or fragment thereof. An antibody construct is hence capable of binding to its specific target or antigen. Furthermore, the binding domain of an antibody construct defined in the context of the invention comprises the minimum structural requirements of an antibody which allow for the target binding. This minimum requirement may e.g. be defined by the presence of at least the three light chain CDRs (i.e. CDR1, CDR2 and CDR3 of the VL region) and/or the three heavy chain CDRs (i.e. CDR1, CDR2 and CDR3 of the VH region), preferably of all six CDRs. An alternative approach to define the minimal structure requirements of an antibody is the definition of the epitope of the antibody within the structure of the specific target, respectively, the protein domain of the target protein composing the epitope region (epitope cluster) or by reference to a specific antibody competing with the epitope of the defined antibody. The antibodies on which the constructs defined in the context of the invention are based include for example monoclonal, recombinant, chimeric, deimmunized, humanized and human antibodies.

The binding domain of an antibody construct defined in the context of the invention may e.g. comprise the above referred groups of CDRs. Preferably, those CDRs are comprised in the framework of an antibody light chain variable region (VL) and an antibody heavy chain variable region (VH); however, it does not have to comprise both. Fd fragments, for example, have two VH regions and often retain some antigen-binding function of the intact antigen-binding domain. Additional examples for the format of antibody fragments, antibody variants or binding domains include (1) a Fab fragment, a monovalent fragment having the VL, VH, CL and CH1 domains; (2) a F(ab')₂fragment, a bivalent fragment having two Fab fragments linked by a disulfide bridge at the hinge region; (3) an Fd fragment having the two VH and CH1 domains; (4) an Fv fragment having the VL and VH domains of a single arm of an antibody, (5) a dAb fragment (Ward et al., (1989) Nature 341 :544-546), which has a VH domain; (6) an isolated complementarity determining region (CDR), and (7) a single chain Fv (scFv), the latter being preferred (for example, derived from an scFV-library).

Also, within the definition of "binding domain" or "domain which binds" are fragments of full-length antibodies, such as VH, VHH, VL, (s)dAb, Fv, Fd, Fab, Fab', F(ab')₂ or "r IgG" ("half antibody"). Antibody constructs as defined in the context of the invention may also comprise modified fragments of antibodies, also called antibody variants, such as scFv, di-scFv or bi(s)-scFv, scFv-Fc, scFv-zipper, scFab, Fab₂, Fab₃, diabodies, single chain diabodies, tandem diabodies (Tandab's), tandem di-scFv, tandem tri-scFv, "multibodies" such as triabodies or tetrabodies, and single domain antibodies such as nanobodies or single variable domain antibodies comprising merely one variable domain, which might be VHH, VH or VL, that specifically bind an antigen or epitope independently of other V regions or domains.

As used herein, the terms "single-chain Fv," "single-chain antibodies" or "scFv" refer to single polypeptide chain antibody fragments that comprise the variable regions from both the heavy and light chains, but lack the constant regions. Generally, a single-chain antibody further comprises a polypeptide linker between the VH and VL domains which enables it to form the desired structure which would allow for antigen binding. Single chain antibodies are discussed in detail by Plueckthun in The Pharmacology of Monoclonal Antibodies, vol. 1 13, Rosenburg and Moore eds. Springer-Verlag, New York, pp. 269-315 (1994). Various methods of generating single chain antibodies are known, including those described in U.S. Pat. Nos. 4,694,778 and 5,260,203; International Patent Application Publication No. WO 88/01649; Bird (1988) Science 242:423-442; Huston et al. (1988) Proc. Natl. Acad. Sci. USA 85:5879-5883; Ward et al. (1989) Nature 334:54454; Skerra et al. (1988) Science 242:1038-1041. In specific embodiments, single-chain antibodies can also be bispecific, multispecific, human, and/or humanized and/or synthetic.

Furthermore, the definition of the term "antibody construct" includes monovalent, bivalent and polyvalent / multivalent constructs and, thus, bispecific constructs, specifically binding to only two antigenic structure, as well as polyspecific/multispecific constructs, which specifically bind more than two antigenic structures, e.g. three, four or more, through distinct binding domains. Moreover, the definition of the term "antibody construct" includes molecules consisting of only one polypeptide chain as well as molecules consisting of more than one polypeptide chain, which chains can be either identical (homodimers, homotrimers or homo oligomers) or different (heterodimer, heterotrimer or heterooligomer). Examples for the above identified antibodies and variants or derivatives thereof are described inter alia in Harlow and Lane, Antibodies a laboratory manual, CSHL Press (1988) and Using Antibodies: a laboratory manual, CSHL Press (1999), Kontermann and Dubel, Antibody Engineering, Springer, 2nd ed. 2010 and Little, Recombinant Antibodies for Immunotherapy, Cambridge University Press 2009.

The term "valent" denotes the presence of a determined number of antigen-binding domains in the antigen-binding protein. A natural IgG has two antigen-binding domains and is bivalent. The antigen-binding proteins as defined in the context of the invention are at least trivalent. Examples of tetra-, penta- and hexavalent antigen-binding proteins are described herein.

The term "bispecific" as used herein refers to an antibody construct which is "at least bispecific", i.e., it comprises at least a first binding domain and a second binding domain, wherein the first binding domain binds to one antigen or target (here: NK cell receptor, e.g. CD16a), and the second binding domain binds to another antigen or target (here: the target cell surface antigen) . Accordingly, antibody constructs as defined in the context of the invention comprise specificities for at least two different antigens or targets. For example, the first domain does preferably bind to an extracellular epitope of an NK cell receptor of one or more of the species selected from human, Macaca spec. and rodent species.

The term "NK cell receptor" as used in the context of the invention defines proteins and protein complexes on the surface of NK cells. Thus, the term defines cell surface molecules, which are characteristic to NK cells, but are not necessary exclusively expressed on the surface of NK cells but also on other cells such as macrophages or T cells. Examples for NK cell receptors comprise, but are not limited to CD16a, CD16b, NKp46 and NKG2D.

"CD16A" refers to the activating receptor CD16A, also known as FcγRIIIA, expressed on the cell surface of NK cells. CD16A is an activating receptor triggering the cytotoxic activity of NK cells. The affinity of antibodies for CD16A directly correlates with their ability to trigger NK cell activation, thus higher affinity towards CD16A reduces the antibody dose required for activation. The antigen-binding site of the antigen-binding protein binds to CD16A, but not to CD16B. For example, an antigen-binding site comprising heavy (VH) and light (VL) chain variable domains binding to CD16A, but not binding to CD16B, may be provided by an antigen-binding site which specifically binds to an epitope of CD16A which comprises amino acid residues of the C-terminal sequence SFFPPGYQ (SEQ ID NO:172) and/or residues G130 and/or Y141 of CD16A (SEQ ID NO:23)) which are not present in CD16B.

"CD16B" refers to receptor CD16B, also known as FcγRIIIB, expressed on neutrophils and eosinophils. The receptor is glycosylphosphatidyl inositol (GPI) anchored and is understood to not trigger any kind of cytotoxic activity of CD16B positives immune cells.

"NKp46" refers to a cytotoxicity-activating receptor that may contribute to the increased efficiency of activated natural killer (NK) cells to mediate tumor cell lysis. It is also known as NCR1 or CD335.

"NKG2D refers to an activating and costimulatory receptor involved in immuno-surveillance upon binding to various cellular stress-inducible ligands displayed at the surface of autologous tumor cells and virus-infected cells. Provides both stimulatory and costimulatory innate immune responses on activated killer (NK) cells, leading to cytotoxic activity. Acts as a costimulatory receptor for T-cell receptor (TCR) in CD8(+) T-cell-mediated adaptive immune responses by amplifying T-cell activation. Stimulates perforin-mediated elimination of ligand-expressing tumor cells. It is also known as Killer Cell Lectin Like Receptor K1, KLRK1 or CD314.

The term "target cell surface antigen" refers to an antigenic structure expressed by a cell and which is present at the cell surface such that it is accessible for an antibody construct as described herein. It may be a protein, preferably the extracellular portion of a protein, a peptide that is presented on the cell surface in an MHC context (including HLA-A2, HLA-A11, HLA-A24, HLA-B44, HLA-C4) or a carbohydrate structure, preferably a carbohydrate structure of a protein, such as a glycoprotein. It is preferably a tumor associated or tumor restricted antigen.

The term "bispecific antibody construct" as defined in the context of the invention also encompasses multispecific antibody constructs such as trispecific antibody constructs, the latter ones including three binding domains, or constructs having more than three (e.g. four, five...) specificities. Examples for bi- or multispcific antibody constructs are provided e.g. in WO 2006/125668, WO 2015/158636, WO 2017/064221, PCT/EP2019/056516, PCT/IB2019/ 053040 and Ellwanger et a. (MAbs. 2019 Jul;11(5):899-918).

Given that the antibody constructs as defined in the context of the invention are (at least) bispecific, they do not occur naturally and they are markedly different from naturally occurring products. A "bispecific" antibody construct or immunoglobulin is hence an artificial hybrid antibody or immunoglobulin having at least two distinct binding sides with different specificities. Bispecific antibody constructs can be produced by a variety of methods including fusion of hybridomas or linking of Fab' fragments. See, e.g., Songsivilai & Lachmann, Clin. Exp. Immunol. 79:315- 321 (1990).

The at least two binding domains and the variable domains (VH / VL) of the antibody construct of the present invention may or may not comprise peptide linkers (spacer peptides). The term "peptide linker" comprises in accordance with the present invention an amino acid sequence by which the amino acid sequences of one (variable and/or binding) domain and another (variable and/or binding) domain of the antibody construct defined herein are linked with each other. The peptide linkers can also be used to fuse the third domain to the other domains of the antibody construct defined herein. An essential technical feature of such peptide linker is that it does not comprise any polymerization activity.

The antibody constructs as defined in the context of the invention are preferably "in vitro generated antibody constructs". This term refers to an antibody construct according to the above definition where all or part of the variable region (e.g., at least one CDR) is generated in a non-immune cell selection, e.g., an in vitro phage display, protein chip or any other method in which candidate sequences can be tested for their ability to bind to an antigen. This term thus preferably excludes sequences generated solely by genomic rearrangement in an immune cell in an animal. A "recombinant antibody" is an antibody made through the use of recombinant DNA technology or genetic engineering.

The term "monoclonal antibody" (mAb) or monoclonal antibody construct as used herein refers to an antibody obtained from a population of substantially homogeneous antibodies, i.e., the individual antibodies comprising the population are identical except for possible naturally occurring mutations and/or post-translation modifications (e.g., isomerizations, amidations) that may be present in minor amounts. Monoclonal antibodies are highly specific, being directed against a single antigenic side or determinant on the antigen, in contrast to conventional (polyclonal) antibody preparations which typically include different antibodies directed against different determinants (or epitopes). In addition to their specificity, the monoclonal antibodies are advantageous in that they are synthesized by the hybridoma culture, hence uncontaminated by other immunoglobulins. The modifier "monoclonal" indicates the character of the antibody as being obtained from a substantially homogeneous population of antibodies, and is not to be construed as requiring production of the antibody by any particular method.

For the preparation of monoclonal antibodies, any technique providing antibodies produced by continuous cell line cultures can be used. For example, monoclonal antibodies to be used may be made by the hybridoma method first described by Koehler et al., Nature, 256: 495 (1975), or may be made by recombinant DNA methods (see, e.g., U.S. Patent No. 4,816,567). Examples for further techniques to produce human monoclonal antibodies include the trioma technique, the human B-cell hybridoma technique (Kozbor, Immunology Today 4 (1983), 72) and the EBV-hybridoma technique (Cole et al., Monoclonal Antibodies and Cancer Therapy, Alan R. Liss, Inc. (1985), 77-96).

Hybridomas can then be screened using standard methods, such as enzyme-linked immunosorbent assay (ELISA) and surface plasmon resonance (BIACORE^{™}) analysis, to identify one or more hybridomas that produce an antibody that specifically binds with a specified antigen. Any form of the relevant antigen may be used as the immunogen, e.g., recombinant antigen, naturally occurring forms, any variants or fragments thereof, as well as an antigenic peptide thereof. Surface plasmon resonance as employed in the BIAcore system can be used to increase the efficiency of phage antibodies which bind to an epitope of a target cell surface antigen, (Schier, Human Antibodies Hybridomas 7 (1996), 97-105; Malmborg, J. Immunol. Methods 183 (1995), 7-13). Another exemplary method of making monoclonal antibodies includes screening protein expression libraries, e.g., phage display or ribosome display libraries. Phage display is described, for example, in Ladner et al., U.S. Patent No. 5,223,409; Smith (1985) Science 228:1315-1317, Clackson et ai, Nature, 352: 624-628 (1991) and Marks et al., J. Mol. Biol., 222: 581 -597 (1991).

In addition to the use of display libraries, the relevant antigen can be used to immunize a non-human animal, e.g., a rodent (such as a mouse, hamster, rabbit or rat). In one embodiment, the non-human animal includes at least a part of a human immunoglobulin gene. For example, it is possible to engineer mouse strains deficient in mouse antibody production with large fragments of the human Ig (immunoglobulin) loci. Using the hybridoma technology, antigen-specific monoclonal antibodies derived from the genes with the desired specificity may be produced and selected. See, e.g., XENOMOUSE^{™}, Green et al. (1994) Nature Genetics 7:13-21, US 2003-0070185, WO 96/34096, and WO 96/33735.

A monoclonal antibody can also be obtained from a non-human animal, and then modified, e.g., humanized, deimmunized, rendered chimeric etc., using recombinant DNA techniques known in the art. Examples of modified antibody constructs include humanized variants of non-human antibodies, "affinity matured" antibodies (see, e.g. Hawkins et al. J. Mol. Biol. 254, 889-896 (1992) and Lowman et al., Biochemistry 30, 10832- 10837 (1991 )) and antibody mutants with altered effector function(s) (see, e.g., US Patent 5,648,260, Kontermann and Dubel (2010), loc. cit. and Little (2009), loc. cit).

In immunology, affinity maturation is the process by which B cells produce antibodies with increased affinity for antigen during the course of an immune response. With repeated exposures to the same antigen, a host will produce antibodies of successively greater affinities. Like the natural prototype, the in vitro affinity maturation is based on the principles of mutation and selection. The in vitro affinity maturation has successfully been used to optimize antibodies, antibody constructs, and antibody fragments. Random mutations inside the CDRs are introduced using radiation, chemical mutagens or error-prone PCR. In addition, the genetic diversity can be increased by chain shuffling. Two or three rounds of mutation and selection using display methods like phage display usually results in antibody fragments with affinities in the low nanomolar range.

A preferred type of an amino acid substitutional variation of the antibody constructs involves substituting one or more hypervariable region residues of a parent antibody (e. g. a humanized or human antibody). Generally, the resulting variant(s) selected for further development will have improved biological properties relative to the parent antibody from which they are generated. A convenient way for generating such substitutional variants involves affinity maturation using phage display. Briefly, several hypervariable region sides (e. g. 6-7 sides) are mutated to generate all possible amino acid substitutions at each side. The antibody variants thus generated are displayed in a monovalent fashion from filamentous phage particles as fusions to the gene III product of M13 packaged within each particle. The phage-displayed variants are then screened for their biological activity (e. g. binding affinity) as herein disclosed. In order to identify candidate hypervariable region sides for modification, alanine scanning mutagenesis can be performed to identify hypervariable region residues contributing significantly to antigen binding. Alternatively, or additionally, it may be beneficial to analyze a crystal structure of the antigen-antibody complex to identify contact points between the binding domain and, e.g., human target cell surface antigen. Such contact residues and neighboring residues are candidates for substitution according to the techniques elaborated herein. Once such variants are generated, the panel of variants is subjected to screening as described herein and antibodies with superior properties in one or more relevant assays may be selected for further development.

The monoclonal antibodies and antibody constructs of the present invention specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is/are identical with or homologous to corresponding sequences in antibodies derived from another species or belonging to another antibody class or subclass, as well as fragments of such antibodies, so long as they exhibit the desired biological activity (U.S. Patent No. 4,816,567; Morrison et al., Proc. Natl. Acad. Sci. USA, 81 : 6851 -6855 (1984)). Chimeric antibodies of interest herein include "primitized" antibodies comprising variable domain antigen-binding sequences derived from a non-human primate (e.g., Old World Monkey, Ape etc.) and human constant region sequences. A variety of approaches for making chimeric antibodies have been described. See e.g., Morrison et al., Proc. Natl. Acad. Sci U.S.A. 81 :6851, 1985; Takeda et al., Nature 314:452, 1985, Cabilly et al., U.S. Patent No. 4,816,567; Boss et al., U.S. Patent No. 4,816,397; Tanaguchi et al., EP 0171496; EP 0173494; and GB 2177096.

An antibody, antibody construct, antibody fragment or antibody variant may also be modified by specific deletion of human T cell epitopes (a method called "deimmunization") by the methods disclosed for example in WO 98/52976 or WO 00/34317. Briefly, the heavy and light chain variable domains of an antibody can be analyzed for peptides that bind to MHC class II; these peptides represent potential T cell epitopes (as defined in WO 98/52976 and WO 00/34317). For detection of potential T cell epitopes, a computer modeling approach termed "peptide threading" can be applied, and in addition a database of human MHC class II binding peptides can be searched for motifs present in the VH and VL sequences, as described in WO 98/52976 and WO 00/34317. These motifs bind to any of the 18 major MHC class II DR allotypes, and thus constitute potential T cell epitopes. Potential T cell epitopes detected can be eliminated by substituting small numbers of amino acid residues in the variable domains, or preferably, by single amino acid substitutions. Typically, conservative substitutions are made. Often, but not exclusively, an amino acid common to a position in human germline antibody sequences may be used. Human germline sequences are disclosed e.g. in Tomlinson, et al. (1992) J. Mol. Biol. 227:776-798; Cook, G.P. et al. (1995) Immunol. Today Vol. 16 (5): 237-242; and Tomlinson et al. (1995) EMBO J. 14: 14:4628- 4638. The V BASE directory provides a comprehensive directory of human immunoglobulin variable region sequences (compiled by Tomlinson, LA. et al. MRC Centre for Protein Engineering, Cambridge, UK). These sequences can be used as a source of human sequence, e.g., for framework regions and CDRs. Consensus human framework regions can also be used, for example as described in US Patent No. 6,300,064.

"Humanized" antibodies, antibody constructs, variants or fragments thereof (such as Fv, Fab, Fab', F(ab')₂ or other antigen-binding subsequences of antibodies) are antibodies or immunoglobulins of mostly human sequences, which contain (a) minimal sequence(s) derived from non-human immunoglobulin. For the most part, humanized antibodies are human immunoglobulins (recipient antibody) in which residues from a hypervariable region (also CDR) of the recipient are replaced by residues from a hypervariable region of a non-human (e.g., rodent) species (donor antibody) such as mouse, rat, hamster or rabbit having the desired specificity, affinity, and capacity. In some instances, Fv framework region (FR) residues of the human immunoglobulin are replaced by corresponding non-human residues. Furthermore, "humanized antibodies" as used herein may also comprise residues which are found neither in the recipient antibody nor the donor antibody. These modifications are made to further refine and optimize antibody performance. The humanized antibody may also comprise at least a portion of an immunoglobulin constant region (Fc), typically that of a human immunoglobulin. For further details, see Jones et al., Nature, 321: 522-525 (1986); Reichmann et al., Nature, 332: 323-329 (1988); and Presta, Curr. Op. Struct. Biol., 2: 593- 596 (1992).

Humanized antibodies or fragments thereof can be generated by replacing sequences of the Fv variable domain that are not directly involved in antigen binding with equivalent sequences from human Fv variable domains. Exemplary methods for generating humanized antibodies or fragments thereof are provided by Morrison (1985) Science 229:1202-1207; by Oi et al. (1986) BioTechniques 4:214; and by US 5,585,089; US 5,693,761; US 5,693,762; US 5,859,205; and US 6,407,213. Those methods include isolating, manipulating, and expressing the nucleic acid sequences that encode all or part of immunoglobulin Fv variable domains from at least one of a heavy or light chain. Such nucleic acids may be obtained from a hybridoma producing an antibody against a predetermined target, as described above, as well as from other sources. The recombinant DNA encoding the humanized antibody molecule can then be cloned into an appropriate expression vector.

Humanized antibodies may also be produced using transgenic animals such as mice that express human heavy and light chain genes, but are incapable of expressing the endogenous mouse immunoglobulin heavy and light chain genes. Winter describes an exemplary CDR grafting method that may be used to prepare the humanized antibodies described herein (U.S. Patent No. 5,225,539). All of the CDRs of a particular human antibody may be replaced with at least a portion of a non-human CDR, or only some of the CDRs may be replaced with non-human CDRs. It is only necessary to replace the number of CDRs required for binding of the humanized antibody to a predetermined antigen.

A humanized antibody can be optimized by the introduction of conservative substitutions, consensus sequence substitutions, germline substitutions and/or back mutations. Such altered immunoglobulin molecules can be made by any of several techniques known in the art, (e.g., Teng et al., Proc. Natl. Acad. Sci. U.S.A., 80: 7308-7312, 1983; Kozbor ei a/., Immunology Today, 4: 7279, 1983; Olsson et al., Meth. Enzymol., 92: 3- 16, 1982, and EP 239 400).

The term "human antibody", "human antibody construct" and "human binding domain" includes antibodies, antibody constructs and binding domains having antibody regions such as variable and constant regions or domains which correspond substantially to human germline immunoglobulin sequences known in the art, including, for example, those described by Kabat et al. (1991) (loc. cit.). The human antibodies, antibody constructs or binding domains as defined in the context of the invention may include amino acid residues not encoded by human germline immunoglobulin sequences (e.g., mutations introduced by random or side-specific mutagenesis in vitro or by somatic mutation in vivo), for example in the CDRs, and in particular, in CDR3. The human antibodies, antibody constructs or binding domains can have at least one, two, three, four, five, or more positions replaced with an amino acid residue that is not encoded by the human germline immunoglobulin sequence. The definition of human antibodies, antibody constructs and binding domains as used herein, however, also contemplates "fully human antibodies", which include only non-artificially and/or genetically altered human sequences of antibodies as those can be derived by using technologies or systems such as the Xenomouse. Preferably, a "fully human antibody" does not include amino acid residues not encoded by human germline immunoglobulin sequences.

In some embodiments, the antibody constructs defined herein are "isolated" or "substantially pure" antibody constructs. "Isolated" or "substantially pure", when used to describe the antibody constructs disclosed herein, means an antibody construct that has been identified, separated and/or recovered from a component of its production environment. Preferably, the antibody construct is free or substantially free of association with all other components from its production environment. Contaminant components of its production environment, such as that resulting from recombinant transfected cells, are materials that would typically interfere with diagnostic or therapeutic uses for the polypeptide, and may include enzymes, hormones, and other proteinaceous or non-proteinaceous solutes. The antibody constructs may e.g constitute at least about 5%, or at least about 50% by weight of the total protein in a given sample. It is understood that the isolated protein may constitute from 5% to 99.9% by weight of the total protein content, depending on the circumstances. The polypeptide may be made at a significantly higher concentration through the use of an inducible promoter or high expression promoter, such that it is made at increased concentration levels. The definition includes the production of an antibody construct in a wide variety of organisms and/or host cells that are known in the art. In preferred embodiments, the antibody construct will be purified (1) to a degree sufficient to obtain at least 15 residues of N-terminal or internal amino acid sequence by use of a spinning cup sequenator, or (2) to homogeneity by SDS-PAGE under non-reducing or reducing conditions using Coomassie blue or, preferably, silver stain. Ordinarily, however, an isolated antibody construct will be prepared by at least one purification step.

The term "binding domain" characterizes in connection with the present invention a domain which (specifically) binds to / interacts with / recognizes a given target epitope or a given target side on the target molecules (antigens), e.g. a NK cell receptor antigen, e.g. CD16, and a target cell surface antigen, respectively. The structure and function of the first binding domain (recognizing e.g. CD16), and preferably also the structure and/or function of the second binding domain (recognizing the target cell surface antigen), is/are based on the structure and/or function of an antibody, e.g. of a full-length or whole immunoglobulin molecule and/or is/are drawn from the variable heavy chain (VH) and/or variable light chain (VL) domains of an antibody or fragment thereof. The first binding domain is characterized by the presence of three light chain CDRs (i.e. CDR1, CDR2 and CDR3 of the VL region) and three heavy chain CDRs (i.e. CDR1, CDR2 and CDR3 of the VH region). The second binding domain preferably also comprises the minimum structural requirements of an antibody which allow for the target binding. More preferably, the second binding domain comprises at least three light chain CDRs (i.e. CDR1, CDR2 and CDR3 of the VL region) and/or three heavy chain CDRs (i.e. CDR1, CDR2 and CDR3 of the VH region). It is envisaged that the first and/or second binding domain is produced by or obtainable by phage-display or library screening methods rather than by grafting CDR sequences from a pre-existing (monoclonal) antibody into a scaffold.

According to the present invention, binding domains are in the form of one or more polypeptides. Such polypeptides may include proteinaceous parts and non-proteinaceous parts (e.g. chemical linkers or chemical cross-linking agents such as glutaraldehyde). Proteins (including fragments thereof, preferably biologically active fragments, and peptides, usually having less than 30 amino acids) comprise two or more amino acids coupled to each other via a covalent peptide bond (resulting in a chain of amino acids).

The term "polypeptide" as used herein describes a group of molecules, which usually consist of more than 30 amino acids. Polypeptides may further form multimers such as dimers, trimers and higher oligomers, i.e., consisting of more than one polypeptide molecule. Polypeptide molecules forming such dimers, trimers etc. may be identical or non-identical. The corresponding higher order structures of such multimers are, consequently, termed homo- or heterodimers, homo- or heterotrimers etc. An example for a heteromultimer is an antibody molecule, which, in its naturally occurring form, consists of two identical light polypeptide chains and two identical heavy polypeptide chains. The terms "peptide", "polypeptide" and "protein" also referto naturally modified peptides / polypeptides / proteins wherein the modification is affected e.g. by post-translational modifications like glycosylation, acetylation, phosphorylation and the like. A "peptide", "polypeptide" or "protein" when referred to herein may also be chemically modified such as pegylated. Such modifications are well known in the art and described herein below.

Preferably the binding domain which binds to the NK cell receptor antigen, e.g. CD16 and/or the binding domain which binds to the target cell surface antigen is/are human binding domains. Antibodies and antibody constructs comprising at least one human binding domain avoid some of the problems associated with antibodies or antibody constructs that possess non-human such as rodent (e.g. murine, rat, hamster or rabbit) variable and/or constant regions. The presence of such rodent derived proteins can lead to the rapid clearance of the antibodies or antibody constructs or can lead to the generation of an immune response against the antibody or antibody construct by a patient. In order to avoid the use of rodent derived antibodies or antibody constructs, human or fully human antibodies / antibody constructs can be generated through the introduction of human antibody function into a rodent so that the rodent produces fully human antibodies.

The ability to clone and reconstruct megabase-sized human loci in YACs and to introduce them into the mouse germline provides a powerful approach to elucidating the functional components of very large or crudely mapped loci as well as generating useful models of human disease. Furthermore, the use of such technology for substitution of mouse loci with their human equivalents could provide unique insights into the expression and regulation of human gene products during development, their communication with other systems, and their involvement in disease induction and progression.

An important practical application of such a strategy is the "humanization" of the mouse humoral immune system. Introduction of human immunoglobulin (Ig) loci into mice in which the endogenous Ig genes have been inactivated offers the opportunity to study the mechanisms underlying programmed expression and assembly of antibodies as well as their role in B-cell development. Furthermore, such a strategy could provide an ideal source for production of fully human monoclonal antibodies (mAbs) - an important milestone towards fulfilling the promise of antibody therapy in human disease. Fully human antibodies or antibody constructs are expected to minimize the immunogenic and allergic responses intrinsic to mouse or mouse-derivatized mAbs and thus to increase the efficacy and safety of the administered antibodies / antibody constructs. The use of fully human antibodies or antibody constructs can be expected to provide a substantial advantage in the treatment of chronic and recurring human diseases, such as inflammation, autoimmunity, and cancer, which require repeated compound administrations.

One approach towards this goal was to engineer mouse strains deficient in mouse antibody production with large fragments of the human Ig loci in anticipation that such mice would produce a large repertoire of human antibodies in the absence of mouse antibodies. Large human Ig fragments would preserve the large variable gene diversity as well as the proper regulation of antibody production and expression. By exploiting the mouse machinery for antibody diversification and selection and the lack of immunological tolerance to human proteins, the reproduced human antibody repertoire in these mouse strains should yield high affinity antibodies against any antigen of interest, including human antigens. Using the hybridoma technology, antigen-specific human mAbs with the desired specificity could be readily produced and selected. This general strategy was demonstrated in connection with the generation of the first XenoMouse mouse strains (see Green et al. Nature Genetics 7:13-21 (1994)). The XenoMouse strains were engineered with yeast artificial chromosomes (YACs) containing 245 kb and 190 kb-sized germline configuration fragments of the human heavy chain locus and kappa light chain locus, respectively, which contained core variable and constant region sequences. The human Ig containing YACs proved to be compatible with the mouse system for both rearrangement and expression of antibodies and were capable of substituting for the inactivated mouse Ig genes. This was demonstrated by their ability to induce B cell development, to produce an adult-like human repertoire of fully human antibodies, and to generate antigen-specific human mAbs. These results also suggested that introduction of larger portions of the human Ig loci containing greater numbers of V genes, additional regulatory elements, and human Ig constant regions might recapitulate substantially the full repertoire that is characteristic of the human humoral response to infection and immunization. The work of Green et al. was recently extended to the introduction of greater than approximately 80% of the human antibody repertoire through introduction of megabase sized, germline configuration YAC fragments of the human heavy chain loci and kappa light chain loci, respectively. See Mendez et al. Nature Genetics 15:146-156 (1997) and U.S. patent application Ser. No. 08/759,620.

The production of the XenoMouse mice is further discussed and delineated in U.S. patent applications Ser. No. 07/466,008, Ser. No. 07/610,515, Ser. No. 07/919,297, Ser. No. 07/922,649, Ser. No. 08/031,801, Ser. No. 08/1 12,848, Ser. No. 08/234,145, Ser. No. 08/376,279, Ser. No. 08/430,938, Ser. No. 08/464,584, Ser. No. 08/464,582, Ser. No. 08/463,191, Ser. No. 08/462,837, Ser. No. 08/486,853, Ser. No. 08/486,857, Ser. No. 08/486,859, Ser. No. 08/462,513, Ser. No. 08/724,752, and Ser. No. 08/759,620; and U.S. Pat. Nos. 6,162,963; 6,150,584; 6,1 14,598; 6,075,181, and 5,939,598 and Japanese Patent Nos. 3 068 180 B2, 3 068 506 B2, and 3 068 507 B2. See also Mendez et al. Nature Genetics 15:146-156 (1997) and Green and Jakobovits J. Exp. Med. 188:483-495 (1998), EP 0 463 151 B1, WO 94/02602, WO 96/34096, WO 98/24893, WO 00/76310, and WO 03/47336.

In an alternative approach, others, including GenPharm International, Inc., have utilized a "minilocus" approach. In the minilocus approach, an exogenous Ig locus is mimicked through the inclusion of pieces (individual genes) from the Ig locus. Thus, one or more VH genes, one or more DH genes, one or more JH genes, a mu constant region, and a second constant region (preferably a gamma constant region) are formed into a construct for insertion into an animal. This approach is described in U.S. Pat. No. 5,545,807 to Surani et al. and U.S. Pat. Nos. 5,545,806; 5,625,825; 5,625,126; 5,633,425; 5,661,016; 5,770,429; 5,789,650; 5,814,318; 5,877,397; 5,874,299; and 6,255,458 each to Lonberg and Kay, U.S. Pat. Nos. 5,591,669 and 6,023.010 to Krimpenfort and Berns, U.S. Pat. Nos. 5,612,205; 5,721,367; and 5,789,215 to Berns et al., and U.S. Pat. No. 5,643,763 to Choi and Dunn, and GenPharm International U.S. patent application Ser. No. 07/574,748, Ser. No. 07/575,962, Ser. No. 07/810,279, Ser. No. 07/853,408, Ser. No. 07/904,068, Ser. No. 07/990,860, Ser. No. 08/053,131, Ser. No. 08/096,762, Ser. No. 08/155,301, Ser. No. 08/161,739, Ser. No. 08/165,699, Ser. No. 08/209,741. See also EP 0 546 073 B1, WO 92/03918, WO 92/22645, WO 92/22647, WO 92/22670, WO 93/12227, WO 94/00569, WO 94/25585, WO 96/14436, WO 97/13852, and WO 98/24884 and U.S. Pat. No. 5,981,175. See further Taylor et al. (1992), Chen et al. (1993), Tuaillon et al. (1993), Choi et al. (1993), Lonberg et al. (1994), Taylor et al. (1994), and Tuaillon et al. (1995), Fishwild et al. (1996).

Kirin has also demonstrated the generation of human antibodies from mice in which, through microcell fusion, large pieces of chromosomes, or entire chromosomes, have been introduced. See European Patent Application Nos. 773 288 and 843 961. Xenerex Biosciences is developing a technology for the potential generation of human antibodies. In this technology, SCID mice are reconstituted with human lymphatic cells, e.g., B and/or T cells.

Mice are then immunized with an antigen and can generate an immune response against the antigen. See U.S. Pat. Nos. 5,476,996; 5,698,767; and 5,958,765.

Human anti-mouse antibody (HAMA) responses have led the industry to prepare chimeric or otherwise humanized antibodies. It is however expected that certain human anti- chimeric antibody (HACA) responses will be observed, particularly in chronic or multi-dose utilizations of the antibody. Thus, it would be desirable to provide antibody constructs comprising a human binding domain against the target cell surface antigen and a human binding domain against CD16 in order to vitiate concerns and/or effects of HAMA or HACA response.

The terms "(specifically) binds to", (specifically) recognizes", "is (specifically) directed to", and "(specifically) reacts with" mean in accordance with this invention that a binding domain interacts or specifically interacts with a given epitope or a given target side on the target molecules (antigens), here: the NK cell receptor, e.g. CD16a, and the target cell surface antigen, respectively.

The term "epitope" refers to a side on an antigen to which a binding domain, such as an antibody or immunoglobulin, or a derivative, fragment or variant of an antibody or an immunoglobulin, specifically binds. An "epitope" is antigenic and thus the term epitope is sometimes also referred to herein as "antigenic structure" or "antigenic determinant". Thus, the binding domain is an "antigen interaction side". Said binding/interaction is also understood to define a "specific recognition".

"Epitopes" can be formed both by contiguous amino acids or non-contiguous amino acids juxtaposed by tertiary folding of a protein. A "linear epitope" is an epitope where an amino acid primary sequence comprises the recognized epitope. A linear epitope typically includes at least 3 or at least 4, and more usually, at least 5 or at least 6 or at least 7, for example, about 8 to about 10 amino acids in a unique sequence.

A "conformational epitope", in contrast to a linear epitope, is an epitope wherein the primary sequence of the amino acids comprising the epitope is not the sole defining component of the epitope recognized (e.g., an epitope wherein the primary sequence of amino acids is not necessarily recognized by the binding domain). Typically, a conformational epitope comprises an increased number of amino acids relative to a linear epitope. With regard to recognition of conformational epitopes, the binding domain recognizes a three-dimensional structure of the antigen, preferably a peptide or protein or fragment thereof (in the context of the present invention, the antigenic structure for one of the binding domains is comprised within the target cell surface antigen protein). For example, when a protein molecule folds to form a three-dimensional structure, certain amino acids and/or the polypeptide backbone forming the conformational epitope become juxtaposed enabling the antibody to recognize the epitope. Methods of determining the conformation of epitopes include, but are not limited to, x-ray crystallography, two-dimensional nuclear magnetic resonance (2D-NMR) spectroscopy and site-directed spin labelling and electron paramagnetic resonance (EPR) spectroscopy.

The interaction between the binding domain and the epitope or the region comprising the epitope implies that a binding domain exhibits appreciable affinity for the epitope / the region comprising the epitope on a particular protein or antigen (here: the NK cell receptor, e.g. CD16a, and the target cell surface antigen, respectively) and, generally, does not exhibit significant reactivity with proteins or antigens other than the NK cell receptor, e.g. CD16a, and the target cell surface antigen. "Appreciable affinity" includes binding with an affinity of about 106 M (KD) or stronger. Preferably, binding is considered specific when the binding affinity is about 10-12 to 10-8 M, 10-12 to 10-9 M, 10-12 to 10-10 M, 10-11 to 10-8 M, preferably of about 10-11 to 10-9 M. Whether a binding domain specifically reacts with or binds to a target can be tested readily by, inter alia, comparing the reaction of said binding domain with a target protein or antigen with the reaction of said binding domain with proteins or antigens other than the NK cell receptor, e.g. CD16a, and the target cell surface antigen. Preferably, a binding domain as defined in the context of the invention does not essentially or substantially bind to proteins or antigens other than the NK cell receptor, e.g. CD16a, and the target cell surface antigen (i.e., the first binding domain is not capable of binding to proteins other than the NK cell receptor, e.g. CD16a, and the second binding domain is not capable of binding to proteins other than the target cell surface antigen).

The term "does not essentially / substantially bind" or "is not capable of binding" means that a binding domain of the present invention does not bind a protein or antigen other than the NK cell receptor, e.g. CD16a, and the target cell surface antigen, i.e., does not show reactivity of more than 30%, preferably not more than 20%, more preferably not more than 10%, particularly preferably not more than 9%, 8%, 7%, 6% or 5% with proteins or antigens other than the NK cell receptor, e.g. CD16a, and the target cell surface antigen, whereby binding to the NK cell receptor, e.g. CD16a, and the target cell surface antigen, respectively, is set to be 100%.

Specific binding is believed to be affected by specific motifs in the amino acid sequence of the binding domain and the antigen. Thus, binding is achieved as a result of their primary, secondary and/or tertiary structure as well as the result of secondary modifications of said structures. The specific interaction of the antigen-interaction-side with its specific antigen may result in a simple binding of said side to the antigen. Moreover, the specific interaction of the antigen-interaction-side with its specific antigen may alternatively or additionally result in the initiation of a signal, e.g. due to the induction of a change of the conformation of the antigen, an oligomerization of the antigen, etc.

The term "variable" refers to the portions of the antibody or immunoglobulin domains that exhibit variability in their sequence and that are involved in determining the specificity and binding affinity of a particular antibody (i.e., the "variable domain(s)"). The pairing of a variable heavy chain (VH) and a variable light chain (VL) together forms a single antigen-binding side.

Variability is not evenly distributed throughout the variable domains of antibodies; it is concentrated in sub-domains of each of the heavy and light chain variable regions. These sub-domains are called "hypervariable regions" or "complementarity determining regions" (CDRs). The more conserved (i.e., non-hypervariable) portions of the variable domains are called the "framework" regions (FRM or FR) and provide a scaffold for the six CDRs in three dimensional space to form an antigen-binding surface. The variable domains of naturally occurring heavy and light chains each comprise four FRM regions (FR1, FR2, FR3, and FR4), largely adopting a β-sheet configuration, connected by three hypervariable regions, which form loops connecting, and in some cases forming part of, the β-sheet structure. The hypervariable regions in each chain are held together in close proximity by the FRM and, with the hypervariable regions from the other chain, contribute to the formation of the antigen-binding side (see Kabat et al., loc. cit.).

The terms "CDR", and its plural "CDRs", refer to the complementarity determining region of which three make up the binding character of a light chain variable region (CDR-L1, CDR-L2 and CDR-L3) and three make up the binding character of a heavy chain variable region (CDR-H1, CDR-H2 and CDR-H3). CDRs contain most of the residues responsible for specific interactions of the antibody with the antigen and hence contribute to the functional activity of an antibody molecule: they are the main determinants of antigen specificity.

The exact definitional CDR boundaries and lengths are subject to different classification and numbering systems. CDRs may therefore be referred to by Kabat, Chothia, contact or any other boundary definitions, including the numbering system described herein. Despite differing boundaries, each of these systems has some degree of overlap in what constitutes the so called "hypervariable regions" within the variable sequences. CDR definitions according to these systems may therefore differ in length and boundary areas with respect to the adjacent framework region. See for example Kabat (an approach based on cross-species sequence variability), Chothia (an approach based on crystallographic studies of antigen-antibody complexes), and/or MacCallum (Kabat et al., loc. cit; Chothia et al., J. Mol. Biol, 1987, 196: 901 -917; and MacCallum et al., J. Mol. Biol, 1996, 262: 732). Still another standard for characterizing the antigen binding side is the AbM definition used by Oxford Molecular's AbM antibody modeling software. See, e.g., Protein Sequence and Structure Analysis of Antibody Variable Domains. In: Antibody Engineering Lab Manual (Ed.: Duebel, S. and Kontermann, R., Springer-Verlag, Heidelberg). To the extent that two residue identification techniques define regions of overlapping, but not identical regions, they can be combined to define a hybrid CDR. However, the numbering in accordance with the so-called Kabat system is preferred.

Typically, CDRs form a loop structure that can be classified as a canonical structure. The term "canonical structure" refers to the main chain conformation that is adopted by the antigen binding (CDR) loops. From comparative structural studies, it has been found that five of the six antigen binding loops have only a limited repertoire of available conformations. Each canonical structure can be characterized by the torsion angles of the polypeptide backbone. Correspondent loops between antibodies may, therefore, have very similar three dimensional structures, despite high amino acid sequence variability in most parts of the loops (Chothia and Lesk, J. Mol. Biol., 1987, 196: 901; Chothia et al., Nature, 1989, 342: 877; Martin and Thornton, J. Mol. Biol, 1996, 263: 800). Furthermore, there is a relationship between the adopted loop structure and the amino acid sequences surrounding it. The conformation of a particular canonical class is determined by the length of the loop and the amino acid residues residing at key positions within the loop, as well as within the conserved framework (i.e., outside of the loop). Assignment to a particular canonical class can therefore be made based on the presence of these key amino acid residues.

The term "canonical structure" may also include considerations as to the linear sequence of the antibody, for example, as catalogued by Kabat (Kabat et al., loc. cit.). The Kabat numbering scheme (system) is a widely adopted standard for numbering the amino acid residues of an antibody variable domain in a consistent manner and is the preferred scheme applied in the present invention as also mentioned elsewhere herein. Additional structural considerations can also be used to determine the canonical structure of an antibody. For example, those differences not fully reflected by Kabat numbering can be described by the numbering system of Chothia et al. and/or revealed by other techniques, for example, crystallography and two- or three-dimensional computational modeling. Accordingly, a given antibody sequence may be placed into a canonical class which allows for, among other things, identifying appropriate chassis sequences (e.g., based on a desire to include a variety of canonical structures in a library). Kabat numbering of antibody amino acid sequences and structural considerations as described by Chothia et al., loc. cit. and their implications for construing canonical aspects of antibody structure, are described in the literature. The subunit structures and three-dimensional configurations of different classes of immunoglobulins are well known in the art. For a review of the antibody structure, see Antibodies: A Laboratory Manual, Cold Spring Harbor Laboratory, eds. Harlow et al., 1988. A global reference in immunoinformatics is the three-dimensional (3D) structure database of IMGT (international ImMunoGenetics information system) (Ehrenmann et al., 2010, Nucleic Acids Res., 38, D301-307). The IMGT/3Dstructure-DB structural data are extracted from the Protein Data Bank (PDB) and annotated according to the IMGT concepts of classification, using internal tools. Thus, IMGT/3Dstructure-DB provides the closest genes and alleles that are expressed in the amino acid sequences of the 3D structures, by aligning these sequences with the IMGT domain reference directory. This directory contains, for the antigen receptors, amino acid sequences of the domains encoded by the constant genes and the translation of the germline variable and joining genes. The CDR regions of our amino acid sequences were preferably determined by using the IMGT/3Dstructure database.

The CDR3 of the light chain and, particularly, the CDR3 of the heavy chain may constitute the most important determinants in antigen binding within the light and heavy chain variable regions. In some antibody constructs, the heavy chain CDR3 appears to constitute the major area of contact between the antigen and the antibody. In vitro selection schemes in which CDR3 alone is varied can be used to vary the binding properties of an antibody or determine which residues contribute to the binding of an antigen. Hence, CDR3 is typically the greatest source of molecular diversity within the antibody-binding side. H3, for example, can be as short as two amino acid residues or greater than 26 amino acids.

In a classical full-length antibody or immunoglobulin, each light (L) chain is linked to a heavy (H) chain by one covalent disulfide bond, while the two H chains are linked to each other by one or more disulfide bonds depending on the H chain isotype. The CH domain most proximal to VH is usually designated as CH1. The constant ("C") domains are not directly involved in antigen binding, but exhibit various effector functions, such as antibody-dependent, cell-mediated cytotoxicity and complement activation. The Fc region of an antibody is comprised within the heavy chain constant domains and is for example able to interact with cell surface located Fc receptors.

The sequence of antibody genes after assembly and somatic mutation is highly varied, and these varied genes are estimated to encode 10¹⁰ different antibody molecules (Immunoglobulin Genes, 2nd ed., eds. Jonio et al., Academic Press, San Diego, CA, 1995). Accordingly, the immune system provides a repertoire of immunoglobulins. The term "repertoire" refers to at least one nucleotide sequence derived wholly or partially from at least one sequence encoding at least one immunoglobulin. The sequence(s) may be generated by rearrangement in vivo of the V, D, and J segments of heavy chains, and the V and J segments of light chains. Alternatively, the sequence(s) can be generated from a cell in response to which rearrangement occurs, e.g., in vitro stimulation. Alternatively, part or all of the sequence(s) may be obtained by DNA splicing, nucleotide synthesis, mutagenesis, and other methods, see, e.g., U.S. Patent 5,565,332. A repertoire may include only one sequence or may include a plurality of sequences, including ones in a genetically diverse collection.

The antibody construct defined in the context of the invention may also comprise additional domains, which are e.g. helpful in the isolation of the molecule or relate to an adapted pharmacokinetic profile of the molecule. Domains helpful for the isolation of an antibody construct may be selected from peptide motives or secondarily introduced moieties, which can be captured in an isolation method, e.g. an isolation column. Non-limiting embodiments of such additional domains comprise peptide motives known as Myc-tag, HAT-tag, HA-tag, TAP-tag, GST-tag, chitin binding domain (CBD-tag), maltose binding protein (MBP-tag), Flag-tag, Strep-tag and variants thereof (e.g. Strepll-tag) and His-tag. All herein disclosed antibody constructs characterized by the identified CDRs may comprise a His-tag domain, which is generally known as a repeat of consecutive His residues in the amino acid sequence of a molecule, preferably of five, and more preferably of six His residues (hexa-histidine). The His-tag may be located e.g. at the N- or C-terminus of the antibody construct, preferably it is located at the C-terminus. Most preferably, a hexa-histidine tag (HHHHHH) (SEQ ID NO:25) is linked via peptide bond to the C-terminus of the antibody construct according to the invention. Additionally, a conjugate system of PLGA-PEG-PLGA may be combined with a poly-histidine tag for sustained release application and improved pharmacokinetic profile.

Amino acid sequence modifications of the antibody constructs described herein are also contemplated. For example, it may be desirable to improve the binding affinity and/or other biological properties of the antibody construct. Amino acid sequence variants of the antibody constructs are prepared by introducing appropriate nucleotide changes into the antibody constructs nucleic acid, or by peptide synthesis. All of the below described amino acid sequence modifications should result in an antibody construct which still retains the desired biological activity (binding to the NK cell receptor, e.g. CD16a, and the target cell surface antigen) of the unmodified parental molecule.

The term "amino acid" or "amino acid residue" typically refers to an amino acid having its art recognized definition such as an amino acid selected from the group consisting of: alanine (Ala or A); arginine (Arg or R); asparagine (Asn or N); aspartic acid (Asp or D); cysteine (Cys or C); glutamine (Gin or Q); glutamic acid (Glu or E); glycine (Gly or G); histidine (His or H); isoleucine (He or I): leucine (Leu or L); lysine (Lys or K); methionine (Met or M); phenylalanine (Phe or F); pro line (Pro or P); serine (Ser or S); threonine (Thr or T); tryptophan (Trp or W); tyrosine (Tyr or Y); and valine (Val or V), although modified, synthetic, or rare amino acids may be used as desired. Generally, amino acids can be grouped as having a nonpolar side chain (e.g., Ala, Cys, He, Leu, Met, Phe, Pro, Val); a negatively charged side chain (e.g., Asp, Glu); a positively charged sidechain (e.g., Arg, His, Lys); or an uncharged polar side chain (e.g., Asn, Cys, Gin, Gly, His, Met, Phe, Ser, Thr, Trp, and Tyr).

Amino acid modifications include, for example, deletions from, and/or insertions into, and/or substitutions of, residues within the amino acid sequences of the antibody constructs. Any combination of deletion, insertion, and substitution is made to arrive at the final construct, provided that the final construct possesses the desired characteristics. The amino acid changes also may alter post-translational processes of the antibody constructs, such as changing the number or position of glycosylation sites.

For example, 1, 2, 3, 4, 5, or 6 amino acids may be inserted, substituted or deleted in each of the CDRs (of course, dependent on their length), while 1, 2, 3, 4, 5, 6, 7, 8, 9, 10, 11, 12, 13, 14, 15, 16, 17, 18, 19, 20, or 25 amino acids may be inserted, substituted or deleted in each of the FRs. Preferably, amino acid sequence insertions into the antibody construct include amino- and/or carboxyl-terminal fusions ranging in length from 1, 2, 3, 4, 5, 6, 7, 8, 9 or 10 residues to polypeptides containing a hundred or more residues, as well as intra- sequence insertions of single or multiple amino acid residues. Corresponding modifications may also performed within a third domain of the antibody construct defined in the context of the invention. An insertional variant of the antibody construct defined in the context of the invention includes the fusion to the N- terminus or to the C-terminus of the antibody construct of an enzyme or the fusion to a polypeptide.

The sites of greatest interest for substitutional mutagenesis include (but are not limited to) the CDRs of the heavy and/or light chain, in particular the hypervariable regions, but FR alterations in the heavy and/or light chain are also contemplated. The substitutions are preferably conservative substitutions as described herein. Preferably, 1, 2, 3, 4, 5, 6, 7, 8, 9, or 10 amino acids may be substituted in a CDR, while 1, 2, 3, 4, 5, 6, 7, 8, 9, 10, 11, 12, 13, 14, 15, 16, 17, 18, 19, 20, or 25 amino acids may be substituted in the framework regions (FRs), depending on the length of the CDR or FR. For example, if a CDR sequence encompasses 6 amino acids, it is envisaged that one, two or three of these amino acids are substituted. Similarly, if a CDR sequence encompasses 15 amino acids it is envisaged that one, two, three, four, five or six of these amino acids are substituted.

A useful method for identification of certain residues or regions of the antibody constructs that are preferred locations for mutagenesis is called "alanine scanning mutagenesis" as described by Cunningham and Wells in Science, 244: 1081 -1085 (1989). Here, a residue or group of target residues within the antibody construct is/are identified (e.g. charged residues such as arg, asp, his, lys, and glu) and replaced by a neutral or negatively charged amino acid (most preferably alanine or polyalanine) to affect the interaction of the amino acids with the epitope.

Those amino acid locations demonstrating functional sensitivity to the substitutions are then refined by introducing further or other variants at, or for, the sites of substitution. Thus, while the site or region for introducing an amino acid sequence variation is predetermined, the nature of the mutation per se needs not to be predetermined. For example, to analyze or optimize the performance of a mutation at a given site, alanine scanning or random mutagenesis may be conducted at a target codon or region, and the expressed antibody construct variants are screened for the optimal combination of desired activity. Techniques for making substitution mutations at predetermined sites in the DNA having a known sequence are well known, for example, M13 primer mutagenesis and PCR mutagenesis. Screening of the mutants is done using assays of antigen binding activities, such as the NK cell receptor, e.g. CD16a, and the target cell surface antigen binding.

Generally, if amino acids are substituted in one or more or all of the CDRs of the heavy and/or light chain, it is preferred that the then-obtained "substituted" sequence is at least 60% or 65%, more preferably 70% or 75%, even more preferably 80% or 85%, and particularly preferably 90% or 95% identical to the "original" CDR sequence. This means that it is dependent of the length of the CDR to which degree it is identical to the "substituted" sequence. For example, a CDR having 5 amino acids is preferably 80% identical to its substituted sequence in order to have at least one amino acid substituted. Accordingly, the CDRs of the antibody construct may have different degrees of identity to their substituted sequences, e.g., CDRL1 may have 80%, while CDRL3 may have 90%.

Preferred substitutions (or replacements) are conservative substitutions. However, any substitution (including non-conservative substitution or one or more from the "exemplary substitutions" listed in Table 3, below) is envisaged as long as the antibody construct retains its capability to bind to the NK cell receptor, e.g. CD16a via the first domain and to the target cell surface antigen via the second domain and/or its CDRs have an identity to the then substituted sequence (at least 60% or 65%, more preferably 70% or 75%, even more preferably 80% or 85%, and particularly preferably 90% or 95% identical to the "original" CDR sequence).

Conservative substitutions are shown in Table 1 under the heading of "preferred substitutions". If such substitutions result in a change in biological activity, then more substantial changes, denominated "exemplary substitutions" in Table 1, or as further described below in reference to amino acid classes, may be introduced and the products screened for a desired characteristic.

**Table 1: Amino acid substitutions**

| **Original** | **Exemplary Substitutions** | **Preferred Substitutions** |
|---|---|---|
| Ala (A) | val, leu, ile | val |
| Arg (R) | lys, gln, asn | lys |
| Asn (N) | gln, his, asp, lys, arg | gln |
| Asp (D) | glu, asn | glu |
| Cys (C) | ser, ala | ser |
| Gln (Q) | asn, glu | asn |
| Glu (E) | asp, gln | asp |
| Gly (G) | ala | ala |
| His (H) | asn, gln, lys, arg | arg |
| Ile(I) | leu, val, met, ala, phe | leu |
| Leu (L) | norleucine, ile, val, met, ala | lie |
| Lys (K) | arg, gln, asn | arg |
| Met (M) | leu, phe, ile | leu |
| Phe (F) | leu, val, ile, ala, tyr | tyr |
| Pro (P) | ala | ala |
| Ser (S) | thr | thr |
| Thr (T) | ser | ser |
| Trp (W) | tyr, phe | tyr |
| Tyr (Y) | trp, phe, thr, ser | phe |
| Val (V) | ile, leu, met, phe, ala | leu |

Substantial modifications in the biological properties of the antibody construct of the present invention are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Naturally occurring residues are divided into groups based on common side-chain properties: (1) hydrophobic: norleucine, met, ala, val, leu, ile; (2) neutral hydrophilic: cys, ser, thr, asn, gin; (3) acidic: asp, glu; (4) basic: his, lys, arg; (5) residues that influence chain orientation: gly, pro; and (6) aromatic: trp, tyr, phe.

Non-conservative substitutions will entail exchanging a member of one of these classes for another class. Any cysteine residue not involved in maintaining the proper conformation of the antibody construct may be substituted, generally with serine, to improve the oxidative stability of the molecule and prevent aberrant crosslinking. Conversely, cysteine bond(s) may be added to the antibody to improve its stability (particularly where the antibody is an antibody fragment such as an Fv fragment).

For amino acid sequences, sequence identity and/or similarity is determined by using standard techniques known in the art, including, but not limited to, the local sequence identity algorithm of Smith and Waterman, 1981, Adv. Appl. Math. 2:482, the sequence identity alignment algorithm of Needleman and Wunsch, 1970, J. Mol. Biol. 48:443, the search for similarity method of Pearson and Lipman, 1988, Proc. Nat. Acad. Sci. U.S.A. 85:2444, computerized implementations of these algorithms (GAP, BESTFIT, FASTA, and TFASTA in the Wisconsin Genetics Software Package, Genetics Computer Group, 575 Science Drive, Madison, Wis.), the Best Fit sequence program described by Devereux et al., 1984, Nucl. Acid Res. 12:387-395, preferably using the default settings, or by inspection.

Preferably, percent identity is calculated by FastDB based upon the following parameters: mismatch penalty of 1; gap penalty of 1; gap size penalty of 0.33; and joining penalty of 30, "Current Methods in Sequence Comparison and Analysis," Macromolecule Sequencing and Synthesis, Selected Methods and Applications, pp 127-149 (1988), Alan R. Liss, Inc.

An example of a useful algorithm is PILEUP. PILEUP creates a multiple sequence alignment from a group of related sequences using progressive, pairwise alignments. It can also plot a tree showing the clustering relationships used to create the alignment. PILEUP uses a simplification of the progressive alignment method of Feng & Doolittle, 1987, J. Mol. Evol. 35:351-360; the method is similar to that described by Higgins and Sharp, 1989, CABIOS 5:151 -153. Useful PILEUP parameters including a default gap weight of 3.00, a default gap length weight of 0.10, and weighted end gaps.

Another example of a useful algorithm is the BLAST algorithm, described in: Altschul et al., 1990, J. Mol. Biol. 215:403-410; Altschul et al., 1997, Nucleic Acids Res. 25:3389- 3402; and Karin et al., 1993, Proc. Natl. Acad. Sci. U.S.A. 90:5873-5787. A particularly useful BLAST program is the WU-BLAST-2 program which was obtained from Altschul et al., 1996, Methods in Enzymology 266:460-480. WU-BLAST-2 uses several search parameters, most of which are set to the default values. The adjustable parameters are set with the following values: overlap span=1, overlap fraction=0.125, word threshold (T)=ll. The HSP S and HSP S2 parameters are dynamic values and are established by the program itself depending upon the composition of the particular sequence and composition of the particular database against which the sequence of interest is being searched; however, the values may be adjusted to increase sensitivity.

An additional useful algorithm is gapped BLAST as reported by Altschul et al., 1993, Nucl. Acids Res. 25:3389-3402. Gapped BLAST uses BLOSUM-62 substitution scores; threshold T parameter set to 9; the two-hit method to trigger ungapped extensions, charges gap lengths of k a cost of 10+k; Xu set to 16, and Xg set to 40 for database search stage and to 67 for the output stage of the algorithms. Gapped alignments are triggered by a score corresponding to about 22 bits.

Generally, the amino acid homology, similarity, or identity between individual variant CDRs or VH / VL sequences are at least 60% to the sequences depicted herein, and more typically with preferably increasing homologies or identities of at least 65% or 70%, more preferably at least 75% or 80%, even more preferably at least 85%, 90%, 91 %, 92%, 93%, 94%, 95%, 96%, 97%, 98%, 99%, and almost 100%. In a similar manner, "percent (%) nucleic acid sequence identity" with respect to the nucleic acid sequence of the binding proteins identified herein is defined as the percentage of nucleotide residues in a candidate sequence that are identical with the nucleotide residues in the coding sequence of the antibody construct. A specific method utilizes the BLASTN module of WU-BLAST-2 set to the default parameters, with overlap span and overlap fraction set to 1 and 0.125, respectively.

Generally, the nucleic acid sequence homology, similarity, or identity between the nucleotide sequences encoding individual variant CDRs or VH / VL sequences and the nucleotide sequences depicted herein are at least 60%, and more typically with preferably increasing homologies or identities of at least 65%, 70%, 75%, 80%, 81 %, 82%, 83%, 84%, 85%, 86%, 87%, 88%, 89%, 90%, 91 %, 92%, 93%, 94%, 95%, 96%, 97%, 98%, or 99%, and almost 100%. Thus, a "variant CDR" or a "variant VH / VL region" is one with the specified homology, similarity, or identity to the parent CDR / VH / VL defined in the context of the invention, and shares biological function, including, but not limited to, at least 60%, 65%, 70%, 75%, 80%, 81 %, 82%, 83%, 84%, 85%, 86%, 87%, 88%, 89%, 90%, 91 %, 92%, 93%, 94%, 95%, 96%, 97%, 98%, or 99% of the specificity and/or activity of the parent CDR or VH / VL.

In one embodiment, the percentage of identity to human germline of the antibody constructs according to the invention is≥ 70% or≥ 75%, more preferably≥ 80% or≥ 85%, even more preferably ≥ 90%, and most preferably ≥ 91 %, ≥92%, ≥ 93%, ≥ 94%, ≥ 95% or even ≥ 96%. Identity to human antibody germline gene products is thought to be an important feature to reduce the risk of therapeutic proteins to elicit an immune response against the drug in the patient during treatment. Hwang & Foote ("Immunogenicity of engineered antibodies"; Methods 36 (2005) 3-10) demonstrate that the reduction of non- human portions of drug antibody constructs leads to a decrease of risk to induce anti-drug antibodies in the patients during treatment. By comparing an exhaustive number of clinically evaluated antibody drugs and the respective immunogenicity data, the trend is shown that humanization of the V-regions of antibodies makes the protein less immunogenic (average 5.1 % of patients) than antibodies carrying unaltered non-human V regions (average 23.59 % of patients). A higher degree of identity to human sequences is hence desirable for V-region based protein therapeutics in the form of antibody constructs. For this purpose of determining the germline identity, the V-regions of VL can be aligned with the amino acid sequences of human germline V segments and J segments (http://vbase.mrc-cpe.cam.ac.uk/) using Vector NTI software and the amino acid sequence calculated by dividing the identical amino acid residues by the total number of amino acid residues of the VL in percent. The same can be for the VH segments (http://vbase.mrc-cpe.cam.ac.uk/) with the exception that the VH CDR3 may be excluded due to its high diversity and a lack of existing human germline VH CDR3 alignment partners. Recombinant techniques can then be used to increase sequence identity to human antibody germline genes.

The term "Myeloma cell" is a malignant (cancerous) plasma cell arising from a plasma cell in the bone marrow by neoplastic transformation. In myeloma, malignant plasma cells produce large amounts of abnormal antibodies that lack the capability to fight infection. These abnormal antibodies are the so called monoclonal protein, or M-protein, that functions as a tumor marker for myeloma. The myeloma cell has the phenotype CD19⁻/CD38⁺/CD138⁺/BCMA⁺. Hence, CD38, CD138 and BCMA represent antigens expressed on a myeloma cell. Also included are malignant phenotypes in the B cell lineage that are positive for CD19/CD20/CD22/BCMA and other antigens (this should include phenotypes that are not classically understood as plasma cells, but may be evolutions from memory B cells or the pre-plasma cell lineage).

The term "EGFR" refers to the epidermal growth factor receptor (EGFR; ErbB-1; HER1 in humans, including all isoforms or variants described with activation, mutations and implicated in pathophysiological processes. The EGFR antigen-binding site recognizes an epitope in the extracellular domain of the EGFR. In certain embodiments the antigen-binding site specifically binds to human and cynomolgus EGFR. The epidermal growth factor receptor (EGFR) is a member of the HER family of receptor tyrosine kinases and consists of four members: EGFR (ErbB1/HER1), HER2/neu (ErbB2), HER3 (ErbB3) and HER4 (ErbB4). Stimulation of the receptor through ligand binding (e.g. EGF, TGFa, HB-EGF, neuregulins, betacellulin, amphiregulin) activates the intrinsic receptor tyrosine kinase in the intracellular domain through tyrosine phosphorylation and promotes receptor homo- or heterodimerization with HER family members. These intracellular phospho-tyrosines serve as docking sites for various adaptor proteins or enzymes including SHC, GRB2, PLCg and PI(3)K/Akt, which simultaneously initiate many signaling cascades that influence cell proliferation, angiogenesis, apoptosis resistance, invasion and metastasis.

MFI defines median fluorescence intensity.

### Detailed description of the invention:

Thus, in a first aspect the present invention provides isolated human NK cells in a cryopreserved state, preloaded prior to the freezing with an antibody construct, the antibody construct comprising at least a first binding domain binding to an NK cell receptor antigen on the cell surface of an immunological effector cell, said first binding domain comprises three heavy chain CDRs and three light chain CDRs, and a second binding domain binding to the cell surface an antigen on the cell surface of a target cell.

Moreover, in another aspect the present invention provides a method for reconstituting/preparing such viable human NK cells preloaded with such antibody construct from a cryopreserved state.

As of today off-the-shelf products (allogeneic) are far behind the individualized (autologous) approaches. While autologous cell transfer is clinically validated, manufacturing of cell products needs to be established for each single patient. Individualized production requires 7 to 14 days for cell isolation, expansion and preparation, even though different strategies for optimization are being pursued. One limitation is that for some patients the autologous expansion of their immune cells does not yield sufficient numbers of cells. In addition, immune effector cells of a cancer patient have been described to be functionally impaired due to immunosuppression and altered phenotypes. Hence, the inter-patient variability negatively influences both individualized manufacturing, as well as clinical efficacy.

In contrast to autologous approaches allogeneic off-the-shelf products offer unique advantages: such products may be immediately applied to the patient, i.e. immediately after thawing, which dramatically reduces the time of the supply chain / logistics and increased availability of the product beyond one centralized hospital. Large batches of allogeneic immune effector cells could be manufactured in a centralized production site, which does not only reduce the manufacturing costs, but also increases the homogeneity of the immune effector cell product, enabling high quality regulatory standards. Such centralized facilities may also enable the selection of appropriate healthy donors to use the most functional effector cell population for the manufacturing process.

As already stated above, the invention provides in a first embodiment isolated human NK cells in a cryopreserved state, preloaded prior to freezing with an antibody construct, the antibody construct comprising at least a first binding domain binding to an NK cell receptor antigen on the cell surface of an immunological effector cell, said first binding domain comprises three heavy chain CDRs and three light chain CDRs, and a second binding domain binding to the cell surface an antigen on the cell surface of a target cell which may be derived from any of the above sources.

NK cells used in the context of the invention are e.g. isolated from peripheral blood mononuclear cell (PBMC) of healthy donors characterized by various phenotypes (e.g. adaptive memory NK100 cells), isolated from umbilical cord or placenta tissue, induced pluripotent stem cell-derived (iPSC-derived) NK cells (e.g. FT500 or FT516) or NK cell lines. Examples for suitable NK cell lines comprise, but are not limited to the cell lines NK-92 cells (ATCC^{®} CRL-2407^{™}), NK-YS cells (Tsuchiyama et al. 1998, Blood), or NK-YT cells (Teshigawara et al. 1985, J Immunol). Also, within the group of suitable NK cells are chimeric antigen receptor (CAR) NK cells which may be derived from any of the above sources.

The term "iPSC" defines in the context of the invention induced pluripotent stem cells that can be used to create differentiated and specialized cell types (e.g. NK cells) utilizing genetic engineering principles, defined treatments with small molecules and expansion of cells.

In some embodiments of the invention it is necessary to expand and/or activate the NK cells prior to the step of preloading the cells with said antibody construct. Protocols for such expansion are known in the art e.g. from WO2017/042393, WO2015/132415, US20110014162, US20120308986A1, US9062287B2, US8026097B2, US9260696B2; US20170119865.

In line with the invention the NK cells may be cultured prior to the incubation with the antibody construct. Such step of cell culture may be necessary to expand and/or activate the respective cells. Typical expansion protocols may comprise a selection CD34⁺ cell, a CD3⁺ cell depletion and an incubation with feeder cells carrying 4-1BBL and/or transmembrane IL-21 and optionally additional stimulation with lymphoproliferative cytokines such as IL-2. In certain embodiments a batch of NK cells preloaded with the antibody construct comprises 10⁶ and 10¹¹ cells. The number of preincubated cells, which may be administered to a patient may be in a range of 1×10⁵ to 1×10⁸ NK cells per kg bodyweight.

In the context of the invention "cells in a cryopreserved state" are cells that have been preserved by cooling to a sub-zero temperature (degree Celsius). Cryopreserved cells may or may not be preserved in the presence of a cryoprotective agent. A cryoprotective agent, such as DMSO, glycerol, ethylene glycol or propylene glycol, is a substance that protects cells from damage associated with storage at sub-zero temperature and/or freezing, e.g. cell membrane damage due to ice crystal formation.

The term "preloaded prior to freezing" relates to a preincubation of the cells prior to the cooling to sub-zero temperature with the identified antibody construct.

It is of note that the first binding domain of the described antibody construct, which an NK cell receptor antigen on the cell surface of an immunological effector cell binds to this receptor in a way, that allows for the maintenance of the binding on the cell surface even during and after this cell is in the cryopreserved state. As demonstrated in appended example 1, antibody constructs having a first binding domain specific for CD16 (a or b) bind to a specific epitope different from the IgG binding domain of the FcRγIII. It is assumed that the binding to such specific epitope allows for the maintenance of the binding even during the process of cooling the NK cells according to one aspect of the invention to a sub-zero temperature and the subsequent reconstitution of the cells for administration to a subject in the need thereof. Similar epitopes exist also for other NK cell receptor antigens on the cell surface of an immunological effector cells, e.g. NKp46 or NKG2D.

According to one embodiment of the invention the isolated human NK cells have been isolated from umbilical cord tissue or PBMC from healthy donors.

Also, in one embodiment the isolated human NK cells according to one aspect of the invention have been conserved in a cryo solution. A cryo solution in the context of the invention comprises at least a basal cell culture medium and cryoprotective agent. Moreover, a cryosolution may further comprise an isotonic solution such as Plasma-lyte, and /or serum albumin.

Non-limiting examples for cryoprotective agents have been provided herein above. Certain embodiments of cryoprotective agents in the context of the invention are DMSO and glycerol. The concentration of the cryoprotective agent may be in the range of 1% to 30% (v/v), in certain embodiments in the range of 5% to 25% (v/v), in other embodiments in the range of 10% to 25% (v/v), also in some embodiments is a concentration of 20% (v/v). The term "basal cell culture medium" relates in the context of the invention to liquid cell culture media, which are suitable for cell culture of mammalian cells. Examples for such "basal cell culture media" are well known in the art. The concentration of the isotonic solution may be in the range of 1% to 60% (v/v), in some embodiments in the range of 10% to 50% (v/v), in other embodiments in the range of 20% to 45% (v/v), and in one embodiment at a concentration of 40% (v/v). The serum albumin may be a human or (fetal) bovine (preferably human) serum albumin, which has been isolated from donors or recombinantly produced. The concentration of the albumin in the cryosolution may be in the range of 1% to 40% (v/v), in some embodiments in the range of 5% to 25% (v/v), also in some embodiments in the range of 10% to 20% (v/v). An example for a cryo solution (freezing medium) may comprise basal medium, 40% Plasma-lyte, human serum albumin (HSA) and 10% dimethyl sulphoxide (DMSO). Alternatively, fetal bovine serum (FBS) or glycerol may be used to replace HSA and/or DMSO, respectively. The most commonly used cryo-protectant is DMSO. For some reasons DSMO may be substituted by alternative compounds such as glycerol, ethylene glycol or propylene glycol.

According to certain embodiment the NK cells of one aspect of the invention have been preloaded in a solution comprising the antibody construct in a concentration of at least 5nM. In certain embodiments the concentration is of at least 10nM, moreover, in certain embodiments the concentration is of at least 25nM, also certain embodiments the concentration is of at least 50nM, in certain embodiments the concentration is of at least 75nM, 90nM, 100nM, 125nM or 150nM.

The NK cell receptor antigen to which the first binding domain of the antibody construct binds to on the surface of the isolated human NK cells according to one aspect of the invention is selected from the group consisting of CD16a, CD16b, NKp46, NKG2D and CD16a + CD16b.

Certain embodiments of NK cell receptors have been defined herein above. The first binding domain of the recited antibody construct binds thus either specifically to CD16a, CD16b or CD16a and CD16b. Moreover, the first binding domain may be specifically binding to NKp46 or NKG2D.

The cell surface antigen on the cell surface of a target cell to which the second binding domain of the antibody construct binds to is selected from the group consisting of CD19, CD20, CD22, CD30, CD33, CD52, CD70, CD74, CD79b, CD123, CLL1, BCMA, FCRH5, EGFR, EGFRvlll, HER2, GD2.

These cell surface antigens on the surface of target cells are connected with specific disease entities. CD30 is a cell surface antigen characteristic for malignant cells in Hodgkin lymphoma. CD19, CD20, CD22, CD70, CD74 and CD79b are cell surface antigens characteristic for malignant cells in Non-Hodgkin lymphomas (Diffuse large B-cell lymphoma (DLBCL), Mantle cell lymphoma (MCL), Follicular lymphoma (FL), T-cell lymphomas (both peripheral and cutaneous, including transformed mycosis fungoides/Sezary syndrome TMF/SS and Anaplastic large-cell lymphoma (ALCL)). CD52, CD33, CD123, CLL1 are cell surface antigens characteristic for malignant cells in Leukemias (Chronic lymphocytic leukemia (CLL), Acute lymphoblastic leukemia (ALL), Acute myeloid leukemia (AML)). BCMA, FCRH5 are cell surface antigens characteristic for malignant cells in Multiple Myeloma. EGFR, HER2, GD2 are cell surface antigens characteristic for solid cancers (Triple-negative breast cancer (TNBC), breast cancer BC, Colorectal cancer (CRC), Non-small-cell lung carcinoma (NSCLC), Small-cell carcinoma (SCLC also known as "small-cell lung cancer", or "oat-cell carcinoma"), Prostate cancer (PC), Glioblastoma (also known as glioblastoma multiforme (GBM)).

In one embodiment of the human NK cells according to one aspect of the invention the antibody construct comprises in the first binding domain three heavy chain CDRs and three light chain CDRs selected from the group consisting of:
(a) a CDR-H1 as depicted in SEQ ID NO: 29, a CDR-H2 as depicted in SEQ ID NO: 30, a CDR-H3 as depicted in SEQ ID NO: 31, a CDR-L1 as depicted in SEQ ID NO: 32, a CDR-L2 as depicted in SEQ ID NO: 33, a CDR-L3 as depicted in SEQ ID NO: 34;
(b) a CDR-H1 as depicted in SEQ ID NO: 40, a CDR-H2 as depicted in SEQ ID NO: 41, a CDR-H3 as depicted in SEQ ID NO: 42, a CDR-L1 as depicted in SEQ ID NO: 43, a CDR-L2 as depicted in SEQ ID NO: 44, a CDR-L3 as depicted in SEQ ID NO: 45;
(c) a CDR-H1 as depicted in SEQ ID NO: 51, a CDR-H2 as depicted in SEQ ID NO: 52, a CDR-H3 as depicted in SEQ ID NO: 53, a CDR-L1 as depicted in SEQ ID NO: 54, a CDR-L2 as depicted in SEQ ID NO: 55, a CDR-L3 as depicted in SEQ ID NO: 56;
(d) a CDR-H1 as depicted in SEQ ID NO: 62, a CDR-H2 as depicted in SEQ ID NO: 63, a CDR-H3 as depicted in SEQ ID NO: 64, a CDR-L1 as depicted in SEQ ID NO: 65, a CDR-L2 as depicted in SEQ ID NO: 66, a CDR-L3 as depicted in SEQ ID NO: 67;
(e) a CDR-H1 as depicted in SEQ ID NO: 73, a CDR-H2 as depicted in SEQ ID NO: 74, a CDR-H3 as depicted in SEQ ID NO: 75, a CDR-L1 as depicted in SEQ ID NO: 76, a CDR-L2 as depicted in SEQ ID NO: 77, a CDR-L3 as depicted in SEQ ID NO: 78;
(f) a CDR-H1 as depicted in SEQ ID NO: 84, a CDR-H2 as depicted in SEQ ID NO: 85, a CDR-H3 as depicted in SEQ ID NO: 86, a CDR-L1 as depicted in SEQ ID NO: 87, a CDR-L2 as depicted in SEQ ID NO: 88, a CDR-L3 as depicted in SEQ ID NO: 89; and
(g) a CDR-H1 as depicted in SEQ ID NO: 95, a CDR-H2 as depicted in SEQ ID NO: 96, a CDR-H3 as depicted in SEQ ID NO: 97, a CDR-L1 as depicted in SEQ ID NO: 98, a CDR-L2 as depicted in SEQ ID NO: 99, a CDR-L3 as depicted in SEQ ID NO: 100.

Also in one embodiment of the isolated human NK cells according to one aspect of the invention, the antibody construct comprises in the first binding domain pairs of VH- and VL-chains having a sequence as depicted in the pairs of sequences selected from the group consisting of SEQ ID NO: 35 and SEQ ID NO: 36, SEQ ID NO: 46 and SEQ ID NO: 47, SEQ ID NO: 57 and SEQ ID NO: 58, SEQ ID NO: 68 and SEQ ID NO: 69, SEQ ID NO: 79 and SEQ ID NO: 80, SEQ ID NO: 90 and SEQ ID NO: 91,and SEQ ID NO: 101 and SEQ ID NO: 102.

In an embodiment of the isolated human NK cells according to one aspect of the invention, the antibody construct comprises in the second binding domain three heavy chain CDRs and three light chain CDRs selected from the group consisting of:
(a) a CDR-H1 as depicted in SEQ ID NO: 106, a CDR-H2 as depicted in SEQ ID NO: 107, a CDR-H3 as depicted in SEQ ID NO: 108, a CDR-L1 as depicted in SEQ ID NO: 109, a CDR-L2 as depicted in SEQ ID NO: 110, a CDR-L3 as depicted in SEQ ID NO: 111;
(b) a CDR-H1 as depicted in SEQ ID NO: 106, a CDR-H2 as depicted in SEQ ID NO: 107, a CDR-H3 as depicted in SEQ ID NO: 108, a CDR-L1 as depicted in SEQ ID NO: 109, a CDR-L2 as depicted in SEQ ID NO: 110, a CDR-L3 as depicted in SEQ ID NO: 111; and
(c) a CDR-H1 as depicted in SEQ ID NO: 117, a CDR-H2 as depicted in SEQ ID NO: 118, a CDR-H3 as depicted in SEQ ID NO: 119, a CDR-L1 as depicted in SEQ ID NO: 120, a CDR-L2 as depicted in SEQ ID NO: 121, a CDR-L3 as depicted in SEQ ID NO: 122.

Also, in one embodiment of the isolated human NK cells according to one aspect of the invention, the antibody construct comprises in the second binding domain pairs of VH- and VL-chains having a sequence as depicted in the pairs of sequences selected from the group consisting of SEQ ID NO: 112 and SEQ ID NO: 113, SEQ ID NO: 123 and SEQ ID NO: 124, and SEQ ID NO: 134 and SEQ ID NO: 135.

In one embodiment of the isolated human NK cell according to one aspect of the invention the antibody construct comprises a protein sequence as depicted in SEQ ID NOs: 161-171.

In an alternative embodiment the invention provides a method for preparation of cryopreserved preloaded human NK cells in a cryopreserved state, the method comprising
(i) incubating NK cells with an antibody construct, the antibody construct comprising at least a first binding domain binding to an NK cell receptor antigen on the cell surface of an immunological effector cell, said first binding domain comprises three heavy chain CDRs and three light chain CDRs, and a second binding domain binding to the cell surface an antigen on the cell surface of a target cell; and
(ii) freezing the NK cells.

Moreover, the invention provides such method, wherein the NK cells are isolated from umbilical cord tissue, iPSC or PBMC from healthy donors.

As described above, the NK cells recited in the method according to one aspect of the invention have been preloaded in a solution comprising the antibody construct in a concentration of at least 5nM. In certain embodiments the concentration is of at least 10nM, in some embodiments is in a concentration of at least 25nM, of at least 50nM, or of at least 75nM. In certain embodiments the concentration is of at least 90nM, 100nM, 125nM or 150nM.

In one embodiment of the method according to one aspect of the invention the NK cell receptor antigen to which the first binding domain of the antibody construct binds to is selected from the group consisting of CD16a, CD16b, NKp46, NKG2D and CD16a + CD16b.

Also, according to certain embodiments, the cell surface antigen on the cell surface of a target cell to which the second binding domain of the antibody construct binds to is selected from the group consisting of CD19, CD20, CD22, CD30, CD33, CD52, CD70, CD74, CD79b, CD123, CLL1, BCMA, FCRH5, EGFR, HER2, GD2.

In line with the method according to one aspect of the invention for the preparation of cryopreserved preloaded human NK cells, the step of freezing the NK cells is performed using a freezing medium/cryo solution. Certain compositions and compounds of a freezing medium/cryo solution in line with the invention have been described herein above.

In certain embodiments of this method the NK cells are isolated from umbilical cord tissue, iPSC or PBMC from healthy donors.

It is also envisaged for this method in one aspect of the invention that the NK cells have been preloaded in a solution comprising the antibody construct in a concentration of at least 5nM.

Moreover, it is in line with the invention that the NK cell receptor antigen to which the first binding domain of the antibody construct binds to is selected from the group consisting of CD16a, CD16b, NKp46, NKG2D and CD16a + CD16b.

Also envisaged for this method in one aspect of the invention is that the cell surface antigen on the cell surface of a target cell to which the second binding domain of the antibody construct binds to is selected from the group consisting of CD19, CD22, CD30, CD33, CD52, CD70, CD74, CD79b, CD123, CLL1, BCMA, FCRH5, EGFR, HER2, GD2.

According to one embodiment of this method in one aspect of the invention the step of freezing the NK cells is performed using a freezing medium which contains least a basal cell culture medium and cryoprotective agent.

In one aspect of the method of the invention the antibody construct used in the method is one of the antibody constructs described herein above. In certain embodiments of the method of the invention that said antibody construct comprises a protein sequence as depicted in SEQ ID NOs: 161-171.

In an alternative embodiment the invention provides a method for reconstituting/preparing viable preloaded human NK cells from human NK cells according to one aspect of the invention in a cryopreserved state described herein above or prepared according to a method according to one aspect of the invention for preparation of cryopreserved preloaded human NK cells in a cryopreserved state for an administration of said cells to a subject in the need thereof, the method comprising the step of reconstituting/preparing the cells for administration to a patient by thawing.

Thawing of frozen cells may be accomplished according to well-known methods. General thawing procedures involve rapidly transferring the frozen cells to a 37° C water bath and providing gentle agitation until the activated NK cells are completely thawed. After thawing, the thawed NK cells may be prepared for administration into a patient by adding, preferably in a dropwise fashion, pharmaceutically acceptable carriers at room temperature, e.g., to dilute the concentration of freeze medium and/or wash the previously activated NK cells. As demonstrated herein, the cell populations of the present invention retain their activated state in combination with the antibody construct after such preservation. Preparation of the cells for administration after preservation depends on the preservation method. For example, cells may be prepared for administration after preservation by cell culture and/or refrigeration by one or more of the following: washing the cells, resuspending the cells in a pharmaceutically acceptable carrier, and/or containing the cells in suitable delivery device, e.g., a syringe. In one embodiment, cells are prepared for administration after cryopreservation by thawing, e.g., by gentle agitation in a 37° C water bath and then containing the cells in a suitable delivery device, e.g., a syringe. These thawed cell populations may be surprisingly employed in the clinic almost immediately on an as-needed basis, e.g., without reactivation or other extensive and/or time-consuming manipulation.

Accordingly, although unnecessary, cells prepared for administration after cryopreservation may be further prepared by the addition, preferably in a dropwise fashion, a pharmaceutically acceptable carrier at room temperature to, e.g., dilute the concentration of and/or wash the cells free of freeze medium. In one embodiment, the pharmaceutically acceptable carrier is substantially free of an activating agent.

In a further alternative embodiment, the invention provides a pharmaceutical composition comprising human NK cells which have been reconstituted from human NK cells in a cryopreserved state according to the invention or prepared by a method according to the invention.

Certain embodiments provide pharmaceutical compositions comprising the preloaded NK cells defined in the context of the invention and further one or more excipients such as those illustratively described in this section and elsewhere herein. Excipients can be used in the invention in this regard for a wide variety of purposes, such as adjusting physical, chemical, or biological properties of formulations, such as adjustment of viscosity, and or processes of one aspect of the invention to improve effectiveness and or to stabilize such formulations and processes against degradation and spoilage due to, for instance, stresses that occur during manufacturing, shipping, storage, pre-use preparation, administration, and thereafter.

In certain embodiments, the pharmaceutical composition may contain formulation materials for the purpose of modifying, maintaining or preserving, e.g., the pH, osmolarity, viscosity, clarity, color, isotonicity, odor, sterility, stability, rate of dissolution or release, adsorption or penetration of the composition (see, REMINGTON'S PHARMACEUTICAL SCIENCES, 18" Edition, (A.R. Genrmo, ed.), 1990, Mack Publishing Company). In such embodiments, suitable formulation materials may include, but are not limited to:
- amino acids such as glycine, alanine, glutamine, asparagine, threonine, proline, 2-phenylalanine, including charged amino acids, preferably lysine, lysine acetate, arginine, glutamate and/or histidine
- antimicrobials such as antibacterial and antifungal agents
- antioxidants such as ascorbic acid, methionine, sodium sulfite or sodium hydrogensulfite;
- buffers, buffer systems and buffering agents which are used to maintain the composition at physiological pH or at a slightly lower pH; examples of buffers are borate, bicarbonate,
- Tris-HCl, citrates, phosphates or other organic acids, succinate, phosphate, and histidine; for example Tris buffer of about pH 7.0-8.5;
- non-aqueous solvents such as propylene glycol, polyethylene glycol, vegetable oils such as olive oil, and injectable organic esters such as ethyl oleate;
- aqueous carriers including water, alcoholic/aqueous solutions, emulsions or suspensions, including saline and buffered media;
- biodegradable polymers such as polyesters;
- bulking agents such as mannitol or glycine;
- chelating agents such as ethylenediamine tetraacetic acid (EDTA);
- isotonic and absorption delaying agents;
- complexing agents such as caffeine, polyvinylpyrrolidone, beta-cyclodextrin or hydroxypropyl-beta-cyclodextrin)
- fillers;
- monosaccharides; disaccharides; and other carbohydrates (such as glucose, mannose or dextrins); carbohydrates may be non-reducing sugars, preferably trehalose, sucrose, octasulfate, sorbitol or xylitol;
- (low molecular weight) proteins, polypeptides or proteinaceous carriers such as human or bovine serum albumin, gelatin or immunoglobulins, preferably of human origin;
- coloring and flavouring agents;
- sulfur containing reducing agents, such as glutathione, thioctic acid, sodium thioglycolate, thioglycerol, [alpha]-monothioglycerol, and sodium thio sulfate
- diluting agents;
- emulsifying agents;
- hydrophilic polymers such as polyvinylpyrrolidone)
- salt-forming counter-ions such as sodium;
- preservatives such as antimicrobials, anti-oxidants, chelating agents, inert gases and the like; examples are: benzalkonium chloride, benzoic acid, salicylic acid, thimerosal, phenethyl alcohol, methylparaben, propylparaben, chlorhexidine, sorbic acid or hydrogen peroxide);
- metal complexes such as Zn-protein complexes;
- solvents and co-solvents (such as glycerin, propylene glycol or polyethylene glycol);
- sugars and sugar alcohols, such as trehalose, sucrose, octasulfate, mannitol, sorbitol or xylitol stachyose, mannose, sorbose, xylose, ribose, myoinisitose, galactose, lactitol, ribitol, myoinisitol, galactitol, glycerol, cyclitols (e.g., inositol), polyethylene glycol; and polyhydric sugar alcohols;
- suspending agents;
- surfactants or wetting agents such as pluronics, PEG, sorbitan esters, polysorbates such as polysorbate 20, polysorbate, triton, tromethamine, lecithin, cholesterol, tyloxapal; surfactants may be detergents, preferably with a molecular weight of >1.2 KD and/or a polyether, preferably with a molecular weight of >3 KD; non-limiting examples for preferred detergents are Tween 20, Tween 40, Tween 60, Tween 80 and Tween 85; non-limiting examples for preferred polyethers are PEG 3000, PEG 3350, PEG 4000 and PEG 5000;
- stability enhancing agents such as sucrose or sorbitol;
- tonicity enhancing agents such as alkali metal halides, preferably sodium or potassium chloride, mannitol sorbitol;
- parenteral delivery vehicles including sodium chloride solution, Ringer's dextrose, dextrose and sodium chloride, lactated Ringer's, or fixed oils;
- intravenous delivery vehicles including fluid and nutrient replenishers, electrolyte replenishers (such as those based on Ringer's dextrose).

It is evident to those skilled in the art that the different constituents of the pharmaceutical composition (e.g., those listed above) can have different effects, for example, and amino acid can act as a buffer, a stabilizer and/or an antioxidant; mannitol can act as a bulking agent and/or a tonicity enhancing agent; sodium chloride can act as delivery vehicle and/or tonicity enhancing agent; etc.

In certain embodiments, the optimal pharmaceutical composition will be determined by one skilled in the art depending upon, for example, the intended route of administration, delivery format and desired dosage. See, for example, REMINGTON'S PHARMACEUTICAL SCIENCES, supra. For example, a suitable vehicle or carrier may be water for injection, physiological saline solution or artificial cerebrospinal fluid, possibly supplemented with other materials common in compositions for parenteral administration. Neutral buffered saline or saline mixed with serum albumin are further exemplary vehicles.

In one embodiment of the pharmaceutical composition according to one aspect of the invention the composition is administered to a patient intravenously.

Methods and protocols for the intravenous (iv) administration of pharmaceutical compositions described herein are well known in the art.

In one aspect of the a pharmaceutical composition of the invention said pharmaceutical composition is used in the prevention, treatment or amelioration of a disease selected from a proliferative disease, a tumorous disease, or an immunological disorder. Preferably, said tumorous disease is a malignant disease, preferably cancer.

In one embodiment of the pharmaceutical composition of the invention the identified malignant disease is selected from the group consisting of Hodgkin lymphoma, Non-Hodgkin lymphoma, leukemia, multiple myeloma and solid tumors.

Also, in one embodiment the invention provides preloaded human NK cells which have been reconstituted from human NK cells in a cryopreserved state according to the invention or prepared by a method according to the aspects of the invention for reconstituting/preparing viable preloaded human NK cells from a cryopreserved state for use in the treatment or amelioration of a disease.

In line with one aspect of the use for the treatment or amelioration of a disease of the invention the preloaded human NK cells are administered to a patient intravenously.

In one embodiment of said use the subject suffers from a proliferative disease, a tumorous disease, or an immunological disorder.

It is preferred that said tumorous disease is a malignant disease, preferably cancer.

In one embodiment of said use said malignant disease is selected from the group consisting of Hodgkin lymphoma, Non-Hodgkin lymphoma, leukemia, multiple myeloma and solid tumors.

It must be noted that as used herein, the singular forms "a", "an", and "the", include plural references unless the context clearly indicates otherwise. Thus, for example, reference to "a reagent" includes one or more of such different reagents and reference to "the method" includes reference to equivalent steps and methods known to those of ordinary skill in the art that could be modified or substituted for the methods described herein.

Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein. Such equivalents are intended to be encompassed by the present invention.

The term "and/or" wherever used herein includes the meaning of "and", "or" and "all or any other combination of the elements connected by said term".

The term "about" or "approximately" as used herein means within 20%, preferably within 10%, and more preferably within 5% of a given value or range. It includes, however, also the concrete number, e.g., about 20 includes 20.

The term "less than" or "greater than" includes the concrete number. For example, less than 20 means less than or equal to. Similarly, more than or greater than means more than or equal to, or greater than or equal to, respectively.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having".

When used herein "consisting of" excludes any element, step, or ingredient not specified in the claim element. When used herein, "consisting essentially of" does not exclude materials or steps that do not materially affect the basic and novel characteristics of the claim.

In each instance herein, any of the terms "comprising", "consisting essentially of" and "consisting of" may be replaced with either of the other two terms.

It should be understood that this invention is not limited to the particular methodology, protocols, material, reagents, and substances, etc., described herein and as such can vary. The terminology used herein is for the purpose of describing particular embodiments only, and is not intended to limit the scope of the present invention, which is defined solely by the claims.

Examples of antibody constructs which can be used according to this invention are described in MAbs. 2019 Jul;11(5):899-918.

A better understanding of the present invention and of its advantages will be obtained from the following examples, offered for illustrative purposes only. The examples are not intended to limit the scope of the present invention in any way.

### Example1: Assessment of antibody-mediated cytotoxicity by NK cells after preloading and freeze/thaw

### Culture of cell lines

MM.1S (ATCC, cat.: CRL2974) and DK-MG cells (DSMZ, cat.: ACC277) were cultured in RPMI 1640 medium supplemented with 10% heat-inactivated FCS, 2 mM L-glutamine and 100 IU/mL penicillin G sodium and 100 µg/mL streptomycin sulfate. All cell lines were cultured under standard conditions and sub-cultured as recommended by the supplier at 37°C in a humidified atmosphere with 5% CO₂.

### Isolation of PBMC from buffy coats and enrichment of human NK cells

PBMCs were isolated from buffy coats (German Red Cross, Mannheim, Germany) by density gradient centrifugation. The buffy coat samples were diluted with a two-to-threefold volume of PBS (Invitrogen, cat.: 14190-169), layered on a cushion of Lymphoprep (Stem Cell Technologies, cat.: 07861) and centrifuged at 800 × g for 25 min at room temperature w/o brake. PBMC located in the interface were collected and washed 3 times with PBS before they were cultured in complete RPMI 1640 medium (RPMI 1640 medium supplemented 10% heat-inactivated FCS, 2 mM L-glutamine and 100 IU/mL penicillin G sodium and 100 µg/mL streptomycin sulfate (all components from Invitrogen)) overnight without stimulation. For the enrichment of NK cells PBMC were harvested from overnight cultures and used for one round of negative selection using the EasySep^{™} Human NK Cell Enrichment Kit (Stem Cell Technologies, cat.: 17055) for the immunomagnetic isolation of untouched human NK cells and the Big Easy EasySep^{™} Magnet (Stem Cell Technologies, cat.: 18001) according to the manufacturer's instructions.

### Preloading of NK cells and freeze/thaw

Aliquots of enriched primary human NK cells were resuspended in complete RPMI 1640 medium containing 10 µg/mL of the indicated antibody constructs in a total volume of 0.5 mL and incubated for 30 min at room temperature. As indicated, half of the aliquots was washed twice with 5 mL complete RPMI 1640 medium and then resuspended in 0.5 mL complete RPMI 1640 medium, and the second half of the aliquots was not washed before FCS supplemented with 20% DMSO (Sigma) was added. The cell suspensions were then transferred to 2 mL cryo tubes (Greiner bio-one) and frozen at -80°C in cryo 1°C freezing container (Nalgene) for more than 12 h.

### 4 h calcein-release cytotoxicity assays

For calcein-release cytotoxicity assays the indicated target cells were harvested from cultures, washed with RPMI 1640 medium without FCS, and labeled with 10 µM calcein AM (Invitrogen/Molecular Probes, cat.: C3100MP) for 30 min in RPMI medium without FCS at 37°C. After gently washing the labeled cells were resuspended in complete RPMI 1640 medium to a density of 1×10⁵/mL, and aliquots of 1×10⁴ target cells were then seeded in individual wells of a 96-well round-bottom micro plate. In parallel, NK cells were thawed from -80°C by incubation of the cryo tubes in a 37°C water bath. When the last ice crystals were visible, the cell suspension was diluted with complete RPMI 1640 medium centrifuged and washed once before the cells were added to the target cells at the indicated effector-to-target (E:T) ratio with or without addition of fresh antibody as indicated in a total volume of 200 µL in duplicates. Spontaneous release was determined by incubation of target cells in the absence of effector cells and without antibodies, and maximal release was induced by the addition of 1% Triton x-100 (final concentration, Roth) and also measured in quadruplicate on each plate.

After centrifugation for 2 min at 200 g the assay was incubated for 4 h at 37°C in a humidified atmosphere with 5% CO₂. 100 µL cell culture supernatant were harvested from each well after an additional centrifugation for 5 min at 500 g, and the fluorescence of the released calcein was measured at 520 nm using a fluorescence multi-plate reader (Victor 3 or EnSight, Perkin Elmer). On the basis of the measured counts, the specific cell lysis was calculated according to the following formula: [fluorescence (sample) - fluorescence (spontaneous)] / [fluorescence (maximum) - fluorescence (spontaneous)] × 100%. Fluorescence (spontaneous) represents the fluorescent counts from target cells in the absence of effector cells and antibodies and fluorescence (maximum) represents the total cell lysis induced by the addition of Triton X-100. Lysis values were analyzed and mean and standard deviation were plotted using the GraphPad Prism software (GraphPad Prism version 7.04 for Windows, GraphPad Software, La Jolla California USA).

The results are shown in figure 1.

### Example 2: Analysis of binding of anti-CD16 antibodies to different antigen variants

FcgRIII (CD16), the low-affinity receptor for the Fc part of IgG exists in two isoforms encoded by two nearly identical genes: FCGR3A (CD16A) and FCGR3B (CD16B), resulting in tissue-specific expression of alternative membrane-anchored isoforms (Ravetch and Perussia, 1989, J Exp Med.):
- FcgRIIIA (CD16A) is expressed as a transmembrane protein on NK cells, makrophages, and subsets of monocytes and T-cells
- FcgRIIIB (CD16B) is expressed glycosylphosphatidylinositol (GPI)-anchored by neutrophils and after gamma-interferon stimulation by eosinophils.

Allelic variants/polymorphisms of both, CD16A and CD16B are well known (summarized in Table 2).

CD16B allotypic variants differ in five amino acids of the extracellular domain (ECD) (Table 2). Two of these enable additional glycosylation, resulting in three different variants of the GPI-anchored FcγRIIIB granulocyte antigens HNA-1a, -1b, and -1c (formerly named NA1, NA2 and SH).

There are only few differences in the amino acid sequences of CD16A and B. Their differences are restricted to two alternative amino acids in the mature ECD (Table 2). Additional differences reside in the disparate modes of membrane anchorage of CD16A and CD16B. The C-terminal propeptide sequence of CD16B which is cleaved off in the mature GPI-anchored form is highly homologous to the sequence connecting CD16A ECD to its transmembrane domain (Table 2).

**Table 2: Summary of sequence variations of FcgRIIIA (CD16A) and FcgRIIIB (CD16B) alleles. We use amino acid numbers displayed in the left column. Corresponding numbering from Uniprot is shown on the right for comparison. Positions where CD16A differs from CD16B are highlighted in bold and underlined. The propeptide sequence which is removed in the mature form of CD16B is shown in crossed out letters.**

| **Amino acid #** | **CD16A** | **CD16B** | | **Amino acid position (Uniprot)** |
|---|---|---|---|---|
| 18 | R | R/S | CD16B polymorphism | 36 |
| 47 | S | N/S | CD16B polymorphism | 65 |
| 48 | R/L/H | L | CD16A polymorphism | 66 |
| 60 | A | A/D | CD16B polymorphism | 78 |
| 64 | D | D/N | CD16B polymorphism | 82 |
| 88 | I | I/V | CD16B polymorphism | 106 |
| **129** | **G** | **D** | **CD16A/CD16B sequence variation** | 147 |
| **140** | **Y** | **H** | **CD16A/CD16B sequence variation** | 158 |
| 158 | V/F | V | CD16A polymorphism | 176 |
| **182** | S | S **w/ GPI- anchor** | **CD16A/CD16B variation** | 200 |
| 183 | SF**F**PPGY | | **CD16A/CD16B variation** | 201 |
| -190ff | Q... | | CD16B- propeptide cleaved off | -208ff |
| **185** | **F** | **S** | **CD16A/CD16B sequence variation** | 203 |

The isolation of the CD16A specific antibody "LSIV21" lacking CD16B binding was previously described (Reusch et al., 2014, MAbs).

To investigate which of the CD16A/CD16B sequence variations determines the binding site for our CD16A-selective antibodies, we generated and characterized a set of soluble recombinant CD16A- and CD16B-antigen variants.

CD16B(NA1) ECD recombinant protein was either expressed as the mature form (without propeptide) (CD16B^{mat}), or as a variant containing the propeptide sequence (CD16B^{pr}). In addition, CD16B ECD mutants in which CD16B-specific amino acids were replaced with the corresponding residues of CD16A were constructed, expressed and tested in binding assays: CD16B^{D129G}, CD16B^{H140Y}, CD16B^{S185F}. All these antigen variants were fused via glycin-serin linker to soluble monomeric Fc (Ying et al, 2012, J. Biol. Chem.), expressed in CHO and purified from cell culture supernatants.

A scFv antibody comprising the variable heavy and light chain sequences of the published CD16A- and CD16B-reactive antibody 3G8 recognizes all tested CD16A and CD16B antigen variants (Figure 2, Table 3). Different antibodies containing the variable heavy and light chain sequences of "LSIV21" or the affinity-improved "P2C47" (in form of monovalent scFv or bivalently binding tetravalent bispecific tandem diabody antibodies) recognizes CD16A but not the mature or propeptide-containing ECD fusion antigens of CD16B. If however Histidine (H) at position 140 in the CD16B ECD was mutated to Tyrosine (Y) - the amino acid present at the corresponding position in CD16A - this mutant form of CD16B (CD16B^{H140Y}) was well recognized by antibodies containing the "LSIV21" or "P2C47" variable domains (Figure 2, Table 3).

**Table 3: Summary of binding of different anti-CD16 antibodies to coated CD16 antigen variants analyzed in ELISA. Binding EC₅₀ values were calculated using four-parameter logistic (4PL) curve model log(agonist) vs. response - Variable slope in Graphpad Prism.**

| | **Binding EC₅₀ [nM] in ELISA** | | | |
|---|---|---|---|---|
| **coated antigens** (all fused to monomeric Fc) | **3G8 scFv** | **CD16-2 scFv** | **EGFR-1/CD16-1 TandAb (CD16-1)** | **BCMA-1/CD16-2 TandAb (CD16-2)** |
| Human CD16A^{(48R, 158V)} | 1,0 | 1,6 | 0,6 | 1,1 |
| Human CD16B^{mat} | 0,9 | **no binding** | **no binding** | **no binding** |
| Human CD16B^{pr} | 0,8 | **no binding** | **no binding** | **no binding** |
| Human CD16B^{D129G} | 0,8 | **no binding** | **no binding** | **no binding** |
| Human CD16B^{H140Y} | 0,7 | 1,3 | 0,5 | 1,2 |
| Human CD16B^{S185F} | 0,7 | **no binding** | **no binding** | **no binding** |

Additional affinity matured anti-CD16 scFv antibodies were similarly analyzed in ELISA. Results show that except for scFv "CD16-5" which shows weak cross-reactivity with CD16B, the other affinity matured anti-CD16 scFv antibodies maintain the CD16A selective binding property of the parental antibody. They do not bind to CD16B, unless CD16B contains the previously described amino acid exchange CD16B^{H140Y} (Table 4). All anti-CD16 antibodies show very good cross-reactivity with cynomolgus CD16A (Table 4), despite the fact that the cynomolgus CD16A ECD sequence differs in a total of 16 amino acids from human CD16A (see alignment in Figure 4).

The importance of Tyrosine (Y) at position 140 in the CD16 ECD for CD16A-specific recognition by our antibodies was also confirmed using recombinant CHO cells with transgenic expression of different CD16 antigen variants on the cell surface. ECD sequences were either fused to the transmembrane domain of human EGFR (Wang et al., 2011, Blood 118(5)1255) or anchored via GPI using the human CD16B endogenous sequence with the full length propeptide sequence for posttranslational processing and lipidation for GPI-anchorage.

scFv antibody comprising the variable heavy and light chain sequences of the published CD16A- and CD16B-reactive antibody 3G8 recognizes all cell surface expressed CD16A and CD16B antigen variants (Figure 3), but not recombinant EGFR expressed as a specificity control. scFv antibodies containing the variable heavy and light chain sequences of "LSIV21" or the affinity-improved "P2C47" recognize CHO cell surface expressed CD16A variants but not the mature or propeptide-containing ECD of CD16B(NA1). If however Histidine (H) at position 140 in the CHO membrane-anchored CD16B ECD was mutated to Tyrosine (Y) - the amino acid present at the corresponding position in CD16A - this mutant form of CD16B (CD16BH140Y) was well recognized by scFv antibodies containing the "LSIV21" or "P2C47" variable domains (Figure 3).

CD16A and CD16BH140Y antigen variants, but not CD16B antigen are also recognized after SDS-PAGE and Western blot when samples are treated under non-reducing conditions, by antibodies containing "LSIV21", "P2C47" or affinity matured variable domains (Figure 5), whereas Antibody 3G8 recognizes all CD16A and CD16B antigen variants. When samples are reduced prior to loading on SDS-PAGE gels, no signals are obtained on Western blots (data not shown), suggesting a non-linear sequence, but rather three-dimensional structure as the binding epitope. In the case of recognition by "LSIV21", "P2C47" or affinity matured variants of these antibodies, the three-dimensional epitope structure is likely determined by Tyrosine in position 140 of CD16.

**Table 3: Summary of binding of different anti-CD16 antibodies to coated CD16 antigen variants analyzed in ELISA. Binding EC₅₀ values were calculated using four-parameter logistic (4PL) curve model log(agonist) vs. response - Variable slope in Graphpad Prism.**

| | **Binding EC₅₀ [nM] in ELISA** | | | | | | | | | | |
|---|---|---|---|---|---|---|---|---|---|---|---|
| **Coated antigen** (all fused to IgG Fc) | 3G8 | CD16-1 | CD16-2 | CD16-6 | CD16-8 | CD16-9 | CD16-10 | CD16-11 | CD16-12 | CD16-5 | CD16-3 |
| Human CD16A^{(48R, 158V)} | 1,2 | 14,4 | 1,5 | 1,4 | 1,7 | 1,8 | 1,3 | 2,7 | 1,4 | 1,2 | 1,2 |
| Human CD16A^{(48R, 158F)} | 7,6 | 25,7 | 2,8 | 0,9 | 1,5 | 1,7 | 1,2 | 2,8 | 1,2 | 0,8 | 1,2 |
| Cynomolgus CD16A | 7,5 | 16,2 | 2,5 | 1,0 | 1,7 | 1,4 | 1,2 | 3,9 | 1,1 | 1,4 | 1,4 |
| Human CD16B(NA1)^{pr} | 0,8 | **no binding** | **no binding** | **no binding** | **no binding** | **no binding** | **no binding** | **no binding** | **no binding** | 24,9 | **no binding** |
| Human CD16B^{H140Y} | 0,7 | 9,5 | 1,2 | 1,1 | 1,5 | 1,6 | 1,3 | 2,0 | 1,1 | 1,1 | 1,3 |

| | **Binding EC₅₀ [nM] in ELISA** | | | | | | | | | | |
|---|---|---|---|---|---|---|---|---|---|---|---|
| **Coated antigen** (all fused to IgG Fc) | CD16-13 | CD16-14 | CD16-15 | CD16-16 | CD16-4 | CD16-17 | CD16-18 | CD16-19 | CD16-20 | CD16-21 | CD16-7 |
| Human CD16A^{(48R,158V)} | 1,6 | 1,1 | 1,3 | 1,4 | 2,2 | 6,7 | 1,2 | 1,3 | 1,7 | 1,1 | 1,8 |
| Human CD16A^{(48R,158F)} | 1,6 | 1,0 | 1,1 | 1,1 | 2,5 | 14,5 | 1,0 | 1,3 | 1,8 | 1,3 | 2,4 |
| Cynomolgus CD16A | 1,8 | 1,1 | 1,8 | 1,4 | 1,4 | 10,7 | 1,7 | 2,1 | 2,6 | 1,6 | 1,6 |
| Human CD16B(NA1)^{pr} | **no binding** | **no binding** | **no binding** | **no binding** | **no binding** | **no binding** | **no binding** | **no binding** | **no binding** | **no binding** | **no binding** |
| Human CD16B^{H140Y} | 1,6 | 1,2 | 1,4 | 1,4 | 1,7 | 4,9 | 1,1 | 1,1 | 1,4 | 1,0 | 1,7 |

The IgG binding site of FcgRIII is a discontinuous binding area on the membrane proximal extracellular domain of the receptor. Crucial amino acids for the low-affinity interaction of FcgRIIIA (CD16A) with the Fc part of IgG were previously identified and described (e.g. Ahmed et al., 2016). Residues in CD16A involved in the interaction with the CH2 domain of Fc portions are K120, Y132, H134, H135 and K161. While the antibody 3G8 was reported to bind CD16A and CD16B and block IgG binding (Tamm and Schmidt, 1996), we did not observe blocking of binding of our anti-CD16 antibodies to CD16A by IgGs. To support the hypothesis that the binding site of our anti-CD16A specific antibodies is different from the IgG binding site on FcgRIIIA, we performed a competition ELISA. While binding of 3G8 mAb to CD16A is blocked with increasing concentrations of a scFv antibody containing the variable domains of 3G8, the other anti-CD16 scFv antibodies do not block or displace 3G8 mAb on CD16A and bind independently (Figure 6). Since the 3G8 binding site is known to block IgG binding, we conclude that the binding site of our anti-CD16 antibodies is distinct from the IgG binding site.

### Analysis of binding in ELISA

96-well ELISA plates (Immuno MaxiSorp; Nunc) were coated overnight at 4°C with recombinantly produced fusion proteins of CD16 extracellular domain (ECD) antigen variants fused to human IgG1 Fc or monomeric mFc.67 (Ying et al, 2012, J. Biol. Chem.) in 100mM Carbonate-bicarbonate buffer at a concentration of 1.5 or 3µg/mL. After a blocking step with 3% (w/v) skim milk powder (Merck) dissolved in PBS, serial dilutions of the different antibodies in PBS containing 0.3% (w/v) skim milk powder were incubated on the plates for 1.5h at room temperature. After washing three times with 300µL per well of PBS containing 0.1% (v/v) Tween 20, plates were incubated with detection antibodies for 1 h at room temperature. For the detection of His-tagged analytes, Penta-HIS-HRP (Qiagen) was used at 1:3000 dilution. For the detection of tetravalent bispecific BCMA/CD16 TandAb antibody, T763, Protein L-HRP conjugate (Pierce) was used at 1:20.000 dilution for 1 hour at room temperature. After washing three times with 300µL per well of PBS containing 0.1% (v/v) Tween 20, plates were incubated with Tetramethylbenzidine (TMB) substrate (Seramun) until color development was clearly visible. The reaction was stopped through the addition of 100µL per well of 0.5M H2SO4. The absorbance was measured at 450nm using a multilabel plate reader (Victor, Perkin Elmer). Absorbance values were plotted and analyzed using nonlinear regression, sigmoidal dose-response (variable slope), least squares (ordinary) fit with GraphPad Prism version 6.07 (GraphPad Software, La Jolla California USA).

### Analysis of binding after SDS-PAGE and Western blot

Purified recombinant CD16 antigen variants (human CD16A^{48R}, ^{158V-}mFc.67, human CD16B(NA1)^{pr}-mFc.67 and huCD16B(NA1)^{pr}H140Y-mFc.67 were diluted in PBS to 0,5µg/mL, mixed with an equal volume of non-reducing sample buffer and 8µl of the mix were loaded into the wells of 4-20% Criterion TGX Precast Gels (Biorad). Gels were run at 300V in 1x TGS buffer (Biorad) for 22min and total protein was imaged using the Biorad Molecular Imager Gel Documentation system. Protein was transferred by Western blotting to PVDF Midi Membranes (Biorad) using the Trans-Blot Turbo system (Biorad). Membranes were incubation for 30min with 3% (w/v) skimmed milk powder (Merck) dissolved in TBS to block unspecific binding sites and then incubated with primary antibody dilutions: anti-CD16 antibodies were diluted in TBS containing 3% (w/v) skimmed milk powder to a concentration of 4µg/mL and incubated on the membranes for 1 hour at room temperature. After washing with TBST (TBS containing 0.1% (v/v) Tween 20) and two times with TBS, membranes were incubated with secondary antibody-detection conjugates: Membranes incubated with anti-CD16A containing scFv-IgAb antibodies were incubated with Protein L-HRP (Pierce), 1:10.000 diluted in TBS containing 3% (w/v) skimmed milk powder, membranes incubated with anti-CD16 scFv antibodies were incubated with anti-Penta-His-HRP (QIAGEN) 1:3000 diluted in TBS containing 3% (w/v) skimmed milk powder, for 1 hour at room temperature. After washing with TBST and two times with TBS, colorimetric development was started by addition of a freshly prepared mixture of 0.66mg/mL DAB, 0,02% CoCl2, 0.015% H2O2 in TBS and incubated on the membranes until color developed. Reaction was stopped by washing membranes in water. Membranes were dried and photographed.

### CD16A competition ELISA

96-well ELISA plates (Immuno MaxiSorp; Nunc) were coated overnight at 4°C with recombinantly produced fusion proteins of CD16A(48R, 158V) ECD fused to monomeric mFc.67 (Ying et al, 2012, J. Biol. Chem.) in 100mM Carbonate-bicarbonate buffer at a concentration of 1.5µg/mL. After a blocking step with 3% (w/v) skim milk powder (Merck) dissolved in PBS, serial dilutions of the different scFv-antibodies in PBS containing 0.3% (w/v) skim milk powder were mixed with 1nM of anti-CD16 mAb 3G8 (BD Bioscience) and co-incubated on the plates for 1.5h at room temperature. After washing three times with 300µL per well of PBS containing 0.1% (v/v) Tween 20, plates were incubated with detection antibodies for 1 h at room temperature. For the detection of CD16A-bound 3G8, goat anti-mouse IgG(H+L)-HRPO (Dianova 115-035-003) was used at 1:10.000 dilution. After washing three times with 300µL per well of PBS containing 0.1% (v/v) Tween 20, plates were incubated with Tetramethylbenzidine (TMB) substrate (Seramun) until color development was clearly visible. The reaction was stopped through the addition of 100µL per well of 0.5M H2SO4. The absorbance was measured at 450nm using a multilabel plate reader (Victor, Perkin Elmer). Absorbance values were plotted and analyzed using nonlinear regression, sigmoidal dose-response (variable slope), least squares (ordinary) fit with GraphPad Prism version 6.07 (GraphPad Software, La Jolla California USA).

### Example 3: Binding of anti-CD16A scFv to primary human NK cells at 37°C in the presence or absence of 10 mg/mL polyclonal human IgG

### Methods:

### Isolation of PBMC from buffy coats and enrichment of human NK cells

PBMCs were isolated from buffy coats (German Red Cross, Mannheim, Germany) by density gradient centrifugation. The buffy coat samples were diluted with a two-to-threefold volume of PBS (Invitrogen, cat.: 14190-169), layered on a cushion of Lymphoprep (Stem Cell Technologies, cat.: 07861) and centrifuged at 800 × g for 25 min at room temperature w/o brake. PBMC located in the interface were collected and washed 3 times with PBS before they were cultured in complete RPMI 1640 medium (RPMI 1640 medium supplemented 10% heat-inactivated FCS, 2 mM L-glutamine and 100 IU/mL penicillin G sodium, and 100 µg/mL streptomycin sulfate (all components from Invitrogen) overnight without stimulation. For the enrichment of NK cells, PBMCs were harvested from overnight cultures and used for one round of negative selection using the EasySep^{™} Human NK Cell Enrichment Kit (Stem Cell Technologies, cat.: 19955) for the immunomagnetic isolation of untouched human NK cells and the Big Easy EasySep^{™} Magnet (Stem Cell Technologies, cat.: 18001) according to the manufacturer's instructions.

### Cell binding assays and flow cytometric analyses

Aliquots of 0.2-1 × 106 enriched human NK cells were incubated with 100 µL of serial dilutions of the indicated scFv constructs in FACS buffer (PBS, Invitrogen, cat.: 14190-169) containing 2% heat-inactivated FCS (Invitrogen, cat.: 10270-106), 0.1% sodium azide (Roth, Karlsruhe, Germany, cat.: A1430.0100) in the presence of 10 mg/mL polyclonal human IgG (Gammanorm, Octapharma) or the corresponding buffer for 45 min at 37°C. After repeated washing with FACS buffer, cell-bound antibodies were detected with 10 µg/mL anti-His mAb 13/45/31-2 (Dianova, Hamburg, Germany, cat.: DIA910-1MG) followed by 15 µg/mL FITC-conjugated goat anti-mouse IgG (Dianova, cat.: 115-095-062). After the last staining step, the cells were washed again and resuspended in 0.2 mL of FACS buffer containing 2 µg/mL propidium iodide (PI) (Sigma, cat.: P4170) in order to exclude dead cells. The fluorescence of 2-5 × 103 living cells was measured using a Millipore Guava EasyCyte flow cytometer (Merck Millipore, Schwalbach, Germany) or CytoFlex cytometer (Beckman Coulter, Krefeld, Germany) and median fluorescence intensities of the cell samples were determined. After subtracting the fluorescence intensity values of the cells stained with the secondary and tertiary reagents alone, the values were used for non-linear regression analysis using the GraphPad Prism software (GraphPad Prism version 7.04 for Windows, GraphPad Software, La Jolla California USA). For the calculation of KD, the equation for one-site-binding (hyperbola) was used.

### Results

The anti-CD16 scFv containing the human Fv domains from clone CD16-1, scFv containing the human anti-CD16 Fv domains from clone CD16-2, and scFv_3G8, containing the murine Fv domains from mAb 3G8 were titrated on primary human NK cells at 37°C in the presence or absence of 10 mg/mL polyclonal human IgG. The respective results are depicted in Figure 7.

### Example 4: Assessment of antibody-mediated cytotoxicity by NK cells after preloading with anti-EpCAM and anti-CD30 antibodies and freeze/thaw

### Methods:

**Table 4: Antibody constructs**

| **Construct** | **target specificity** | **target domain** | **effector specificity** | **effector domain** |
|---|---|---|---|---|
| **scFv-IgAb_73** | **EpCAM** | **42** | **NKp46** | **NKp46-2** |
| **scFv-IgAb_75** | **EpCAM** | **42** | **NKG2D** | **KYK_2_0** |
| **scFv-IgAb_80** | **EpCAM** | **42** | **CD16A** | **P2C-47** |
| **IgG anti-EpCAM** | **EpCAM** | **42** | **n.a.** | **n.a.** |
| **^{§}TandAb** | **CD30** | **HRS-3** | **CD16A** | **LSIV21** |
| **^{§}IgG anti-CD30** | **CD30** | **HRS-3** | **n.a.** | **n.a.** |
| **^{§}Fc-enh IgG anti-CD30** | **CD30** | **HRS-3** | **n.a.** | **n.a.** |

| | | | | |
|---|---|---|---|---|
| ^{§}for construction, production, and purification of CD30/CD16A TandAb, IgG anti-CD30, and Fc-enhanced IgG anti-CD30 see Reusch et al., MAbs. 2014 May-Jun;6(3):728-39. | | | | |

### Culture of cell lines

COLO 205 (Kindly provided by Dr. G. Moldenhauer, German Cancer Research Center, Heidelberg, Germany) and KARPAS-299 cells (DSMZ, cat.: ACC31) were cultured in RPMI 1640 medium supplemented with 10% heat-inactivated FCS, 2 mM L-glutamine and 100 IU/mL penicillin G sodium and 100 µg/mL streptomycin sulfate. All cell lines were cultured under standard conditions and sub-cultured as recommended by the supplier at 37°C in a humidified atmosphere with 5% CO₂.

### Isolation of PBMC from buffy coats and enrichment of human NK cells

PBMCs were isolated from buffy coats (German Red Cross, Mannheim, Germany) by density gradient centrifugation. The buffy coat samples were diluted with a two-to-threefold volume of PBS (Invitrogen, cat.: 14190-169), layered on a cushion of Lymphoprep (Stem Cell Technologies, cat.: 07861) and centrifuged at 800 × g for 25 min at room temperature w/o brake. PBMC located in the interface were collected and washed 3 times with PBS before they were cultured in complete RPMI 1640 medium (RPMI 1640 medium supplemented 10% heat-inactivated FCS, 2 mM L-glutamine and 100 IU/mL penicillin G sodium and 100 µg/mL streptomycin sulfate (all components from Invitrogen)) overnight without stimulation. For the enrichment of NK cells PBMC were harvested from overnight cultures and used for one round of negative selection using the EasySep^{™} Human NK Cell Enrichment Kit (Stem Cell Technologies, cat.: 17055) for the immunomagnetic isolation of untouched human NK cells and the Big Easy EasySep^{™} Magnet (Stem Cell Technologies, cat.: 18001) according to the manufacturer's instructions.

### Preloading of NK cells and freeze/thaw

Aliquots of enriched primary human NK cells were resuspended in complete RPMI 1640 medium containing 10 µg/mL of the indicated antibody constructs in a total volume of 0.5 mL and incubated for 30 min at room temperature. After adding 0.5 mL FCS supplemented with 20% DMSO (Sigma) cell suspensions were then transferred to 2 mL cryo tubes (Greiner bio-one) and frozen at -80°C in cryo 1°C freezing container (Nalgene) for more than 12 h.

### 4 h calcein-release cytotoxicity assays

For calcein-release cytotoxicity assays the indicated target cells were harvested from cultures, washed with RPMI 1640 medium without FCS, and labeled with 10 µM calcein AM (Invitrogen/Molecular Probes, cat.: C3100MP) for 30 min in RPMI medium without FCS at 37°C. After gently washing the labeled cells were resuspended in complete RPMI 1640 medium to a density of 1×10⁵/mL, and aliquots of 1×10⁴ target cells were then seeded in individual wells of a 96-well round-bottom micro plate. In parallel, NK cells were thawed from -80°C by incubation of the cryo tubes in a 37°C water bath. When the last ice crystals were visible, the cell suspension was diluted with complete RPMI 1640 medium centrifuged and washed once before the cells were added to the target cells at the indicated effector-to-target (E:T) ratio with or without addition of fresh antibody as indicated in a total volume of 200 µL in duplicates. Spontaneous release was determined by incubation of target cells in the absence of effector cells and without antibodies, and maximal release was induced by the addition of 1% Triton x-100 (final concentration, Roth) and also measured in quadruplicate on each plate.

After centrifugation for 2 min at 200 g the assay was incubated for 4 h at 37°C in a humidified atmosphere with 5% CO₂. 100 µL cell culture supernatant were harvested from each well after an additional centrifugation for 5 min at 500 g, and the fluorescence of the released calcein was measured at 520 nm using a fluorescence multi-plate reader (Victor 3 or EnSight, Perkin Elmer). On the basis of the measured counts, the specific cell lysis was calculated according to the following formula: [fluorescence (sample) - fluorescence (spontaneous)] / [fluorescence (maximum) - fluorescence (spontaneous)] × 100%. Fluorescence (spontaneous) represents the fluorescent counts from target cells in the absence of effector cells and antibodies and fluorescence (maximum) represents the total cell lysis induced by the addition of Triton X-100. Lysis values were analyzed and mean and standard deviation were plotted using the GraphPad Prism software (GraphPad Prism version 7.04 for Windows, GraphPad Software, La Jolla California USA).

### Results:

Primary human NK cells that were pre-loaded with anti-EpCAM antibody constructs, such as EpCAM/NKp46 scFv-lgAb_73, EpCAM/NKG2D scFv-lgAb_75, EpCAM/CD16A scFv-lgAb_80, and IgG anti-EpCAM induced specific lysis of EpCAM-positive COLO 205 cells but not of EpCAM-negative KARPAS-299 cells. Among the bispecific anti-EpCAM constructs, NK cells preloaded with EpCAM/CD16A scFv-lgAb_80 exhibited the highest efficacy in COLO 205 cell lysis and NK cells loaded with EpCAM/NKG2D scFv-lgAb_75 the lowest efficacy. None of the NK cell preparations preloaded with anti-CD30 antibody constructs mediated substantial lysis of COLO 205 (Figure 8A and Table 5).

In contrast, NK cells that were pre-loaded with CD30/CD16A TandAb or Fc-enhanced IgG anti-CD30 mediated lysis of CD30-positive KARPAS-299 cells but not of CD30-negative COLO 205 cells. NK cells preloaded with wildtype IgG anti-CD30 did not mediate lysis of KARPAS-299 suggesting that this wildtype IgG anti-CD30 dissociated too fast from NK cells during freezing, thawing and washing, and that the number of remaining IgG was too low to mediate substantial target cell lysis (Figure 8B and Table 5).

Interstingly, NK cells that were preloaded with 10 µg/mL EpCAM/NKG2D scFv-lgAb_75 or CD30/CD16A TandAb, frozen, thawed, and washed induced similar lysis of COLO 205 or KARPAS-299 cells, respectively, as NK cells that were not preloaded but supplemented in the cytotoxicity assay with 10 µg/mL of fresh EpCAM/NKG2D scFv-lgAb_75 or CD30/CD16A TandAb.

**Table 5: Efficacy of target cell lysis by pre-loaded and frozen NK cells at an E:T ratio of 5:1. NK cells were preloaded with 10 µg/mL of the indicated antibody constructs or without antibody (w/o antibody) for 30 min at room temperature and then frozen at -80°C. Thawed NK cells were washed and used as effector cells in a 4 h calcein-release cytotoxicity assay on COLO 205 or KARPAS-299 target cells. As a control, EpCAM/NKG2D scFv-lgAb_75 and CD30/CD16A T116 were freshly added at a concentration of 10 µg/mL to NK cells that were not preloaded prior freezing (w/o antibody). Mean of duplicate lysis values at an E:T ratio of 5:1 are shown. Exp. No. RBL 1464.**

| **Efficacy [%] of target cell lysis at an E:T ratio of 5:1** | | |
|---|---|---|
| **Antibody construct** | **COLO 205** | **KARPAS-299** |
| EpCAM/NKp46 scFv-IgAb_73 | 51.7 | 2.8 |
| EpCAM/NKG2D scFv-IgAb_75 | 22.2 | 4.4 |
| EpCAM/CD16A scFv-IgAb_80 | 100.9 | 1.8 |
| IgG anti-EpCAM | 32.4 | 2.4 |
| CD30/CD16A TandAb | -2.7 | 86.1 |
| IgG anti-CD30 | -1.9 | 5.1 |
| Fc-enh. IgG anti-CD30 | 5.1 | 43.0 |
| w/o antibody | 8.2 | 3.2 |
| w/o antibody + EpCAM/NKG2D scFv-IgAb_75 | 33.5 | 5.7 |
| w/o antibody + CD30/CD16A T116 | 4.9 | 90.4 |

| **Seq ID NO** | **Description** | **Sequence** |
|---|---|---|
| 1 | Linker L1 | GGSGGS |
| 2 | Linker L2 | GGSGGSGGSGGSGGSGGS |
| 3 | Linker L3 | GGSGGSGGSGGSGGSGGSGGS |
| 4 | Linker L4 | GGSGGSGGS |
| 5 | Connector 1 | GGGGS |
| 6 | Connector 2 | GGGGSGGGGS |
| 7 | Connector 3 | GGGGSGGGGSGGGGSGGGGS |
| 8 | Connector 4 | GGGGSGGGGSGGGGSGGGGSGGGGSGGGGS |
| 9 | hinge | EPKSCDKTHTCPPCP |
| 10 | middle.hinge | DKTHTCPPCP |
| 11 | Human IgG1 CH1, CH2 and CHR heavy chain constant domain with silencing mutations | ASTKGPSVFPLAPSSKSTSGGTAALGCLVKDYFPEPVTVSWNSGALTSGVHTFPAVLQSSGLYSLSSVVTVPSSSLGTQTYICNVNHKP SNTKVDKKVEPKSCDKTHTCPPCPAPEFEGGPSVFLFPPKPKDTLMISRTPEVTCVVVAVSHEDPEVKFNWYVDGVEVHNAKTKPREE QYNSTYRVVSVLTVLHQDWLNGKEYKCKVSNKALPAPIEKTISKAKGQPREPQVYTLPPSREEMTKNQVSLTCLVKGFYPSDIAVEWE SNGQPENNYKTTPPVLDSDGSFFLYSKLTVDKSRWQQGNVFSCSVMHEALHNHYTQKSLSLSPG |
| 12 | Human IgG1 CH1, CH2 and CHR heavy chain constant domain (wild-type) | ASTKGPSVFPLAPSSKSTSGGTAALGCLVKDYFPEPVTVSWNSGALTSGVHTFPAVLQSSGLYSLSSVVVPSSSLGTQTY ICNVNHKPSNTKVDKKVEPKSCDKTHTCPPCPAPELLGGPSVFLFPPKPKDTLMISRTPEVTCVVVDVSHEDPEVKFNWY VDGVEVHNAKTKPREEQYNSTYRVVSVLTVLHQDWLNGKEYKCKVSNKALPAPIEKTISKAKGQPREPQVYTLPPSREEM TKNQVSLTCLVKGFYPSDIAVEWESNGQPENNYKTTPPVLDSDGSFFLYSKLTVDKSRWQQGNVFSCSVMHEALHNHYTQ KSLSLSPG |
| 13 | Human lambda light chain constant domain | GQPKAAPSVTLFPPSSEELQANKATLVCLISDFYPGAVTVAWKADSSPVKAGVETTTPSKQSNNKYAASSYLSLTPEQWK SHRSYSCQVTHEGSTVEKTVAPTECS |
| 14 | Human Kappa light chain constant domain | |
| 15 | CH2-CH3 heavy chain constant domain with silencing mutations | APEFEGGPSVFLFPPKPKDTLMISRTPEVTCVVVAVSHEDPEVKFNWYVDGVEVHNAKTKPREEQYNSTYRVVSVLTVLH QDWLNGKEYKCKVSNKALPAPIEKTISKAKGQPREPQVYTLPPSREEMTKNQVSLTCLVKGFYPSDIAVEWESNGQPENN YKTTPPVLDSDGSFFLYSKLTVDKSRWQQGNVFSCSVMHEALHNHYTQKSLSLSPG |
| 16 | Monomeric CH2-CH3 heavy chain constant domain with silencing mutations | APEFEGGPSVFLFPPKPKDTLMISRTPEVTCVVVAVSHEDPEVKFNWYVDGVEVHNAKTKPREEQYNSTYRVVSVLTVLH QDWLNGKEYKCKVSNKALPAPIEKTISKAKGQPREPQVYTKPPSRDELTKNQVSLSCLVKGFYPSDIAVEWESNGQPENN YKTTVPVLDSDGSFRLASYLTVDKSRWQQGNVFSCSVMHEALHNHYTQKSLSLSP |
| 17 | Knob-chain: CH2-CH3 heavy chain constant domain with silencing mutations | APEFEGGPSVFLFPPKPKDTLMISRTPEVTCVVVAVSHEDPEVKFNWYVDGVEVHNAKTKPREEQYNSTYRVVSVLTVLH QDWLNGKEYKCKVSNKALPAPIEKTISKAKGQPREPQVYTLPPSREEMTKNQVSLYCLVKGFYPSDIAVEWESNGQPENN YKTTPPVLDSDGSFFLYSKLTVDKSRWQQGNVFSCSVMHEALHNHYTQKSLSLSPG |
| 18 | Hole-chain: CH2-CH3 heavy chain constant domain with silencing mutations | APEFEGGPSVFLFPPKPKDTLMISRTPEVTCVVVAVSHEDPEVKFNWYVDGVEVHNAKTKPREEQYNSTYRVVSVLTVLH QDWLNGKEYKCKVSNKALPAPIEKTISKAKGQPREPQVYTLPPSREEMTKNQVSLTCLVKGFYPSDIAVEWESNGQPENN YKTTPPVLDSDGSFFLTSKLTVDKSRWQQGNVFSCSVMHEALHNHYTQKSLSLSPG |
| 19 | CH1 heavy chain constant domain | ASTKGPSVFPLAPSSKSTSGGTAALGCLVKDYFPEPVTVSWNSGALTSGVHTFPAVLQSSGLYSLSSVVTVPSSSLGTQT YICNVNHKPSNTKVCKKV |
| 20 | Human lambda light chain constant domain with point-mutation at the C-terminus | GQPKAAPSVTLFPPSSEELQANKATLVCLISDFYPGAVTVAWKADSSPVKAGVETTTPSKQSNNKYAASSYLSLTPEQWK SHRSYSCQVTHEGSTVEKTVAPTESS |
| 21 | Human kappa light chain constant domain with point-mutation at the C-terminus | RTVAAPSVFIFPPSDEQLKSGTASVVCLLNNFYPREAKVQWKVDNALQSGNSQESVTEQDSKDSTYSLSSTLTLSKADYE KHKVYACEVTHQGLSSPVTKSFNRGES |
| 22 | CH2-CH3 heavy chain constant domain (wild-type) | APELLGGPSVFLFPPKPKDTLMISRTPEVTCVVVDVSHEDPEVKFNWYVDGVEVHNAKTKPREEQYNSTYRVVSVLTVLH QDWLNGKEYKCKVSNKALPAPIEKTISKAKGQPREPQVYTLPPSREEMTKNQVSLTCLVKGFYPSDIAVEWESNGQPENN YKTTPPVLDSDGSFFLYSKLTVDKSRWQQGNVFSCSVMHEALHNHYTQKSLSLSPG |
| 23 | Human CD16A | GMRTEDLPKAVVFLEPQWYRVLEKDSVTLKCQGAYSPEDNSTQWFHNESLISSQASSYFIDAATVDDSGEYRCQTNLSTL SDPVQLEVHIGWLLLQAPRWVFKEEDPIHLRCHSWKNTALHKVTYLQNGKGRKYFHHNSDFYIPKATLKDSGSYFCRGLF GSKNVSSETVNITITQGLAVSTISSFFPPGYQ |
| 24 | Cynomolgus CD16A | GMRAEDLPKAVVFLEPQWYRVLEKDRVTLKCQGAYSPEDNSTRWFHNESLISSQTSSYFIAAARVNNSGEYRCQTSLSTL SDPVQLEVHIGWLLLQAPRWVFKEEESIHLRCHSWKNTLLHKVTYLQNGKGRKYFHQNSDFYIPKATLKDSGSYFCRGLI GSKNVSSETVNITITQDLAVSSISSFFPPGYQ |
| 25 | His-tag | HHHHHH |
| 26 | C-Tag | EPEA |
| 27 | VH HSA | EVQLLESGGGLVQPGGSLRLSCAVSGIDLSNYAINWVRQAPGKGLEWIGIIWASGTTFYATWAKGRFTISRDNSKNTVYL QMNSLRAEDTAVYYCARTVPGYSTAPYFDLWGQGTLVTVSS |
| 28 | VL HSA | DIQMTQSPSSVSASVGDRVTITCQSSPSVWSNFLSWYQQKPGKAPKLLIYEASKLTSGVPSRFSGSGSGTDFTLTISSLQ PEDFATYYCGGGYSSISDTTFGGGTKVEIK |
| 29 | CDR-H1 CD16A (CD16-1) | GYTFTSYY |
| 30 | CDR-H2 CD16A (CD16-1) | INPSGGST |
| 31 | CDR-H3 CD16A (CD16-1) | ARGSAYYYDFADY |
| 32 | CDR-L1 CD16A (CD16-1) | NIGSKN |
| 33 | CDR-L2 CD16A (CD16-1) | QDN |
| 34 | CDR-L3 CD16A (CD16-1) | QVWDNYSVL |
| 35 | VH CD16A (CD16-1) | QVQLVQSGAEVKKPGESLKVSCKASGYTFTSYYMHWVRQAPGQGLEWMGIINPSGGSTSYAQKFQGRVTMTRDTSTSTVY MELSSLRSEDTAVYYCARGSAYYYDFADYWGQGTLVTVSS |
| 36 | VL CD16A (CD16-1) | SYVLTQPSSVSVAPGQTATISCGGHNIGSKNVHWYQQRPGQSPVLVIYQDNKRPSGIPERFSGSNSGNTATLTISGTQAM DEADYYCQVWDNYSVLFGGGTKLTVL |
| 37 | scFv CD16A (CD16-1) | QVQLVQSGAEVKKPGESLKVSCKASGYTFTSYYMHWVRQAPGQGLEWMGIINPSGGSTSYAQKFQGRVTMTRDTSTSTVY MELSSLRSEDTAVYYCARGSAYYYDFADYWGQGTLVTVSSGGSGGSGGSGGSGGSGGSSYVLTQPSSVSVAPGQTATISC GGHNIGSKNVHWYQQRPGQSPVLVIYQDNKRPSGIPERFSGSNSGNTATLTISGTQAMDEADYYCQVWDNYSVLFGGGTK LTVLAAAGSHHHHHH |
| 38 | Fab VH CD16A (CD16-1) | QVQLVQSGAEVKKPGESLKVSCKASGYTFTSYYMHWVRQAPGQGLEWMGIINPSGGSTSYAQKFQGRVTMTRDTSTSTVY MELSSLRSEDTAVYYCARGSAYYYDFADYWGQGTLVTVSSASTKGPSVFPLAPSSKSTSGGTAALGCLVKDYFPEPVTVS WNSGALTSGVHTFPAVLQSSGLYSLSSVVTVPSSSLGTQTYICNVNHKPSNTKVDKKV |
| 39 | Fab VL CD16A (CD16-1) | SYVLTQPSSVSVAPGQTATISCGGHNIGSKNVHWYQQRPGQSPVLVIYQDNKRPSGIPERFSGSNSGNTATLTISGTQAM DEADYYCQVWDNYSVLFGGGTKLTVLGQPKAAPSVTLFPPSSEELQANKATLVCLISDFYPGAVTVAWKADSSPVKAGVE TTT[?]SKQSNNKYAASSYLSLTPEQWKSHRSYSCQVTHEGSTVEKTVAPTECS |
| 40 | CDR-H1 CD16A (CD16-2) | GYTFTSYY |
| 41 | CDR-H2 CD16A (CD16-2) | IEPMYGST |
| 42 | CDR-H3 CD16A (CD16-2) | ARGSAYYYDFADY |
| 43 | CDR-L1 CD16A (CD16-2) | NIGSKN |
| 44 | CDR-L2 CD16A (CD16-2) | QDN |
| 45 | CDR-L3 CD16A (CD16-2) | QVWDNYSVL |
| 46 | VH CD16A (CD16-2) | QVQLVQSGAEVKKPGESLKVSCKASGYTFTSYYMHWVRQAPGQGLEWMGAIEPMYGSTSYAQKFQGRVTMTRDTSTSTVY MELSSLRSEDTAVYYCARGSAYYYDFADYWGQGTLVTVSS |
| 47 | VL CD16A (CD16-2) | SYVLTQPSSVSVAPGQTATISCGGHNIGSKNVHWYQQRPGQSPVLVIYQDNKRPSGIPERFSGSNSGNTATLTISGTQAM DEADYYCQVWDNYSVLFGGGTKLTVL |
| 48 | scFv CD16A (CD16-2) | QVQLVQSGAEVKKPGESLKVSCKASGYTFTSYYMHWVRQAPGQGLEWMGAIEPMYGSTSYAQKFQGRVTMTRDTSTSTVY MELSSLRSEDTAVYYCARGSAYYYDFADYWGQGTLVTVSSGGSGGSGGSGGSGGSGGSSYVLTQPSSVSVAPGQTATISC GGHNIGSKNVHWYQQRPGQSPVLVIYQDNKRPSGIPERFSGSNSGNTATLTISGTQAMDEADYYCQVWDNYSVLFGGGTK LTVLAAAGSHHHHHH |
| 49 | Fab VH CD16A (CD16-2) | QVQLVQSGAEVKKPGESLKVSCKASGYTFTSYYMHWVRQAPGQGLEWMGAIEPMYGSTSYAQKFQGRVTMTRDTSTSTVY MELSSLRSEDTAVYYCARGSAYYYDFADYWGQGTLVTVSSASTKGPSVFPLAPSSKSTSGGTAALGCLVKDYFPEPVTVS WNSGALTSGVHTFPAVLQSSGLYSLSSVVTVPSSSLGTQTYICNVNHKPSNTKVDKKV |
| 50 | Fab VL CD16A (CD16-2) | SYVLTQPSSVSVAPGQTATISCGGHNIGSKNVHWYQQRPGQSPVLVIYQDNKRPSGIPERFSGSNSGNTATLTISGTQAM DEADYYCQVWDNYSVLFGGGTKLTVLGQPKAAPSVTLFPPSSEELQANKATLVCLISDFYPGAVTVAWKADSSPVKAGVE TTT[?]SKQSNNKYAASSYLSLTPEQWKSHRSYSCQVTHEGSTVEKTVAPTECS |
| 51 | CDR-H1 CD16A (CD16-3) | GYTFTNYY |
| 52 | CDR-H2 CD16A (CD16-3) | INPGGGST |
| 53 | CDR-H3 CD16A (CD16-3) | ARGSAYYYDFADY |
| 54 | CDR-L1 CD16A (CD16-3) | NIGSQS |
| 55 | CDR-L2 CD16A (CD16-3) | QDS |
| 56 | CDR-L3 CD16A (CD16-3) | QVWDNYSW |
| 57 | VH CD16A (CD16-3) | QVQLVQSGAEVKKPGESLKVSCKASGYTFTNYYMQWVRQAPGQGLEWMGVINPGGGSTSYAQKFQGRVTMTRDTSTSTVY MELSSLRSEDTAVYYCARGSAYYYDFADYWGQGTLVTVSS |
| 58 | VL CD16A (CD16-3) | SYVLTQPSSVSVAPGQTARITCGGHNIGSQSVHWYQQKPGQAPVLVIYQDSKRPSGIPERFSGSNSGNTATLTISGTQAM DEADYYCQVWDNYSVVFGGGTKLTVL |
| 59 | scFv CD16A (CD16-3) | QVQLVQSGAEVKKPGESLKVSCKASGYTFTNYYMQWVRQAPGQGLEWMGVINPGGGSTSYAQKFQGRVTMTRDTSTSTVY MELSSLRSEDTAVYYCARGSAYYYDFADYWGQGTLVTVSSGGSGGSGGSGGSGGSGGSSYVLTQPSSVSVAPGQTARITC GGHNIGSQSVHWYQQKPGQAPVLVIYQDSKRPSGIPERFSGSNSGNTATLTISGTQAMDEADYYCQVWDNYSVVFGGGTK LTVLAAAGSHHHHHH |
| 60 | Fab VH CD16A (CD16-3) | QVQLVQSGAEVKKPGESLKVSCKASGYTFTNYYMQWVRQAPGQGLEWMGVINPGGGSTSYAQKFQGRVTMTRDTSTSTVY MELSSLRSEDTAVYYCARGSAYYYDFADYWGQGTLVTVSSASTKGPSVFPLAPSSKSTSGGTAALGCLVKDYFPEPVTVS WNSGALTSGVHTFPAVLQSSGLYSLSSVVTVPSSSLGTQTYICNVNHKPSNTKVDKKV |
| 61 | Fab VL CD16A (CD16-3) | SYVLTQPSSVSVAPGQTARITCGGHNIGSQSVHWYQQKPGQAPVLVIYQDSKRPSGIPERFSGSNSGNTATLTISGTQAM DEADYYCQVWDNYSVVFGGGTKLTVLGQPKAAPSVTLFPPSSEELQANKATLVCLISDFYPGAVTVAWKADSSPVKAGVE TTTPSKQSNNKYAASSYLSLTPEQWKSHRSYSCQVTHEGSTVEKTVAPTECS |
| 62 | CDR-H1 CD16A (CD16-4) | GYSFSDFY |
| 63 | CDR-H2 CD16A (CD16-4) | INPGGAST |
| 64 | CDR-H3 CD16A (CD16-4) | ARGSAYYYDFADY |
| 65 | CDR-L1 CD16A (CD16-4) | NIGRQS |
| 66 | CDR-L2 CD16A (CD16-4) | QDS |
| 67 | CDR-L3 CD16A (CD16-4) | QVWDNYTVV |
| 68 | VH CD16A (CD16-4) | QVQLVQSGAEVKKPGESLKVSCKASGYSFSDFYIQWVRQAPGQGLEWMGIINPGGASTTYAQKFQGRVTMTRDTSTSTVY MELSSLRSEDTAVYYCARGSAYYYDFADYWGQGTLVTVSS |
| 69 | VL CD16A (CD16-4) | SYVLTQPSSVSVAPGQTARITCGGYNIGRQSVHWYQQKPGQAPVLVIYQDSKRPSGIPERFSGSNSGNTATLTISGTQAM DEADYYCQVWDNYTVVFGGGTKLTVL |
| 70 | scFv CD16A (CD16-4) | QVQLVQSGAEVKKPGESLKVSCKASGYSFSDFYIQWVRQAPGQGLEWMGIINPGGASTTYAQKFQGRVTMTRDTSTSTVY MELSSLRSEDTAVYYCARGSAYYYDFADYWGQGTLVTVSSGGSGGSGGSGGSGGSGGSSYVLTQPSSVSVAPGQTARITC GGYNIGRQSVHWYQQKPGQAPVLVIYQDSKRPSGIPERFSGSNSGNTATLTISGTQAMDEADYYCQVWDNYTVVFGGGTK LTVLAAAGSHHHHHH |
| 71 | Fab VH CD16A (CD16-4) | QVQLVQSGAEVKKPGESLKVSCKASGYSFSDFYIQWVRQAPGQGLEWMGIINPGGASTTYAQKFQGRVTMTRDTSTSTVY MELSSLRSEDTAVYYCARGSAYYYDFADYWGQGTLVTVSSASTKGPSVFPLAPSSKSTSGGTAALGCLVKDYFPEPVTVS WNSGALTSGVHTFPAVLQSSGLYSLSSVVTVPSSSLGTQTYICNVNHKPSNTKVDKKV |
| 72 | Fab VL CD16A (CD16-4) | SYVLTQPSSVSVAPGQTARITCGGYNIGRQSVHWYQQKPGQAPVLVIYQDSKRPSGIPERFSGSNSGNTATLTISGTQAM DEADYYCQVWDNYTVVFGGGTKLTVLGQPKAAPSVTLFPPSSEELQANKATLVCLISDFYPGAVTVAWKADSSPVKAGVE TTTPSKQSNNKYAASSYLSLTPEQWKSHRSYSCQVTHEGSTVEKTVAPTECS |
| 73 | CDR-H1 CD16A (CD16-5) | GYTFSSYY |
| 74 | CDR-H2 CD16A (CD16-5) | IEPRGVRI |
| 75 | CDR-H3 CD16A (CD16-5) | ARGSAYYYDFADY |
| 76 | CDR-L1 CD16A (CD16-5) | NIGSTN |
| 77 | CDR-L2 CD16A (CD16-5) | QDS |
| 78 | CDR-L3 CD16A (CD16-5) | QVWDNYSVQ |
| 79 | VH CD16A (CD16-5) | QVQLVQSGAEVKKPGESLKVSCKASGYTFSSYYMHWVRQAPGQGLEWMGAIEPRGVRISYAQKFQGRVTMTRDTSTSTVY MELSSLRSEDTAVYYCARGSAYYYDFADYWGQGTLVTVSS |
| 80 | VL CD16A (CD16-5) | SYVLTQPSSVSVAPGQTARITCGGHNIGSTNVHWYQQKPGQAPVLVIYQDSKRPSGIPERFSGSNSGNTATLTISGTQAM DEADYYCQVWDNYSVQFGGGTKLTVL |
| 81 | scFv CD16A (CD16-5) | QVQLVQSGAEVKKPGESLKVSCKASGYTFSSYYMHWVRQAPGQGLEWMGAIEPRGVRISYAQKFQGRVTMTRDTSTSTVY MELSSLRSEDTAVYYCARGSAYYYDFADYWGQGTLVTVSSGGSGGSGGSGGSGGSGGSSYVLTQPSSVSVAPGQTARITC GGHNIGSTNVHWYQQKPGQAPVLVIYQDSKRPSGIPERFSGSNSGNTATLTISGTQAMDEADYYCQVWDNYSVQFGGGTK LTVLAAAGSHHHHHH |
| 82 | Fab VH CD16A (CD16-5) | QVQLVQSGAEVKKPGESLKVSCKASGYTFSSYYMHWVRQAPGQGLEWMGAIEPRGVRISYAQKFQGRVTMTRDTSTSTVY MELSSLRSEDTAVYYCARGSAYYYDFADYWGQGTLVTVSSASTKGPSVFPLAPSSKSTSGGTAALGCLVKDYFPEPVTVS WNSGALTSGVHTFPAVLQSSGLYSLSSVVTVPSSSLGTQTYICNVNHKPSNTKVDKKV |
| 83 | Fab VL CD16A (CD16-5) | SYVLTQPSSVSVAPGQTARITCGGHNIGSTNVHWYQQKPGQAPVLVIYQDSKRPSGIPERFSGSNSGNTATLTISGTQAM DEADYYCQVWDNYSVQFGGGTKLTVLGQPKAAPSVTLFPPSSEELQANKATLVCLISDFYPGAVTVAWKADSSPVKAGVE TTTPSKQSNNKYAASSYLSLTPEQWKSHRSYSCQVTHEGSTVEKTVAPTECS |
| 84 | CDR-H1 CD16A (CD16-6) | GYTFTNYY |
| 85 | CDR-H2 CD16A (CD16-6) | INPSGGVT |
| 86 | CDR-H3 CD16A (CD16-6) | ARGSAYYYDFADY |
| 87 | CDR-L1 CD16A (CD16-6) | NIGSKS |
| 88 | CDR-L2 CD16A (CD16-6) | QDK |
| 89 | CDR-L3 CD16A (CD16-6) | QVWDDYIVL |
| 90 | VH CD16A (CD16-6) | QVQLVQSGAEVKKPGESLKVSCKASGYTFTNYYMQWVRQAPGQGLEWMGIINPSGGVTSYAQKFQGRVTMTRDTSTSTVY MELSSLRSEDTAVYYCARGSAYYYDFADYWGQGTLVTVSS |
| 91 | VL CD16A (CD16-6) | SYVLTQPSSVSVAPGQTARITCGGNNIGSKSVHWYQQKPGQAPVLVIYQDKKRPSGIPERFSGSNSGNTATLTISGTQAM DEADYYCQVWDDYIVLFGGGTKLTVL |
| 92 | scFv CD16A (CD16-6) | QVQLVQSGAEVKKPGESLKVSCKASGYTFTNYYMQWVRQAPGQGLEWMGIINPSGGVTSYAQKFQGRVTMTRDTSTSTVY MELSSLRSEDTAVYYCARGSAYYYDFADYWGQGTLVTVSSGGSGGSGGSGGSGGSGGSSYVLTQPSSVSVAPGQTARITC GGNNIGSKSVHWYQQKPGQAPVLVIYQDKKRPSGIPERFSGSNSGNTATLTISGTQAMDEADYYCQVWDDYIVLFGGGTK LTVLAAAGSHHHHHH |
| 93 | Fab VH CD16A (CD16-6) | QVQLVQSGAEVKKPGESLKVSCKASGYTFTNYYMQWVRQAPGQGLEWMGIINPSGGVTSYAQKFQGRVTMTRDTSTSTVY MELSSLRSEDTAVYYCARGSAYYYDFADYWGQGTLVTVSSASTKGPSVFPLAPSSKSTSGGTAALGCLVKDYFPEPVTVS WNSGALTSGVHTFPAVLQSSGLYSLSSVVTVPSSSLGTQTYICNVNHKPSNTKVDKKV |
| 94 | Fab VL CD16A (CD16-6) | SYVLTQPSSVSVAPGQTARITCGGNNIGSKSVHWYQQKPGQAPVLVIYQDKKRPSGIPERFSGSNSGNTATLTISGTQAM DEADYYCQVWDDYIVLFGGGTKLTVLGQPKAAPSVTLFPPSSEELQANKATLVCLISDFYPGAVTVAWKADSSPVKAGVE TTTPSKQSNNKYAASSYLSLTPEQWKSHRSYSCQVTHEGSTVEKTVAPTECS |
| 95 | CDR-H1 CD16A (CD16-7) | GYTFTNYY |
| 96 | CDR-H2 CD16A (CD16-7) | IEPDGGRR |
| 97 | CDR-H3 CD16A (CD16-7) | ARGSAYYYDFADY |
| 98 | CDR-L1 CD16A (CD16-7) | QGVSGD |
| 99 | CDR-L2 CD16A (CD16-7) | QAN |
| 100 | CDR-L3 CD16A (CD16-7) | QQWDNYSVT |
| 101 | VH CD16A (CD16-7) | QVQLVQSGAEVKKPGESLKVSCKASGYTFTNYYMHWVRQAPGQGLEWMGVIEPDGGRRTYAQKFQGRVTMTRDTSTSTVY MELSSLRSEDTAVYYCARGSAYYYDFADYWGQGTLVTVSS |
| 102 | VL CD16A (CD16-7) | EIVLTQSPATLSLSPGERATLSCRGHQGVSGDVHWYQQKPGQAPRLLIYQANKRASGIPARFSGSGSGTEFTLTISSLEP EDFAVYYCQQWDNYSVTFGQGTKVEIK |
| 103 | scFv CD16A (CD16-7) | QVQLVQSGAEVKKPGESLKVSCKASGYTFTNYYMHWVRQAPGQGLEWMGVIEPDGGRRTYAQKFQGRVTMTRDTSTSTVY MELSSLRSEDTAVYYCARGSAYYYDFADYWGQGTLVTVSSGGSGGSGGSGGSGGSGGSEIVLTQSPATLSLSPGERATLS CRGHQGVSGDVHWYQQKPGQAPRLLIYQANKRASGIPARFSGSGSGTEFTLTISSLEPEDFAVYYCQQWDNYSVTFGQGT KVEIKAAAGSHHHHHH |
| 104 | Fab VH CD16A (CD16-7) | QVQLVQSGAEVKKPGESLKVSCKASGYTFTNYYMHWVRQAPGQGLEWMGVIEPDGGRRTYAQKFQGRVTMTRDTSTSTVY MELSSLRSEDTAVYYCARGSAYYYDFADYWGQGTLVTVSSASTKGPSVFPLAPSSKSTSGGTAALGCLVKDYFPEPVTVS WNSGALTSGVHTFPAVLQSSGLYSLSSVVTVPSSSLGTQTYICNVNHKPSNTKVDKKV |
| 105 | Fab VL CD16A (CD16-7) | EIVLTQSPATLSLSPGERATLSCRGHQGVSGDVHWYQQKPGQAPRLLIYQANKRASGIPARFSGSGSGTEFTLTISSLEP EDFAVYYCQQWDNYSVTFGQGTKVEIKRTVAAPSVFIFPPSDEQLKSGTASVVCLLNNFYPREAKVQWKVDNALQSGNSQ ESVTEQDSKDSTYSLSSTLTLSKADYEKHKVYACEVTHQGLSSPVTKSFNRGEC |
| 106 | CDR-H1 BCMA (BCMA-1) | GFTFSNYD |
| 107 | CDR-H2 BCMA (BCMA-1) | ISTRGDIT |
| 108 | CDR-H3 BCMA (BCMA-1) | ARQDYYTDYMGFAY |
| 109 | CDR-L1 BCMA (BCMA-1) | EDIYNG |
| 110 | CDR-L2 BCMA (BCMA-1) | GAS |
| 111 | CDR-L3 BCMA (BCMA-1) | AGPHKYPLT |
| 112 | VH BCMA (BCMA-1) | EVQLLESGGGLVQPGGSLRLSCAASGFTFSNYDMAWVRQAPGKGLEWVSSISTRGDITSYRDSVKGRFTISRDNSKNTLY LQMNSLRAEDTAVYYCARQDYYTDYMGFAYWGQGTLVTVSS |
| 113 | VL BCMA (BCMA-1) | AIQMTQSPSSLSASVGDRVTITCRASEDIYNGLAWYQQKPGKAPKLLIYGASSLQDGVPSRFSGSGSGTEFTLTISSLQP EDEATYYCAGPHKYPLTFGGGTKVEIK |
| 114 | scFv BCMA (BCMA-1) | EVQLLESGGGLVQPGGSLRLSCAASGFTFSNYDMAWVRQAPGKGLEWVSSISTRGDITSYRDSVKGRFTISRDNSKNTLY LQMNSLRAEDTAVYYCARQDYYTDYMGFAYWGQGTLVTVSSGGGGSGGGGSGGGGSAIQMTQSPSSLSASVGDRVTITCR ASEDIYNGLAWYQQKPGKAPKLLIYGASSLQDGVPSRFSGSGSGTEFTLTISSLQPEDEATYYCAGPHKYPLTFGGGTKV EIKAAAGSHHHHHH |
| 115 | Fab VH BCMA (BCMA-1) | EVQLLESGGGLVQPGGSLRLSCAASGFTFSNYDMAWVRQAPGKGLEWVSSISTRGDITSYRDSVKGRFTISRDNSKNTLY LQMNSLRAEDTAVYYCARQDYYTDYMGFAYWGQGTLVTVSSASTKGPSVFPLAPSSKSTSGGTAALGCLVKDYFPEPVTV SWNSGALTSGVHTFPAVLQSSGLYSLSSVVTVPSSSLGTQTYICNVNHKPSNTKVDKKV |
| 116 | Fab VL BCMA (BCMA-1) | AIQMTQSPSSLSASVGDRVTITCRASEDIYNGLAWYQQKPGKAPKLLIYGASSLQDGVPSRFSGSGSGTEFTLTISSLQP EDEATYYCAGPHKYPLTFGGGTKVEIKRTVAAPSVFIFPPSDEQLKSGTASVVCLLNNFYPREAKVQWKVDNALQSGNSQ ESVTEQDSKDSTYSLSSTLTLSKADYEKHKVYACEVTHQGLSSPVTKSFNRGEC |
| 117 | CDR-H1 BCMA (BCMA-2) | GFTFSNFD |
| 118 | CDR-H2 BCMA (BCMA-2) | ITTGGGDT |
| 119 | CDR-H3 BCMA (BCMA-2) | VRHGYYDGYHLFDYWG |
| 120 | CDR-L1 BCMA (BCMA-2) | QGISNN |
| 121 | CDR-L2 BCMA (BCMA-2) | YTS |
| 122 | CDR-L3 BCMA (BCMA-2) | QQFTSLPYT |
| 123 | VH BCMA (BCMA-2) | EVQLVESGGGLVQPGGSLRLSCAASGFTFSNFDMAWVRQAPGKGLVWVSSITTGGGDTYYADSVKGRFTISRDNAKSTLY LQMDSLRSEDTAVYYCVRHGYYDGYHLFDYWGQGTLVTVSS |
| 124 | VL BCMA (BCMA-2) | DIQMTQSPSSLSASVGDRVTITCRANQGISNNLNWYQQKPGKAPKPLIYYTSNLQSGVPSRFSGSGSGTDYTLTISSLQP EDFATYYCQQFTSLPYTFGQGTKLEIK |
| 125 | scFv BCMA (BCMA-2) | EVQLVESGGGLVQPGGSLRLSCAASGFTFSNFDMAWVRQAPGKGLVWVSSITTGGGDTYYADSVKGRFTISRDNAKSTLY LQMDSLRSEDTAVYYCVRHGYYDGYHLFDYWGQGTLVTVSSGGGGSGGGGSGGGGSDIQMTQSPSSLSASVGDRVTITCR ANQGISNNLNWYQQKPGKAPKPLIYYTSNLQSGVPSRFSGSGSGTDYTLTISSLQPEDFATYYCQQFTSLPYTFGQGTKL EIKAAAGSHHHHHH |
| 126 | Fab VH BCMA (BCMA-2) | EVQLVESGGGLVQPGGSLRLSCAASGFTFSNFDMAWVRQAPGKGLVWVSSITTGGGDTYYADSVKGRFTISRDNAKSTLY LQMDSLRSEDTAVYYCVRHGYYDGYHLFDYWGQGTLVTVSSASTKGPSVFPLAPSSKSTSGGTAALGCLVKDYFPEPVTV SWNSGALTSGVHTFPAVLQSSGLYSLSSVVVPSSSLGTQTYICNVNHKPSNTKVDKKV |
| 127 | Fab VL BCMA (BCMA-2) | DIQMTQSPSSLSASVGDRVTITCRANQGISNNLNWYQQKPGKAPKPLIYYTSNLQSGVPSRFSGSGSGTDYTLTISSLQP EDFATYYCQQFTSLPYTFGQGTKLEIKRTVAAPSVFIFPPSDEQLKSGTASVVCLLNNFYPREAKVQWKVDNALQSGNSQ ESVTEQDSKDSTYSLSSTLTLSKADYEKHKVYACEVTHQGLSSPVTKSFNRGEC |
| 128 | CDR-H1 EGFR (EGFR-1) | GSVSSGSYY |
| 129 | CDR-H2 EGFR (EGFR-1) | IYYSGST |
| 130 | CDR-H3 EGFR (EGFR-1) | ARNPISIPAFDI |
| 131 | CDR-L1 EGFR (EGFR-1) | NIGSKS |
| 132 | CDR-L2 EGFR (EGFR-1) | YDS |
| 133 | CDR-L3 EGFR (EGFR-1) | QVWDTSSDHVL |
| 134 | VH EGFR (EGFR-1) | QVQLQESGPGLVKPSETLSLTCTVSGGSVSSGSYYWSWIRQPPGKGLEWIGYIYYSGSTNYNPSLKSRVTISVDTSKNQF SLKLSSVTAADTAVYYCARNPISIPAFDIWGQGTMVTVSS |
| 135 | VL EGFR (EGFR-1) | QPVLTQPPSVSVAPGKTARITCGGNNIGSKSVHWYQQKPGQAPVLVIYYDSDRPSGIPERFSGSNSGNTATLTISRVEAG DEADYYCQVWDTSSDHVLFGGGTKLTVL |
| 136 | scFv EGFR (EGFR-1) | QVQLQESGPGLVKPSETLSLTCTVSGGSVSSGSYYWSWIRQPPGKGLEWIGYIYYSGSTNYNPSLKSRVTISVDTSKNQF SLKLSSVTAADTAVYYCARNPISIPAFDIWGQGTMVTVSSGGSGGSGGSGGSGGSGGSQPVLTQPPSVSVAPGKTARITC GGNNIGSKSVHWYQQKPGQAPVLVIYYDSDRPSGIPERFSGSNSGNTATLTISRVEAGDEADYYCQVWDTSSDHVLFGGG TKLTVLAAAGSHHHHHH |
| 137 | Fab VH EGFR (EGFR-1) | QVQLQESGPGLVKPSETLSLTCTVSGGSVSSGSYYWSWIRQPPGKGLEWIGYIYYSGSTNYNPSLKSRVTISVDTSKNQF SLKLSSVTAADTAVYYCARNPISIPAFDIWGQGTMVTVSSASTKGPSVFPLAPSSKSTSGGTAALGCLVKDYFPEPVTVS WNSGALTSGVHTFPAV[?]QSSGLYSLSSVVVPSSSLGTQTYICNVNHKPSNTKVDKKV |
| 138 | Fab VL EGFR (EGFR-1) | QPVLTQPPSVSVAPGKTARITCGGNNIGSKSVHWYQQKPGQAPVLVIYYDSDRPSGIPERFSGSNSGNTATLTISRVEAG DEADYYCQVWDTSSDHVLFGGGTKLTVLGQPKAAPSVTLFPPSSEELQANKATLVCLISDFYPGAVTVAWKADSSPVKAG VETTTPSKQSNNKYAASSYLSLTPEQWKSHRSYSCQVTHEGSTVEKTVAPTECS |
| 139 | CDR-H1 MHCcomplex (MHC-1) | GYMFSTFW |
| 140 | CDR-H2 MHCcomplex (MHC-1) | IYPGDSNT |
| 141 | CDR-H3 MHCcomplex (MHC-1) | AKIRDGYSYDAFDL |
| 142 | CDR-L1 MHCcomplex (MHC-1) | SSDVGGYNY |
| 143 | CDR-L2 MHCcomplex (MHC-1) | DVS |
| 144 | CDR-L3 MHCcomplex (MHC-1) | SSYTSSSTLAYV |
| 145 | VH MHCcomplex (MHC-1) | QVQLVQSGAEAKKPGESLRISCRASGYMFSTFWIGWVRQMPGKGLEWVASIYPGDSNTIYSPSFQGQVTISADKSINTTY LQWSGLKASDTATYYCAKIRDGYSYDAFDLWGQGTMVTVSS |
| 146 | VL MHCcomplex (MHC-1) | QSALTQPASVSGSPGQSITISCTGTSSDVGGYNYVSWYQQHPGKAPKLMIYDVSNRPSGVSNRFSGSKSGNTASLTISGL QAEDEADYYCSSYTSSSTLAYVFGTGTKLTVL |
| 147 | scFv MHCcomplex (MHC-1) | QVQLVQSGAEAKKPGESLRISCRASGYMFSTFWIGWVRQMPGKGLEWVASIYPGDSNTIYSPSFQGQVTISADKSINTTY LQWSGLKASDTATYYCAKIRDGYSYDAFDLWGQGTMVTVSSGGSGGSGGSGGSGGSGGSQSALTQPASVSGSPGQSITIS CTGTSSDVGGYNYVSWYQQHPGKAPKLMIYDVSNRPSGVSNRFSGSKSGNTASLTISGLQAEDEADYYCSSYTSSSTLAY VFGTGTKLTVLAAAGSHHHHHH |
| 148 | Fab VH MHCcomplex (MHC-1) | QVQLVQSGAEAKKPGESLRISCRASGYMFSTFWIGWVRQMPGKGLEWVASIYPGDSNTIYSPSFQGQVTISADKSINTTY LQWSGLKASDTATYYCAKIRDGYSYDAFDLWGQGTMVTVSSASTKGPSVFPLAPSSKSTSGGTAALGCLVKDYFPEPVTV SWNSGALTSGVHTFPAVLQSSGLYSLSSVVVPSSSLGTQTYICNVNHKPSNTKVDKKV |
| 149 | Fab VL MHCcomplex (MHC-1) | QSALTQPASVSGSPGQSITISCTGTSSDVGGYNYVSWYQQHPGKAPKLMIYDVSNRPSGVSNRFSGSKSGNTASLTISGL QAEDEADYYCSSYTSSSTLAYVFGTGTKLTVLGQPKAAPSVTLFPPSSEELQANKATLVCLISDFYPGAVTVAWKADSSP VKAGVETTTPSKQSNNKYAASSYLSLTPEQWKSHRSYSCQVTHEGSTVEKTVAPTECS |
| 150 | CDR-H1 MHCcomplex (MHC-2) | GFTFSSYA |
| 151 | CDR-H2 MHCcomplex (MHC-2) | ISYDGSNK |
| 152 | CDR-H3 MHCcomplex (MHC-2) | ARDGGYYHYGLDV |
| 153 | CDR-L1 MHCcomplex (MHC-2) | KLGDKY |
| 154 | CDR-L2 MHCcomplex (MHC-2) | QDA |
| 155 | CDR-L3 MHCcomplex (MHC-2) | QAWDSSTGV |
| 156 | VH MHCcomplex (MHC-2) | EVQLVESGGGVVQPGRSLRLSCAASGFTFSSYAMHWVRQAPGKGLEWVAVISYDGSNKYYADSVKGRFTISRDNSKNTLY LQMNSLRTEDTAVYYCARDGGYYHYGLDVWGQGTTVTVSS |
| 157 | VL MHCcomplex (MHC-2) | SYELTQPPSVSVSPGQTASITCSGDKLGDKYASWYQRKPGQSPVLLIYQDAKRPSGIPERFSGSNSGNTATLTISGTQAM DEADYYCQAWDSSTGVFGGGTKLTVL |
| 158 | scFv MHCcomplex (MHC-2) | EVQLVESGGGVVQPGRSLRLSCAASGFTFSSYAMHWVRQAPGKGLEWVAVISYDGSNKYYADSVKGRFTISRDNSKNTLY LQMNSLRTEDTAVYYCARDGGYYHYGLDVWGQGTTVTVSSGGSGGSGGSGGSGGSGGSSYELTQPPSVSVSPGQTASITC SGDKLGDKYASWYQRKPGQSPVLLIYQDAKRPSGIPERFSGSNSGNTATLTISGTQAMDEADYYCQAWDSSTGVFGGGTK LTVLAAAGSHHHHHH |
| 159 | Fab VH MHCcomplex (MHC-2) | EVQLVESGGGVVQPGRSLRLSCAASGFTFSSYAMHWVRQAPGKGLEWVAVISYDGSNKYYADSVKGRFTISRDNSKNTLY LQMNSLRTEDTAVYYCARDGGYYHYGLDVWGQGTTVTVSSASTKGPSVFPLAPSSKSTSGGTAALGCLVKDYFPEPVTVS WNSGALTSGVHTFPAVLQSSGLYSLSSVVVPSSSLGTQTYICNVNHKPSNTKVDKKV |
| 160 | Fab VL MHCcomplex (MHC-2) | SYELTQPPSVSVSPGQTASITCSGDKLGDKYASWYQRKPGQSPVLLIYQDAKRPSGIPERFSGSNSGNTATLTISGTQAM DEADYYCQAWDSSTGVFGGGTKLTVLGQPKAAPSVTLFPPSSEELQANKATLVCLISDFYPGAVTVAWKADSSPVKAGVE TTTPSKQSNNKYAASSYLSLTPEQWKSHRSYSCQVTHEGSTVEKTVAPTECS |
| 161 | TandAb_680 (EGFR-A1_T) | QVQLQESGPGLVKPSETLSLTCTVSGGSVSSGSYYWSWIRQPPGKGLEWIGYIYYSGSTNYNPSLKSRVTISVDTSKNQF SLKLSSVTAADTAVYYCARNPISTPAFDIWGQGTMVTVSSGGSGGSGGSSYVLTQPSSVSVAPGQTATISCGGHNIGSKN VHWYQQRPGQSPVLVIYQDNKRPSGIPERFSGSNSGNTATLTISGTQAMDEADYYCQVWDNYSVLFGGGTKLTVLGGSGG SQVQLVQSGAEVKKPGESLKVSCKASGYTFTSYYMHWVRQAPGQGLEWMGIINPSGGSTSYAQKFQGRVTMTRDTSTSTV YMELSSLRSEDTAVYYCARGSAYYYDFADYWGQGTLVTVSSGGSGGSGGSQPVLTQPPSVSVAPGKTARITCGGNNIGSK SVHWYQQKPGQAPVLVIYYDSDRPSGIPERFSGSNSGNTATLTISRVEAGDEADYYCQVWDTSSDHVLFGGGTKLTVLAA AGSHHHHHH |
| 162 | scFv-IgAb (EGFR-A1_I) Polpeptide chain 1: | QVQLVQSGAEVKKPGESLKVSCKASGYTFTSYYMHWVRQAPGQGLEWMGIINPSGGSTSYAQKFQGRVTMTRDTSTSTVY MELSSLRSEDTAVYYCARGSAYYYDFADYWGQGTLVTVSSASTKGPSVFPLAPSSKSTSGGTAALGCLVKDYFPEPVTVS WNSGALTSGVHTFPAVLQSSGLYSLSSVVTVPSSSLGTQTYICNVNHKPSNTKVDKKVEPKSCDKTHTCPPCPAPEFEGG PSVFLFPPKPKDTLMISRTPEVTCVVVAVSHEDPEVKFNWYVDGVEVHNAKTKPREEQYNSTYRVVSVLTVLHQDWLNGK EYKCKVSNKALPAPIEKTISKAKGQPREPQVYTLPPSREEMTKNQVSLTCLVKGFYPSDIAVEWESNGQPENNYKTTPPV LDSDGSFFLYSKLTVDKSRWQQGNVFSCSVMHEALHNHYTQKSLSLSPGGGGGSGGGGSQVQLQESGPGLVKPSETLSLT CTVSGGSVSSGSYYWSWIRQPPGKGLEWIGYIYYSGSTNYNPSLKSRVTISVDTSKNQFSLKLSSVTAADTAVYYCARNP ISIPAFDIWGQGTMVTVSSGGSGGSGGSGGSGGSGGSQPVLTQPPSVSVAPGKTARITCGGNNIGSKSVHWYQQKPGQAP VLVIYYDSDRPSGIPERFSGSNSGNTATLTISRVEAGDEADYYCQVWDTSSDHVLFGGGTKLTVL |
| 163 | scFv-IgAb (EGFR-A1_I) Polypeptide chain 2: | SYVLTQPSSVSVAPGQTATISCGGHNIGSKNVHWYQQRPGQSPVLVIYQDNKRPSGIPERFSGSNSGNTATLTISGTQAM DEADYYCQVWDNYSVLFGGGTKLTVLGQPKAAPSVTLFPPSSEELQANKATLVCLISDFYPGAVTVAWKADSSPVKAGVE TTTPSKQSNNKYAASSYLSLTPEQWKSHRSYSCQVTHEGSTVEKTVAPTECS |
| 164 | TandAb (BCMA-A1_T) | EVQLLESGGGLVQPGGSLRLSCAASGFTFSNYDMAWVRQAPGKGLEWVSSISTRGDITSYRDSVKGRFTISRDNSKNTLY LQMNSLRAEDTAVYYCARQDYYTDYMGFAYWGQGTLVTVSSGGSGGSGGSGGSSYVLTQPSSVSVAPGQTATISCGGHNI GSKNVHWYQQRPGQSPVLVIYQDNKRPSGIPERFSGSNSGNTATLTISGTQAMDEADYYCQVWDNYSVLFGGGTKLTVLG GSQVQLVQSGAEVKKPGESLKVSCKASGYTFTSYYMHWVRQAPGQGLEWMGAIEPMYGSTSYAQKFQGRVTMTRDTSTST VYMELSSLRSEDTAVYYCARGSAYYYDFADYWGQGTLVTVSSGGSGGSGGSGGSAIQMTQSPSSLSASVGDRVTITCRAS EDIYNGLAWYQQKPGKAPKLLIYGASSLQDGVPSRFSGSGSGTEFTLTISSLQPEDEATYYCAGPHKYPLTFGGGTKVEI K |
| 165 | scFv-IgAb (BCMA-A1_I) Polypeptide chain 1: | EVQLLESGGGLVQPGGSLRLSCAASGFTFSNYDMAWVRQAPGKGLEWVSSISTRGDITSYRDSVKGRFTISRDNSKNTLY LQMNSLRAEDTAVYYCARQDYYTDYMGFAYWGQGTLVTVSSASTKGPSVFPLAPSSKSTSGGTAALGCLVKDYFPEPVTV SWNSGALTSGVHTFPAVLQSSGLYSLSSVVTVPSSSLGTQTYICNVNHKPSNTKVDKKVEPKSCDKTHTCPPCPAPEFEG GPSVFLFPPKPKDTLMISRTPEVTCVVVAVSHEDPEVKFNWYVDGVEVHNAKTKPREEQYNSTYRVVSVLTVLHQDWLNG KEYKCKVSNKALPAPIEKTISKAKGQPREPQVYTLPPSREEMTKNQVSLTCLVKGFYPSDIAVEWESNGQPENNYKTTPP VLDSDGSFFLYSKLTVDKSRWQQGNVFSCSVMHEALHNHYTQKSLSLSPGGGGGSGGGGSGGGGSGGGGSGGGGSGGGGS SYVLTQPSSVSVAPGQTATISCGGHNIGSKNVHWYQQRPGQSPVLVIYQDNKRPSGIPERFSGSNSGNTATLTISGTQAM DEADYYCQVWDNYSVLFGGGTKLTVLGGSGGSGGSGGSGGSGGSGGSQVQLVQSGAEVKKPGESLKVSCKASGYTFTSYY MHWVRQAPGQGLEWMGAIEPMYGSTSYAQKFQGRVTMTRDTSTSTVYMELSSLRSEDTAVYYCARGSAYYYDFADYWGQG TLVTVSS |
| 166 | scFv-IgAb (BCMA-A1_I) Polypeptide chain 2: | AIQMTQSPSSLSASVGDRVTITCRASEDIYNGLAWYQQKPGKAPKLLIYGASSLQDGVPSRFSGSGSGTEFTLTISSLQP EDEATYYCAGPHKYPLTFGGGTKVEIKRTVAAPSVFIFPPSDEQLKSGTASVVCLLNNFYPREAKVQWKVDNALQSGNSQ ESVTEQDSKDSTYSLSSTLTLSKADYEKHKVYACEVTHQGLSSPVTKSFNRGEC |
| 167 | KiH-scDb-Fc (BCMA-A1_K) Polypeptide chain 1: | DKTHTCPPCPAPEFEGGPSVFLFPPKPKDTLMISRTPEVTCVVVAVSHEDPEVKFNWYVDGVEVHNAKTKPREEQYNSTY RVVSVLTVLHQDWLNGKEYKCKVSNKALPAPIEKTISKAKGQPREPQVYTLPPSREEMTKNQVSLTCLVKGFYPSDIAVE WESNGQPENNYKTTPPVLDSDGSFFLTSKLTVDKSRWQQGNVFSCSVMHEALHNHYTQKSLSLSPGGGGGSGGGGSSYVL TQPSSVSVAPGQTATISCGGHNIGSKNVHWYQQRPGQSPVLVIYQDNKRPSGIPERFSGSNSGNTATLTISGTQAMDEAD YYCQVWDNYSVLFGGGTKLTVLGGSGGSQVQLVQSGAEVKKPGESLKVSCKASGYTFTSYYMHWVRQAPGQGLEWMGAIE PMYGSTSYAQKFQGRVTMTRDTSTSTVYMELSSLRSEDTAVYYCARGSAYYYDFADYWGQGTLVTVSSGGSGGSGGSGGS GGSGGSSYVLTQPSSVSVAPGQTATISCGGHNIGSKNVHWYQQRPGQSPVLVIYQDNKRPSGIPERFSGSNSGNTATLTI SGTQAMDEADYYCQVWDNYSVLFGGGTKLTVLGGSGGSQVQLVQSGAEVKKPGESLKVSCKASGYTFTSYYMHWVRQAPG QGLEWMGAIEPMYGSTSYAQKFQGRVTMTRDTSTSTVYMELSSLRSEDTAVYYCARGSAYYYDFADYWGQGTLVTVSSAA AGSHHHHHH |
| 168 | KiH-scDb-Fc (BCMA-A1_K) Polypeptide chain 2: | EVQLLESGGGLVQPGGSLRLSCAASGFTFSNYDMAWVRQAPGKGLEWVSSISTRGDITSYRDSVKGRFTISRDNSKNTLY LQMNSLRAEDTAVYYCARQDYYTDYMGFAYWGQGTLVTVSSGGGGSGGGGSGGGGSAIQMTQSPSSLSASVGDRVTITCR ASEDIYNGLAWYQQKPGKAPKLLIYGASSLQDGVPSRFSGSGSGTEFTLTISSLQPEDEATYYCAGPHKYPLTFGGGTKV EIKDKTHTCPPCPAPEFEGGPSVFLFPPKPKDTLMISRTPEVTCVVVAVSHEDPEVKFNWYVDGVEVHNAKTKPREEQYN STYRVVSVLTVLHQDWLNGKEYKCKVSNKALPAPIEKTISKAKGQPREPQVYTLPPSREEMTKNQVSLYCLVKGFYPSDI AVEWESNGQPENNYKTTPPVLDSDGSFFLYSKLTVDKSRWQQGNVFSCSVMHEALHNHYTQKSLSLSPG |
| 169 | Db-Fc (CD16-2-BCMA-1) | SYVLTQPSSVSVAPGQTATISCGGHNIGSKNVHWYQQRPGQSPVLVIYQDNKRPSGIPERFSGSNSGNTATLTISGTQAM DEADYYCQVWDNYSVLFGGGTKLTVLGGSGGSQVQLVQSGAEVKKPGESLKVSCKASGYTFTSYYMHWVRQAPGQGLEWM GAIEPMYGSTSYAQKFQGRVTMTRDTSTSTVYMELSSLRSEDTAVYYCARGSAYYYDFADYWGQGTLVTVSSGGGGSGGG GSDKTHTCPPCPAPEFEGGPSVFLFPPKPKDTLMISRTPEVTCVVVAVSHEDPEVKFNWYVDGVEVHNAKTKPREEQYNS TYRVVSVLTVLHQDWLNGKEYKCKVSNKALPAPIEKTISKAKGQPREPQVYTLPPSREEMTKNQVSLTCLVKGFYPSDIA VEWESNGQPENNYKTTPPVLDSDGSFFLYSKLTVDKSRWQQGNVFSCSVMHEALHNHYTQKSLSLSPGGGGGSGGGGSEV QLLESGGGLVQPGGSLRLSCAASGFTFSNYDMAWVRQAPGKGLEWVSSISTRGDITSYRDSVKGRFTISRDNSKNTLYLQ MNSLRAEDTAVYYCARQDYYTDYMGFAYWGQGTLVTVSSGGGGSGGGGSGGGGSAIQMTQSPSSLSASVGDRVTITCRAS EDIYNGLAWYQQKPGKAPKLLIYGASSLQDGVPSRFSGSGSGTEFTLTISSLQPEDEATYYCAGPHKYPLTFGGGTKVEI K |
| 170 | scDb-mFc (CD16-2-BCMA-1) | EVQLLESGGGLVQPGGSLRLSCAASGFTFSNYDMAWVRQAPGKGLEWVSSISTRGDITSYRDSVKGRFTISRDNSKNTLY LQMNSLRAEDTAVYYCARQDYYTDYMGFAYWGQGTLVTVSSGGGGSGGGGSGGGGSAIQMTQSPSSLSASVGDRVTITCR ASEDIYNGLAWYQQKPGKAPKLLIYGASSLQDGVPSRFSGSGSGTEFTLTISSLQPEDEATYYCAGPHKYPLTFGGGTKV EIKGGGGSGGGGSAPEFEGGPSVFLFPPKPKDTLMISRTPEVTCVVVAVSHEDPEVKFNWYVDGVEVHNAKTKPREEQYN STYRVVSVLTVLHQDWLNGKEYKCKVSNKALPAPIEKTISKAKGQPREPQVYTKPPSRDELTKNQVSLSCLVKGFYPSDI AVEWESNGQPENNYKTTVPVLDSDGSFRLASYLTVDKSRWQQGNVFSCSVMHEALHNHYTQKSLSLSPGGGGSGGGGSSY VLTQPSSVSVAPGQTATISCGGHNIGSKNVHWYQQRPGQSPVLVIYQDNKRPSGIPERFSGSNSGNTATLTISGTQAMDE ADYYCQVWDNYSVLFGGGTKLTVLGGSGGSQVQLVQSGAEVKKPGESLKVSCKASGYTFTSYYMHWVRQAPGQGLEWMGA IEPMYGSTSYAQKFQGRVTMTRDTSTSTVYMELSSLRSEDTAVYYCARGSAYYYDFADYWGQGTLVTVSSGGSGGSGGSG GSGGSGGSSYVLTQPSSVSVAPGQTATISCGGHNIGSKNVHWYQQRPGQSPVLVIYQDNKRPSGIPERFSGSNSGNTATL TISGTQAMDEADYYCQVWDNYSVLFGGGTKLTVLGGSGGSQVQLVQSGAEVKKPGESLKVSCKASGYTFTSYYMHWVRQA PGQGLEWMGAIEPMYGSTSYAQKFQGRVTMTRDTSTSTVYMELSSLRSEDTAVYYCARGSAYYYDFADYWGQGTLVTVSS |
| 171 | Bi-scFv-Fc (CD16-1-EGFR-1) | SYVLTQPSSVSVAPGQTATISCGGHNIGSKNVHWYQQRPGQSPVLVIYQDNKR[?]SGIPERFSGSNSGNTATLTISGTQAM DEADYYCQVWDNYSVLFGGGTKLTVLGGSGGSGGSGGSGGSGGSGGSQVQLVQSGAEVKKPGESLKVSCKASGYTFTSYY MHWVRQAPGQGLEWMGIINPSGGSTSYAQKFQGRVTMTRDTSTSTVYMELSSLRSEDTAVYYCARGSAYYYDFADYWGQG TLVTVSSEPKSCDKTHTCPPCPAPEFEGGPSVFLFPPKPKDTLMISRTPEVTCVVVAVSHEDPEVKFNWYVDGVEVHNAK TKPREEQYNSTYRVVSVLTVLHQDWLNGKEYKCKVSNKALPAPIEKTISKAKGQPREPQVYTLPPSREEMTKNQVSLTCL VKGFYPSDIAVEWESNGQPENNYKTTPPVLDSDGSFFLYSKLTVDKSRWQQGNVFSCSVMHEALHNHYTQKSLSLSPGGG GGSGGGGSQVQLQESGPGLVKPSETLSLTCTVSGGSVSSGSYYWSWIRQPPGKGLEWIGYIYYSGSTNYNPSLKSRVTIS VDTSKNQFSLKLSSVTAADTAVYYCARNPISIPAFDIWGQGTMVTVSSGGSGGSGGSGGSGGSGGSQPVLTQPPSVSVAP GKTARITCGGNNIGSKSVHWYQQKPGQAPVLVIYYDSDRPSGIPERFSGSNSGNTATLTISRVEAGDEADYYCQVWDTSS DHVLFGGGTKLTVL |
| 172 | CD16A C-terminal sequence | SFFPPGYQ |
| 173 | scFv-IgAb_73 Polypeptide chain 1: | QVQLVQSGAEVKKPGSSVKVSCKASGGTFSSYTISWVRQAPGQGLEWMGGIIPIFGTANYAQKFQGRVTITADESTSTAY MELSSLRSEDTAVYYCARGGSSGWWWGQGTLVTVSSASTKGPSVFPLAPSSKSTSGGTAALGCLVKDYFPEPVTVSWNSG ALTSGVHTFPAVLQSSGLYSLSSVVTVPSSSLGTQTYICNVNHKPSNTKVDKKVEPKSCDKTHTCPPCPAPEFEGGPSVF LFPPKPKDTLMISRTPEVTCVVVAVSHEDPEVKFNWYVDGVEVHNAKTKPREEQYNSTYRVVSVLTVLHQDWLNGKEYKC KVSNKALPAPIEKTISKAKGQPREPQVYTLPPSREEMTKNQVSLTCLVKGFYPSDIAVEWESNGQPENNYKTTPPVLDSD GSFFLYSKLTVDKSRWQQGNVFSCSVMHEALHNHYTQKSLSLSPGGGGGSGGGGSGGGGSGGGGSGGGGSGGGGSDIQMT QSPASLSASVGETVTITCRVSENIYSYLAWYQQKQGKSPQLLVYNAKTLAEGVPSRFSGSGSGTQFSLKINSLQPEDFGS YYCQHHYGTPWTFGGGTKLEIKGGSGGSGGSGGSGGSGGSGGSEVQLQESGPGLVKPSQSLSLTCTVTGYSITSDYAWNW IRQFPGNKLEWMGYITYSGSTSYNPSLESRISITRDTSTNQFFLQLNSVTTEDTATYYCARGGYYGSSWGVFAYWGQGTL VTVSA |
| 174 | scFv-IgAb_73 Polypeptide chain 2: | QSVLTQPPSASGTPGQRVIISCSGSHSNIGSNNVNWYQQLPGTAPKLLIYNNNQRPSGVPDRFSGSKSGTSASLAISGLQ SEDESDYFCGTWDDSLNGPVFGGGTKLTVLGQPKAAPSVTLFPPSSEELQANKATLVCLISDFYPGAVTVAWKADSSPVK AGVETTTPSKQSNNKYAASSYLSLTPEQWKSHRSYSCQVTHEGSTVEKTVAPTECS |
| 175 | scFv-IgAb_75 Polypeptide chain 1: | QVQLVQSGAEVKKPGSSVKVSCKASGGTFSSYTISWVRQAPGQGLEWMGGIIPIFGTANYAQKFQGRVTITADESTSTAY MELSSLRSEDTAVYYCARGGSSGWWWGQGTLVTVSSASTKGPSVFPLAPSSKSTSGGTAALGCLVKDYFPEPVTVSWNSG ALTSGVHTFPAVLQSSGLYSLSSVVTVPSSSLGTQTYICNVNHKPSNTKVDKKVEPKSCDKTHTCPPCPAPEFEGGPSVF LFPPKPKDTLMISRTPEVTCVVVAVSHEDPEVKFNWYVDGVEVHNAKTKPREEQYNSTYRVVSVLTVLHQDWLNGKEYKC KVSNKALPAPIEKTISKAKGQPREPQVYTLPPSREEMTKNQVSLTCLVKGFYPSDIAVEWESNGQPENNYKTTPPVLDSD GSFFLYSKLTVDKSRWQQGNVFSCSVMHEALHNHYTQKSLSLSPGGGGGSGGGGSGGGGSGGGGSGGGGSGGGGSQSALT QPASVSGSPGQSITISCSGSSSNIGNNAVNWYQQLPGKAPKLLIYYDDLLPSGVSDRFSGSKSGTSAFLAISGLQSEDEA DYYCAAWDDSLNGPVFGGGTKLTVLGGSGGSGGSGGSGGSGGSGGSQVQLVESGGGLVKPGGSLRLSCAASGFTFSSYGM HWVRQAPGKGLEWVAFIRYDGSNKYYADSVKGRFTISRDNSKNTLYLQMNSLRAEDTAVYYCAKDRGLGDGTYFDYWGQG TTVTVSS |
| 176 | scFv-IgAb_75 Polypeptide chain 2: | QSVLTQPPSASGTPGQRVIISCSGSHSNIGSNNVNWYQQLPGTAPKLLIYNNNQRPSGVPDRFSGSKSGTSASLAISGLQ SEDESDYFCGTWDDSLNGPVFGGGTKLTVLGQPKAAPSVTLFPPSSEELQANKATLVCLISDFYPGAVTVAWKADSSPVK AGVETTTPSKQSNNKYAASSYLSLTPEQWKSHRSYSCQVTHEGSTVEKTVAPTECS |
| 177 | scFv-IgAb_80 Polypeptide chain 1: | QVQLVQSGAEVKKPGSSVKVSCKASGGTFSSYTISWVRQAPGQGLEWMGGIIPIFGTANYAQKFQGRVTITADESTSTAY MELSSLRSEDTAVYYCARGGSSGWWWGQGTLVTVSSASTKGPSVFPLAPSSKSTSGGTAALGCLVKDYFPEPVTVSWNSG ALTSGVHTFPAVLQSSGLYSLSSVVTVPSSSLGTQTYICNVNHKPSNTKVDKKVEPKSCDKTHTCPPCPAPEFEGGPSVF LFPPKPKDTLMISRTPEVTCVVVAVSHEDPEVKFNWYVDGVEVHNAKTKPREEQYNSTYRVVSVLTVLHQDWLNGKEYKC KVSNKALPAPIEKTISKAKGQPREPQVYTLPPSREEMTKNQVSLTCLVKGFYPSDIAVEWESNGQPENNYKTTPPVLDSD GSFFLYSKLTVDKSRWQQGNVFSCSVMHEALHNHYTQKSLSLSPGGGGGSGGGGSGGGGSGGGGSGGGGSGGGGSSYVLT QPSSVSVAPGQTATISCGGHNIGSKNVHWYQQRPGQSPVLVIYQDNKRPSGIPERFSGSNSGNTATLTISGTQAMDEADY YCQVWDNYSVLFGGGTKLTVLGGSGGSGGSGGSGGSGGSGGSQVQLVQSGAEVKKPGESLKVSCKASGYTFTSYYMHWVR QAPGQGLEWMGAIEPMYGSTSYAQKFQGRVTMTRDTSTSTVYMELSSLRSEDTAVYYCARGSAYYYDFADYWGQGTLVTV SS |
| 178 | scFv-IgAb_80 Polypeptide chain 2: | QSVLTQPPSASGTPGQRVIISCSGSHSNIGSNNVNWYQQLPGTAPKLLIYNNNQRPSGVPDRFSGSKSGTSASLAISGLQ SEDESDYFCGTWDDSLNGPVFGGGTKLTVLGQPKAAPSVTLFPPSSEELQANKATLVCLISDFYPGAVTVAWKADSSPVK AGVETTTPSKQSNNKYAASSYLSLTPEQWKSHRSYSCQVTHEGSTVEKTVAPTECS |

## Claims

1. Isolated human NK cells in a cryopreserved state, preloaded prior to freezing with an antibody construct, the antibody construct comprising at least a first binding domain binding to an NK cell receptor antigen on the cell surface of an immunological effector cell, said first binding domain comprises three heavy chain CDRs and three light chain CDRs, and a second binding domain binding to a cell surface antigen on the cell surface of a target cell.

2. The isolated human NK cells according to claim 1, wherein the NK cells are isolated from umbilical cord or placenta tissue, iPSC or PBMC from healthy donors.

3. The isolated human NK cells according to claim 1 or 2, wherein the NK cells have been conserved in cryo solution.

4. The isolated human NK cells according to anyone of claims 1 to 3, wherein the NK cells have been preloaded in a solution comprising the antibody construct in a concentration of at least 5nM.

5. The isolated human NK cells according to anyone of claims 1 to 4, wherein the NK cell receptor antigen to which the first binding domain of the antibody construct binds to is selected from the group consisting of CD16a, CD16b, NKp46, NKG2D and CD16a + CD16b.

6. The isolated human NK cells according to anyone of claims 1 to 5, wherein the cell surface antigen on the cell surface of a target cell to which the second binding domain of the antibody construct binds to is selected from the group consisting of CD19, CD20, CD22, CD30, CD33, CD52, CD70, CD74, CD79b, CD123, CLL1, BCMA, FCRH5, EGFR, EGFRvIII, HER2, and GD2.

7. The isolated human NK cells according to anyone of claims 1 to 6, wherein the antibody construct comprises a first binding domain binding to CD16a and a second binding domain binding to an antigen selected from the group consisting of CD19, CD20, CD22, CD30, CD33, CD52, CD70, CD74, CD79b, CD123, CLL1, BCMA, FCRH5, EGFR, EGFRvIII HER2, and GD2.

8. The isolated human NK cells according to claim 7, wherein the antibody construct comprises in the first binding domain three heavy chain CDRs and three light chain CDRs selected from the group consisting of:
(a) a CDR-H1 as depicted in SEQ ID NO: 29, a CDR-H2 as depicted in SEQ ID NO: 30, a CDR-H3 as depicted in SEQ ID NO: 31, a CDR-L1 as depicted in SEQ ID NO: 32, a CDR-L2 as depicted in SEQ ID NO: 33, a CDR-L3 as depicted in SEQ ID NO: 34;
(b) a CDR-H1 as depicted in SEQ ID NO: 40, a CDR-H2 as depicted in SEQ ID NO: 41, a CDR-H3 as depicted in SEQ ID NO: 42, a CDR-L1 as depicted in SEQ ID NO: 43, a CDR-L2 as depicted in SEQ ID NO: 44, a CDR-L3 as depicted in SEQ ID NO: 45;
(c) a CDR-H1 as depicted in SEQ ID NO: 51, a CDR-H2 as depicted in SEQ ID NO: 52, a CDR-H3 as depicted in SEQ ID NO: 53, a CDR-L1 as depicted in SEQ ID NO: 54, a CDR-L2 as depicted in SEQ ID NO: 55, a CDR-L3 as depicted in SEQ ID NO: 56;
(d) a CDR-H1 as depicted in SEQ ID NO: 62, a CDR-H2 as depicted in SEQ ID NO: 63, a CDR-H3 as depicted in SEQ ID NO: 64, a CDR-L1 as depicted in SEQ ID NO: 65, a CDR-L2 as depicted in SEQ ID NO: 66, a CDR-L3 as depicted in SEQ ID NO: 67;
(e) a CDR-H1 as depicted in SEQ ID NO: 73, a CDR-H2 as depicted in SEQ ID NO: 74, a CDR-H3 as depicted in SEQ ID NO: 75, a CDR-L1 as depicted in SEQ ID NO: 76, a CDR-L2 as depicted in SEQ ID NO: 77, a CDR-L3 as depicted in SEQ ID NO: 78;
(f) a CDR-H1 as depicted in SEQ ID NO: 84, a CDR-H2 as depicted in SEQ ID NO: 85, a CDR-H3 as depicted in SEQ ID NO: 86, a CDR-L1 as depicted in SEQ ID NO: 87, a CDR-L2 as depicted in SEQ ID NO: 88, a CDR-L3 as depicted in SEQ ID NO: 89; and
(g) a CDR-H1 as depicted in SEQ ID NO: 95, a CDR-H2 as depicted in SEQ ID NO: 96, a CDR-H3 as depicted in SEQ ID NO: 97, a CDR-L1 as depicted in SEQ ID NO: 98, a CDR-L2 as depicted in SEQ ID NO: 99, a CDR-L3 as depicted in SEQ ID NO: 100.

9. The isolated human NK cells according to claim 8, wherein the antibody construct comprises in the first binding domain pairs of VH- and VL-chains having a sequence as depicted in the pairs of sequences selected from the group consisting of SEQ ID NO: 35 and SEQ ID NO: 36, SEQ ID NO: 46 and SEQ ID NO: 47, SEQ ID NO: 57 and SEQ ID NO: 58, SEQ ID NO: 68 and SEQ ID NO: 69, SEQ ID NO: 79 and SEQ ID NO: 80, SEQ ID NO: 90 and SEQ ID NO: 91,and SEQ ID NO: 101 and SEQ ID NO: 102.

10. The isolated human NK cells according to anyone of claims 7 to 9, wherein the antibody construct comprises in the second binding domain three heavy chain CDRs and three light chain CDRs selected from the group consisting of:
(a) a CDR-H1 as depicted in SEQ ID NO: 106, a CDR-H2 as depicted in SEQ ID NO: 107, a CDR-H3 as depicted in SEQ ID NO: 108, a CDR-L1 as depicted in SEQ ID NO: 109, a CDR-L2 as depicted in SEQ ID NO: 110, a CDR-L3 as depicted in SEQ ID NO: 111;
(b) a CDR-H1 as depicted in SEQ ID NO: 128, a CDR-H2 as depicted in SEQ ID NO: 129, a CDR-H3 as depicted in SEQ ID NO: 130, a CDR-L1 as depicted in SEQ ID NO: 131, a CDR-L2 as depicted in SEQ ID NO: 132, a CDR-L3 as depicted in SEQ ID NO: 133; and
(c) a CDR-H1 as depicted in SEQ ID NO: 117, a CDR-H2 as depicted in SEQ ID NO: 118, a CDR-H3 as depicted in SEQ ID NO: 119, a CDR-L1 as depicted in SEQ ID NO: 120, a CDR-L2 as depicted in SEQ ID NO: 121, a CDR-L3 as depicted in SEQ ID NO: 122.

11. The isolated human NK cells according to anyone of claims 7 to 10, wherein the antibody construct comprises in the second binding domain pairs of VH- and VL-chains having a sequence as depicted in the pairs of sequences selected from the group consisting of SEQ ID NO: 112 and SEQ ID NO: 113, SEQ ID NO: 123 and SEQ ID NO: 124, and SEQ ID NO: 134 and SEQ ID NO: 135.

12. The isolated human NK cells according to anyone of claims 7 to 11, wherein the antibody construct comprises a protein sequence as depicted in SEQ ID NOs: 161-171.

13. A method for preparation of cryopreserved preloaded human NK cells, the method comprising
(i) incubating NK cells with an antibody construct, the antibody construct comprising at least a first binding domain binding to an NK cell receptor antigen on the cell surface of an immunological effector cell, said first binding domain comprises three heavy chain CDRs and three light chain CDRs, and a second binding domain binding to a cell surface antigen on the cell surface of a target cell; and
(ii) freezing the NK cells.

14. The method of claims 13, wherein the NK cells are isolated from umbilical cord tissue, iPSC or PBMC from healthy donors.

15. The method of claims 13 and 14, wherein the NK cells have been preloaded in a solution comprising the antibody construct in a concentration of at least 5nM.

16. The method according to anyone of claims 13 to 15, wherein the NK cell receptor antigen to which the first binding domain of the antibody construct binds to is selected from the group consisting of CD16a, CD16b, NKp46, NKG2D and CD16a + CD16b.

17. The method according to anyone of claims 13 to 16, wherein the cell surface antigen on the cell surface of a target cell to which the second binding domain of the antibody construct binds to is selected from the group consisting of CD19, CD22, CD30, CD33, CD52, CD70, CD74, CD79b, CD123, CLL1, BCMA, FCRH5, EGFR, HER2, GD2.

18. The method according to anyone of claims 13 to 17, wherein the step of freezing the NK cells is performed using a freezing medium which contains at least a basal cell culture medium and cryoprotective agent.

19. The method according to anyone of claims 13 to 18, wherein the antibody construct comprises a protein sequence as depicted in SEQ ID NOs: 161-171.

20. A method for reconstituting/preparing viable preloaded human NK cells for the administration to a subject in the need thereof, the method comprising the step of
(a) thawing cryopreserved NK cells according to anyone of claims 1 to 12; or
(b) thawing cryopreserved NK cells prepared by a method according to anyone of claims 13 to 19.

## Patentansprüche

1. Isolierte, humane NK Zellen in einem kryokonservierten Zustand, vorgeladen mit einem Antikörperkonstrukt vor dem Einfrieren, das Antikörperkonstrukt umfassend zumindest eine erste Bindungsdomäne, die an ein NK Zellrezeptor Antigen auf der Zelloberfläche einer immunologischen Effektorzelle bindet, diese erste Bindungsdomäne umfasst drei schwere Kette CDRs und drei leichte Kette CDRs, und eine zweite Bindungsdomäne, die an ein Zelloberflächen-Antigen auf der Zelloberfläche einer Zielzelle bindet.

2. Isolierte, humane NK Zellen gemäß Anspruch 1, wobei die NK Zellen isoliert sind aus Nabelschnur- oder Plazentagewebe, iPSC oder PBMC von gesunden Spendern.

3. Isolierte, humane NK Zellen gemäß Anspruch 1 oder 2, wobei die NK Zellen in Kryolösung konserviert worden sind.

4. Isolierte, humane NK Zellen gemäß irgendeinem der Ansprüche 1 bis 3, wobei die NK Zellen vorgeladen worden sind in einer Lösung umfassend das Antikörperkonstrukt in einer Konzentration von zumindest 5nM.

5. Isolierte, humane NK Zellen gemäß irgendeinem der Ansprüche 1 bis 4, wobei das NK Zellrezeptor Antigen an das die erste Bindungsdomäne des Antikörperkonstrukts bindet, ausgewählt ist aus der Gruppe bestehend aus CD16a, CD16b, NKp46, NKGG2D und CD16a+CD16b.

6. Isolierte, humane NK Zellen gemäß irgendeinem der Ansprüche 1 bis 5, wobei das Zelloberflächen-Antigen auf der Zelloberfläche der Zielzelle an das die zweite Bindungsdomäne des Antikörperkonstrukts bindet, ausgewählt ist aus der Gruppe bestehend aus CD19, CD20, CD22, CD30, CD33, CD52, CD70, CD74, CD79b, CD123, CLL1, BCMA, FCRH5, EGFR, EGFRvIII, HER2 und GD2.

7. Isolierte, humane NK Zellen gemäß irgendeinem der Ansprüche 1 bis 6, wobei das Antikörperkonstrukt umfasst eine erste Bindungsdomäne, die an CD16a bindet, und eine zweite Bindungsdomäne, die an ein Antigen bindet, das ausgewählt ist aus der Gruppe bestehend aus CD19, CD20, CD22, CD30, CD33, CD52, CD70, CD74, CD79b, CD123, CLL1, BCMA, FCRH5, EGFR, EGFRvlll, HER2 und GD2.

8. Isolierte, humane NK Zellen gemäß Anspruch 7, wobei das Antikörperkonstrukt in der ersten Bindungsdomäne drei schwere Kette CDRs und drei leichte Kette CDRs umfasst, die ausgewählt sind aus der Gruppe bestehend aus:
(a) einer CDR-H1 dargestellt in SEQ ID NO: 29, einer CDR-H2 dargestellt in SEQ ID NO: 30, einer CDR-H3 dargestellt in SEQ ID NO: 31, einer CDR-L1 dargestellt in SEQ ID NO: 32, einer CDR-L2 dargestellt in SEQ ID NO: 33, einer CDR-L3 dargestellt in SEQ ID NO: 34;
(b) einer CDR-H1 dargestellt in SEQ ID NO: 40, einer CDR-H2 dargestellt in SEQ ID NO: 41, einer CDR-H3 dargestellt in SEQ ID NO: 42, einer CDR-L1 dargestellt in SEQ ID NO: 43, einer CDR-L2 dargestellt in SEQ ID NO: 44, einer CDR-L3 dargestellt in SEQ ID NO: 45;
(c) einer CDR-H1 dargestellt in SEQ ID NO: 51, einer CDR-H2 dargestellt in SEQ ID NO: 52, einer CDR-H3 dargestellt in SEQ ID NO: 53, einer CDR-L1 dargestellt in SEQ ID NO: 54, einer CDR-L2 dargestellt in SEQ ID NO: 55, einer CDR-L3 dargestellt in SEQ ID NO: 56;
(d) einer CDR-H1 dargestellt in SEQ ID NO: 62, einer CDR-H2 dargestellt in SEQ ID NO: 63, einer CDR-H3 dargestellt in SEQ ID NO: 64, einer CDR-L1 dargestellt in SEQ ID NO: 65, einer CDR-L2 dargestellt in SEQ ID NO: 66, einer CDR-L3 dargestellt in SEQ ID NO: 67;
(e) einer CDR-H1 dargestellt in SEQ ID NO: 73, einer CDR-H2 dargestellt in SEQ ID NO: 74, einer CDR-H3 dargestellt in SEQ ID NO: 75, einer CDR-L1 dargestellt in SEQ ID NO: 76, einer CDR-L2 dargestellt in SEQ ID NO: 77, einer CDR-L3 dargestellt in SEQ ID NO: 78;
(f) einer CDR-H1 dargestellt in SEQ ID NO: 84, einer CDR-H2 dargestellt in SEQ ID NO: 85, einer CDR-H3 dargestellt in SEQ ID NO: 86, einer CDR-L1 dargestellt in SEQ ID NO: 87, einer CDR-L2 dargestellt in SEQ ID NO: 88, einer CDR-L3 dargestellt in SEQ ID NO: 89; and
(g) einer CDR-H1 dargestellt in SEQ ID NO: 95, a CDR-H2 dargestellt in SEQ ID NO: 96, einer CDR-H3 dargestellt in SEQ ID NO: 97, einer CDR-L1 dargestellt in SEQ ID NO: 98, einer CDR-L2 dargestellt in SEQ ID NO: 99, einer CDR-L3 dargestellt in SEQ ID NO: 100.

9. Isolierte, humane NK Zellen gemäß Anspruch 8, wobei das Antikörperkonstrukt in der ersten Bindungsdomäne VH- und VL-Kettenpaare umfasst, die eine Sequenz haben dargestellt in den Sequenzpaaren ausgewählt aus der Gruppe bestehend aus SEQ ID NO: 35 und SEQ ID NO: 36, SEQ ID NO: 46 und SEQ ID NO: 47, SEQ ID NO: 57 und SEQ ID NO: 58, SEQ ID NO: 68 und SEQ ID NO: 69, SEQ ID NO: 79 und SEQ ID NO: 80, SEQ ID NO: 90 und SEQ ID NO: 91,und SEQ ID NO: 101 und SEQ ID NO: 102.

10. Isolierte, humane NK Zellen gemäß irgendeinem Anspruch der Ansprüche 7 bis 9, wobei das Antikörperkonstrukt in der zweiten Bindungsdomäne drei schwere Kette CDRs und drei leichte Kette CDRs umfasst, ausgewählt aus der Gruppe bestehend aus:
(a) einer CDR-H1 dargestellt in SEQ ID NO: 106, einer CDR-H2 dargestellt in SEQ ID NO: 107, einer CDR-H3 dargestellt in SEQ ID NO: 108, einer CDR-L1 dargestellt in SEQ ID NO: 109, einer CDR-L2 dargestellt in SEQ ID NO: 110, einer CDR-L3 dargestellt in SEQ ID NO: 111;
(b) einer CDR-H1 dargestellt in SEQ ID NO: 128, einer CDR-H2 dargestellt in SEQ ID NO: 129, einer CDR-H3 dargestellt in SEQ ID NO: 130, einer CDR-L1 dargestellt in SEQ ID NO: 131, einer CDR-L2 dargestellt in SEQ ID NO: 132, einer CDR-L3 dargestellt in SEQ ID NO: 133; und
(c) einer CDR-H1 dargestellt in SEQ ID NO: 117, einer CDR-H2 dargestellt in SEQ ID NO: 118, einer CDR-H3 dargestellt in SEQ ID NO: 119, einer CDR-L1 dargestellt in SEQ ID NO: 120, einer CDR-L2 dargestellt in SEQ ID NO: 121, einer CDR-L3 dargestellt in SEQ ID NO: 122.

11. Isolierte, humane NK Zellen gemäß irgendeinem der Ansprüche 7 bis 10, wobei das Antikörperkonstrukt in der zweiten Bindungsdomäne VH- und VL-Kettenpaare umfasst, die eine Sequenz haben, dargestellt in den Sequenzpaaren ausgewählt aus der Gruppe bestehend aus of SEQ ID NO: 112 und SEQ ID NO: 113, SEQ ID NO: 123 und SEQ ID NO: 124, und SEQ ID NO: 134 und SEQ ID NO: 135.

12. Isolierte, humane NK Zellen gemäß irgendeinem der Ansprüche 7 bis 11, wobei das Antikörperkonstrukt eine in SEQ ID NOs: 161-171 dargestellte Proteinsequenz umfasst.

13. Verfahren zur Herstellung von kryokonservierten, vorgeladenen, humanen NK Zellen, wobei das Verfahren umfasst
(i) Inkubieren der NK Zellen mit einem Antikörperkonstrukt, das Antikörperkonstrukt umfasst zumindest eine erste Bindungsdomäne, die an ein NK Zellrezeptorantigen auf der Zelloberfläche einer immunologischen Effektorzelle bindet, diese erste Bindungsdomäne umfasst drei schwere Kette CDRs und drei leichte Kette CDRs, und eine zweite Bindungsdomäne, die an ein Zelloberflächen-Antigen auf der Zelloberfläche einer Zielzelle bindet; und
(ii) Einfrieren der NK Zellen.

14. Verfahren der Ansprüche 13, wobei die NK Zellen isoliert sind aus Nabelschnurgewebe, iPSC oder PBMC von gesunden Spendern.

15. Verfahren des Anspruchs 13 oder 14, wobei die NK Zellen vorgeladen wurden in einer Lösung umfassend das Antikörperkonstrukt in einer Konzentration von zumindest 5nM.

16. Verfahren gemäß irgendeinem der Ansprüche 13 bis 15, wobei das NK Zellrezeptor Antigen an das die erste Bindungsdomäne des Antikörperkonstrukts bindet, ausgewählt ist aus der Gruppe bestehend aus CD16a, CD16b, NKp46, NKGG2D und CD16a+CD16b.

17. Verfahren gemäß irgendeinem der Ansprüche 13 bis 16, wobei das Zelloberflächen-Antigen auf der Zelloberfläche der Zielzelle an das die zweite Bindungsdomäne des Antikörperkonstrukts bindet, ausgewählt ist aus der Gruppe bestehend aus CD19, CD22, CD30, CD33, CD52, CD70, CD74, CD79b, CD123, CLL1, BCMA, FCRH5, EGFR, EGFRvlll, HER2, GD2.

18. Verfahren gemäß irgendeinem der Ansprüche 13 bis 17, wobei der Schritt des Einfrierens der NK Zellen mit Verwendung eines Gefriermediums durchgeführt wird, das zumindest ein Basalmedium für eine Zellkultur und Gefrierschutzmittel enthält.

19. Verfahren gemäß irgendeinem der Ansprüche 13 bis 18, wobei das Antikörperkonstrukt eine in SEQ ID NOs: 161-171 dargestellte Proteinsequenz umfasst.

20. Verfahren zum Rekonstituieren/Herstellen lebensfähiger, vorgeladener, humaner NK Zellen zur Verabreichung an ein bedürftiges Subjekt, das Verfahren umfasst den Schritt des
(a) Auftauens kryokonservierte NK Zellen gemäß irgendeinem der Ansprüche 1 bis 12; oder
(b) Auftauens kryokonservierter NK Zellen hergestellt durch ein Verfahren gemäß irgendeinem der Ansprüche 13 bis 19.

## Revendications

1. Cellules NK humaines isolées dans un état cryoconservé, préchargées avant congélation avec une construction d'anticorps, la construction d'anticorps comprenant au moins un premier domaine de liaison se liant à un antigène récepteur de cellules NK sur la surface cellulaire d'une cellule effectrice immunologique, ledit premier domaine de liaison comprenant trois CDR de chaîne lourde et trois CDR de chaîne légère, et un second domaine de liaison se liant à un antigène de surface cellulaire sur la surface cellulaire d'une cellule cible.

2. Cellules NK humaines isolées selon la revendication 1, dans lesquelles les cellules NK sont isolées à partir d'un cordon ombilical ou d'un tissu placentaire, iPSC ou PBMC, provenant de donneurs sains.

3. Cellules NK humaines isolées selon la revendication 1 ou 2, dans lesquelles les cellules NK ont été conservées en solution cryogénique.

4. Cellules NK humaines isolées selon l'une quelconque des revendications 1 à 3, dans lesquelles les cellules NK ont été préchargées dans une solution comprenant la construction d'anticorps en une concentration d'au moins 5 nM.

5. Cellules NK humaines isolées selon l'une quelconque des revendications 1 à 4, dans lesquelles l'antigène récepteur de cellules NK auquel le premier domaine de liaison de la construction d'anticorps se lie à est choisi dans le groupe constitué de CD16a, CD16b, NKp46, NKG2D et CD16a + CD16b.

6. Cellules NK humaines isolées selon l'une quelconque des revendications 1 à 5, dans lesquelles l'antigène de surface cellulaire sur la surface cellulaire d'une cellule cible à laquelle le second domaine de liaison de la construction d'anticorps se lie à est choisi dans le groupe constitué de CD19, CD20, CD22, CD30, CD33, CD52, CD70, CD74, CD79b, CD123, CLL1, BCMA, FCRH5, EGFR, EGFRvIII, HER2 et GD2.

7. Cellules NK humaines isolées selon l'une quelconque des revendications 1 à 6, dans lesquelles la construction d'anticorps comprend un premier domaine de liaison se liant à CD16a et un second domaine de liaison se liant à un antigène choisi dans le groupe constitué de CD19, CD20, CD22, CD30, CD33, CD52, CD70, CD74, CD79b, CD123, CLL1, BCMA, FCRH5, EGFR, EGFRvIII HER2 et GD2.

8. Cellules NK humaines isolées selon la revendication 7, dans lesquelles la construction d'anticorps comprend dans le premier domaine de liaison trois CDR de chaîne lourde et trois CDR de chaîne légère choisis dans le groupe constitué :
(a) d'un CDR-H1 tel que représenté dans SEQ ID NO : 29, d'un CDR-H2 tel que représenté dans SEQ ID NO : 30, d'un CDR-H3 tel que représenté dans SEQ ID NO : 31, d'un CDR-L1 tel que représenté dans SEQ ID NO : 32, d'un CDR-L2 tel que représenté dans SEQ ID NO : 33, d'un CDR-L3 tel que représenté dans SEQ ID NO : 34 ;
(b) d'un CDR-H1 tel que représenté dans SEQ ID NO : 40, d'un CDR-H2 tel que représenté dans SEQ ID NO : 41, d'un CDR-H3 tel que représenté dans SEQ ID NO : 42, d'un CDR-L1 tel que représenté dans SEQ ID NO : 43, d'un CDR-L2 tel que représenté dans SEQ ID NO : 44, d'un CDR-L3 tel que représenté dans SEQ ID NO : 45 ;
(c) d'un CDR-H1 tel que représenté dans SEQ ID NO : 51, d'un CDR-H2 tel que représenté dans SEQ ID NO : 52, d'un CDR-H3 tel que représenté dans SEQ ID NO : 53, d'un CDR-L1 tel que représenté dans SEQ ID NO : 54, d'un CDR-L2 tel que représenté dans SEQ ID NO : 55, d'un CDR-L3 tel que représenté dans SEQ ID NO : 56 ;
(d) d'un CDR-H1 tel que représenté dans SEQ ID NO : 62, d'un CDR-H2 tel que représenté dans SEQ ID NO : 63, d'un CDR-H3 tel que représenté dans SEQ ID NO : 64, d'un CDR-L1 tel que représenté dans SEQ ID NO : 65, d'un CDR-L2 tel que représenté dans SEQ ID NO : 66, d'un CDR-L3 tel que représenté dans SEQ ID NO : 67 ;
(e) d'un CDR-H1 tel que représenté dans SEQ ID NO : 73, d'un CDR-H2 tel que représenté dans SEQ ID NO : 74, d'un CDR-H3 tel que représenté dans SEQ ID NO : 75, d'un CDR-L1 tel que représenté dans SEQ ID NO : 76, d'un CDR-L2 tel que représenté dans SEQ ID NO : 77, d'un CDR-L3 tel que représenté dans SEQ ID NO : 78 ;
(f) d'un CDR-H1 tel que représenté dans SEQ ID NO : 84, d'un CDR-H2 tel que représenté dans SEQ ID NO : 85, d'un CDR-H3 tel que représenté dans SEQ ID NO : 86, d'un CDR-L1 tel que représenté dans SEQ ID NO : 87, d'un CDR-L2 tel que représenté dans SEQ ID NO : 88, d'un CDR-L3 tel que représenté dans SEQ ID NO : 89 ; et
(g) d'un CDR-H1 tel que représenté dans SEQ ID NO : 95, d'un CDR-H2 tel que représenté dans SEQ ID NO : 96, d'un CDR-H3 tel que représenté dans SEQ ID NO : 97, d'un CDR-L1 tel que représenté dans SEQ ID NO : 98, d'un CDR-L2 tel que représenté dans SEQ ID NO : 99, d'un CDR-L3 tel que représenté dans SEQ ID NO : 100 ;

9. Cellules NK humaines isolées selon la revendication 8, dans lesquelles la construction d'anticorps comprend dans les premières paires de domaine de liaison de chaînes VH- et VL- ayant une séquence telle que représentée dans les paires de séquences choisies dans le groupe constitué de SEQ ID NO : 35 et SEQ ID NO : 36, SEQ ID NO : 46 et SEQ ID NO : 47, SEQ ID NO : 57 et SEQ ID NO : 58, SEQ ID NO : 68 et SEQ ID NO : 69, SEQ ID NO : 79 et SEQ ID NO : 80, SEQ ID NO : 90 et SEQ ID NO : 91, et SEQ ID NO : 101 et SEQ ID NO : 102.

10. Cellules NK humaines isolées selon l'une quelconque des revendications 7 à 9, dans lesquelles la construction d'anticorps comprend dans le second domaine de liaison trois CDR de chaîne lourde et trois CDR de chaîne légère choisis dans le groupe constitué :
(a) d'un CDR-H1 tel que représenté dans SEQ ID NO : 106, d'un CDR-H2 tel que représenté dans SEQ ID NO : 107, d'un CDR-H3 tel que représenté dans SEQ ID NO : 108, d'un CDR-L1 tel que représenté dans SEQ ID NO : 109, d'un CDR-L2 tel que représenté dans SEQ ID NO : 110, d'un CDR-L3 tel que représenté dans SEQ ID NO : 111 ;
(b) d'un CDR-H1 tel que représenté dans SEQ ID NO : 128, d'un CDR-H2 tel que représenté dans SEQ ID NO : 129, d'un CDR-H3 tel que représenté dans SEQ ID NO : 130, d'un CDR-L1 tel que représenté dans SEQ ID NO : 131, d'un CDR-L2 tel que représenté dans SEQ ID NO : 132, d'un CDR-L3 tel que représenté dans SEQ ID NO : 133 ; et
(c) d'un CDR-H1 tel que représenté dans SEQ ID NO : 117, d'un CDR-H2 tel que représenté dans SEQ ID NO : 118, d'un CDR-H3 tel que représenté dans SEQ ID NO : 119, d'un CDR-L1 tel que représenté dans SEQ ID NO : 120, d'un CDR-L2 tel que représenté dans SEQ ID NO : 121, d'un CDR-L3 tel que représenté dans SEQ ID NO : 122.

11. Cellules NK humaines isolées selon l'une quelconque des revendications 7 à 10, dans lesquelles la construction d'anticorps comprend dans les secondes paires de domaines de liaison des chaînes VH- et VL- ayant une séquence telle que représentée dans les paires de séquences choisies dans le groupe constitué de SEQ ID NO : 112 et de SEQ ID NO : 113, de SEQ ID NO : 123 et de SEQ ID NO : 124, et de SEQ ID NO : 134 et de SEQ ID NO : 135.

12. Cellules NK humaines isolées selon l'une quelconque des revendications 7 à 11, dans lesquelles la construction d'anticorps comprend une séquence protéique telle que représentée dans les SEQ ID NO : 161-171.

13. Procédé de préparation de cellules NK humaines préchargées cryoconservées, le procédé comprenant
(i) l'incubation des cellules NK avec une construction d'anticorps, la construction d'anticorps comprenant au moins un premier domaine de liaison se liant à un antigène récepteur de cellules NK sur la surface cellulaire d'une cellule effectrice immunologique, ledit premier domaine de liaison comprenant trois CDR de chaîne lourde et trois CDR de chaîne légère, et un second domaine de liaison se liant à un antigène de surface cellulaire sur la surface cellulaire d'une cellule cible ; et
(ii) la congélation des cellules NK.

14. Procédé selon la revendication 13, dans lequel les cellules NK sont isolées à partir d'un tissu de cordon ombilical, d'iPSC ou de PBMC, provenant de donneurs sains.

15. Procédé selon la revendication 13 et 14, dans lequel les cellules NK ont été préchargées dans une solution comprenant la construction d'anticorps en une concentration d'au moins 5 nM.

16. Procédé selon l'une quelconque des revendications 13 à 15, dans lequel l'antigène récepteur de cellules NK auquel le premier domaine de liaison de la construction d'anticorps se lie à et est choisi dans le groupe constitué de CD16a, CD16b, NKp46, NKG2D et CD16a + CD16b.

17. Procédé selon l'une quelconque des revendications 13 à 16, dans lequel l'antigène de surface cellulaire sur la surface cellulaire d'une cellule cible à laquelle le second domaine de liaison de la construction d'anticorps se lie à est choisi dans le groupe constitué de CD19, CD22, CD30, CD33, CD52, CD70, CD74, CD79b, CD123, CLL1, BCMA, FCRH5, EGFR, HER2, GD2.

18. Procédé selon l'une quelconque des revendications 13 à 17, dans lequel l'étape de congélation des cellules NK est réalisée à l'aide d'un milieu de congélation qui contient au moins un milieu de culture cellulaire basal et un agent cryoprotecteur.

19. Procédé selon l'une quelconque des revendications 13 à 18, dans lequel la construction d'anticorps comprend une séquence protéique telle que représentée dans les SEQ ID NO : 161-171.

20. Procédé de reconstitution/préparation de cellules NK humaines préchargées viables pour l'administration à un sujet en son besoin, le procédé comprenant l'étape de
(a) la décongélation de cellules NK cryoconservées selon l'une quelconque des revendications 1 à 12 ; soit
(b) la décongélation de cellules NK cryoconservées préparées par un procédé selon l'une quelconque des revendications 13 à 19.
